# EUROPÄISCHE PATENTSCHRIFT

(11) **EP 1 987 092 B1**
(45) Veröffentlichungstag und Bekanntmachung des Hinweises auf die Patenterteilung: **22.05.2013**
(21) Anmeldenummer: 07726419.0
(22) Anmeldetag: 16.02.2007
(51) Int. Cl.: C08K 5/09, C09B 5/62, H01L 31/055

(54) **FLUORIERTE RYLENTETRACARBONSÄUREDERIVATE UND DEREN VERWENDUNG**
FLUORINATED RYLENETETRACARBOXYLIC ACID DERIVATIVES AND USE THEREOF
DERIVES FLUORES D'ACIDE RYLENETETRACARBOXYLIQUE ET LEUR UTILISATION

(30) Priorität: 17.02.2006 EP 06003266; 23.05.2006 EP 06114427
(43) Veröffentlichungstag der Anmeldung: 05.11.2008
(73) Patentinhaber: BASF SE, 67056 Ludwigshafen (DE)
(72) Erfinder: KÖNEMANN, Martin, 68163 Mannheim (DE); OSSWALD, Peter, 88489 Wain (DE); SCHMIDT, Rüdiger, 33098 Paderborn (DE); WÜRTHNER, Frank, 97204 Höchberg (DE)
(86) Internationale Anmeldenummer: PCT/EP2007/051532
(87) Internationale Veröffentlichungsnummer: WO 2007/093643

(56) Entgegenhaltungen:
- US-A1- 2005 176 970
- JONES ET AL.: "High-Mobility Air-Stable n-Type Semiconductors with Processing Versatility: Dicyanoperylene-3,4:9,10-bis(dicarboximide s)" ANGEWANDTE CHEMIE, Bd. 116, 2004, Seiten 6523-6526, XP002430942 in der Anmeldung erwähnt
- WÜRTHNER ET AL.: "Synthesis and Optical and Electrochemical Properties of Core-Fluorinated Perylene Bisimides" ORGANIC LETTERS, Bd. 8, Nr. 17, 25. Juli 2006 (2006-07-25), Seiten 3765-3768, XP002430943

## Beschreibung

Die vorliegende Erfindung betrifft fluorierte Rylentetracarbonsäurederivate, ein Verfahren zu ihrer Herstellung und deren Verwendung, insbesondere als organische Halbleiter vom n-Typ.

Es wird erwartet, dass zukünftig in vielen Bereichen der Elektronikindustrie neben den klassischen anorganischen Halbleitern zunehmend auch organische Halbleiter auf Basis von niedermolekularen oder polymeren Materialien eingesetzt werden. Diese weisen vielfach Vorteile gegenüber den klassischen anorganischen Halbleitern auf, beispielsweise eine bessere Substratkompatibilität und eine bessere Verarbeitbarkeit der auf ihnen basierenden Halbleiterbauteile. Sie erlauben die Verarbeitung auf flexiblen Substraten und ermöglichen es, ihre Grenzorbitalenergien mit den Methoden des Molecular Modellings auf den jeweiligen Anwendungsbereich genau anzupassen. Die deutlich verringerten Kosten derartiger Bauteile haben dem Forschungsgebiet der organischen Elektronik eine Renaissance gebracht. Die "Organische Elektronik" beschäftigt sich schwerpunktmäßig mit der Entwicklung neuer Materialien und Fertigungsprozesse für die Herstellung elektronischer Bauelemente auf der Basis organischer Halbleiterschichten. Dazu zählen vor allem organische Feldeffekttransistoren (Organic Field-Effect Transistors, OFET) sowie organische Leuchtdioden (Organic Light Emitting Diods, OLED; z. B. für einen Einsatz in Displays) und die Photovoltaik. Organischen Feldeffekttransistoren wird ein großes Entwicklungspotential, beispielsweise in Speicherelementen und integrierten optoelektronischen Vorrichtungen zugeschrieben. Es besteht daher ein großer Bedarf an organischen Verbindungen, die sich als organische Halbleiter, insbesondere Halbleiter vom n-Typ und speziell für einen Einsatz in organischen Feldeffekttransistoren eignen.

Die DE-A-32 35 526 betrifft Perylen-3,4,9,10-tetracarbonsäurediimide, die am Perylengerüst mit Alkoxy- oder Alkylthiogruppen sowie mit Chlor oder Brom substituiert sind, ein Verfahren zu ihrer Herstellung und deren Verwendung in lichtsammelnden Kunststoffen. Zwar wird im nicht erfindungsgemäßen Beispiel 11 dieses Dokuments ein fluoriertes N,N-Bisisooctylperylimid unter Angabe der Summenformel C₄₀H₃₇N₂O₄ClF₄ beschrieben, die Verwendung einer solchen Verbindung als organischer Halbleiter ist jedoch nicht offenbart.

Die DE-A-195 47 209 betrifft 1,7-Diaryloxy- oder 1,7-Diarylthio-substituierte Perylen-3,4,9,10-tetracarbonsäuren und deren Dianhydride und Diimide. Zu deren Herstellung werden 1,7-Dibromperylen-3,4,9,10-tetracarbonsäurediimide als Zwischenprodukte eingesetzt. Mit Fluor substituierte Perylentetracarbonsäurederivate sind in diesem Dokument nicht beschrieben.

Die US 5,986,099 (WO 96/22332) beschreibt substituierte Quaterrylentetracarbonsäurediimide, bei denen das aromatische Grundgerüst bis zu 12 Substituenten aufweisen kann, wobei es sich u. a. um Halogensubstituenten handeln kann. Fluorierte Quaterrylentetracarbonsäurediimide sind nicht beschrieben.

Die US 2005/0222416 A1 (WO 03/104232) beschreibt 1,6,9,14-tetrasubstituierte Terylentetracarbonsäurediimide. Am Aromatengrundgerüst fluorierte Verbindungen sind auch in diesem Dokument nicht beschrieben.

Die DE-A-101 48 172 beschreibt fluoreszierende Naphthalin-1,4,5,8-tetracarbonsäurebisimide mit elektronenschiebenden Substituenten am Aromatenkern.

Die US 2003/0181721 A1 beschreibt Perylen-3,4,9,10-tetracarbonsäurediimide, die in der 1-, 6-, 7- und/oder 12-Position substituiert sein können. Fluorierte Verbindungen sind auch in diesem Dokument nicht beschrieben.

M. J. Ahrens, M. J. Fuller und M. R. Wasielewski beschreiben in Chem. Mater. 2003, 15, S. 2684 - 2686 Cyano-substituierte Perylen-3,4-dicarboximide und Perylen-3,4,9,10-bis(dicarboximide).

B. A. Jones et al. beschreiben in Angew. Chem. 2004, 116, S. 6523 - 6526 den Einsatz von Dicyanoperylen-3,4,9,10-bis(dicarboximiden) als n-Halbleiter.

Die US 2005/0176970 A1 beschreibt n-Halbleiter auf Basis von cyanosubstituierten Perylen-3,4-dicarboximiden und Perylen-3,4,9,10-bis(dicarboximiden).

Die unveröffentlichte deutsche Patentanmeldung 10 2005 061 997.5 beschreibt mit Br, F und/oder CN substituierte Naphthalintetracarbonsäurederivate und deren Verwendung, z. B. in organischen Feldeffekttransistoren.

F. Würther et al. beschreiben in einem nachveröffentlichten Artikel in Organic Letters, Bd. 8, Nr. 17, Seiten 3765 - 3768 die Synthese und optischen und elektrochemischen Eigenschaften von kernfluorierten Perylenbisimiden.

Der vorliegenden Erfindung liegt die Aufgabe zu Grunde, Verbindungen zur Verfügung zu stellen, die sich als Halbleiter, z. B. für einen Einsatz in organischen Feldeffekttransistoren, eignen. Vorzugsweise soll es sich um luftstabile Verbindungen handeln.

Gelöst wird diese Aufgabe durch die Verwendung von Verbindungen der allgemeinen Formel I 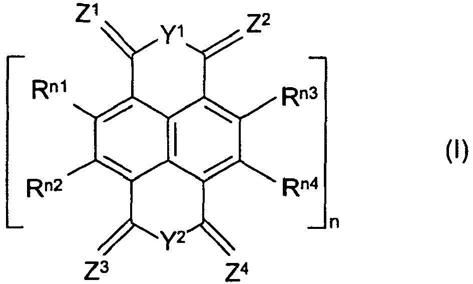 oder von Verbindungen der Formeln I.Bb 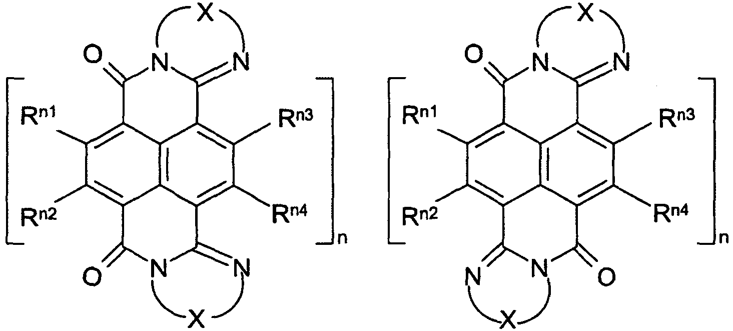 wobei
n in den Verbindungen der allgemeinen Formel I für 3 oder 4 steht und in den Verbindungen der allgemeinen Formeln I.Bb für 2, 3 oder 4 steht,
wenigstens einer der Reste Rⁿ¹, Rⁿ², Rⁿ³ und Rⁿ⁴ für Fluor steht,
gegebenenfalls wenigstens ein weiterer Rest Rⁿ¹, Rⁿ², Rⁿ³ und Rⁿ⁴ für einen Substituenten steht, der ausgewählt ist unter Cl und Br, und die übrigen Reste für Wasserstoff stehen,
X für eine zweiwertige verbrückende Gruppe mit 2 bis 5 Atomen zwischen den flankierenden Bindungen steht,
Y¹ für O oder NR^{a} steht, wobei R^{a} für Wasserstoff oder einen Organylrest steht,
Y² für O oder NR^{b} steht, wobei R^{b} für Wasserstoff oder einen Organylrest steht,
Z¹, Z², Z³ und Z⁴ für O stehen,
als Halbleiter, insbesondere als n-Halbleiter.

Ein weiterer Gegenstand der Erfindung ist die Verwendung von Verbindungen der allgemeinen Formel I für optische Label, zur unsichtbaren Markierung von Produkten, als Fluoreszenzfarbstoffe, als Fluoreszenzlabel für Biomoleküle und als Pigmente.

Ein weiterer Gegenstand der Erfindung sind Verbindungen der Formel I, wie in Anspruch 18 definiert sowie ein organischer Feld-effekttransistor, ein Substrat mit einer Vielzahl von organischen Feld effekttransistoren und ein Halbleiter baustein auf Basis dieser Verbindungen (I).

In den Verbindungen der Formel I bezeichnet n die Anzahl der in der peri-Position verknüpften Naphthalineinheiten, die das Grundgerüst der erfindungsgemäßen Rylenverbindungen bilden. In den einzelnen Resten Rⁿ¹ bis Rⁿ⁴ bezeichnet n die jeweilige Naphthalingruppe des Rylengerüsts, an das die Reste gebunden sind. Reste Rⁿ¹ bis Rⁿ⁴, die an unterschiedliche Naphthalingruppen gebunden sind, können jeweils gleiche oder verschiedene Bedeutungen aufweisen. Demgemäß kann es sich bei den Verbindungen der allgemeinen Formel I um Perylene, Terylene oder Quaterrylene der folgenden Formel handeln:

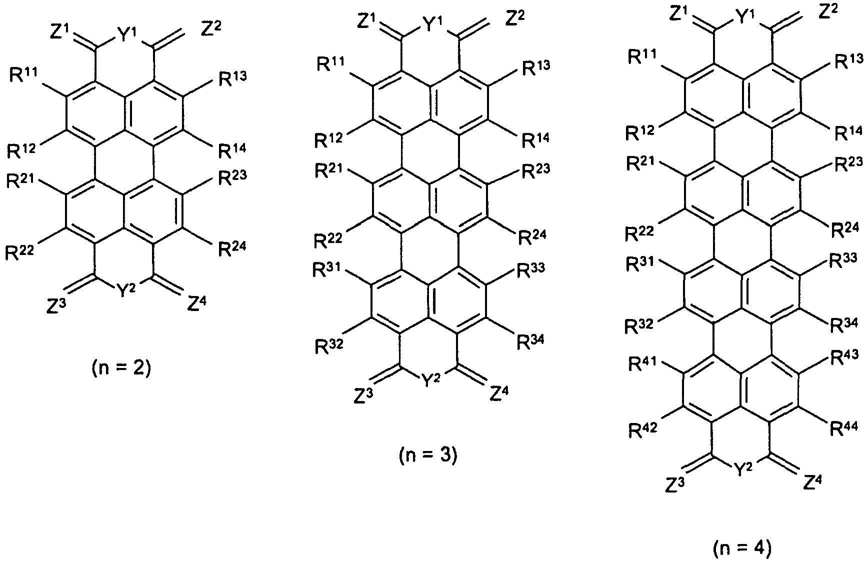

Die höheren Homologen (n = 5, 6) werden analog gebildet.

Im Rahmen der vorliegenden Erfindung umfasst der Ausdruck "Alkyl" geradkettiges oder verzweigtes Alkyl. Vorzugsweise handelt es sich um geradkettiges oder verzweigtes C₁-C₃₀-Alkyl, insbesondere um C₁-C₂₀-Alkyl und ganz besonders bevorzugt C₁-C₁₈-Alkyl. Beispiele für Alkylgruppen sind insbesondere Methyl, Ethyl, n-Propyl, Isopropyl, n-Butyl, Isobutyl, sec.-Butyl, tert.-Butyl, n-Pentyl, n-Hexyl, n-Heptyl, n-Octyl, n-Nonyl, n-Decyl, n-Undecyl, n-Dodecyl, n-Tridecyl, n-Tetradecyl, n-Hexadecyl, n-Octadecyl und n-Eicosyl.

Der Ausdruck Alkyl umfasst auch Alkylreste, deren Kohlenstoffketten durch eine oder mehrere nicht benachbarte Gruppen, die ausgewählt sind unter -O-, -S-, -NR^{e}-, -CO-, -SO- und/oder -SO₂- unterbrochen sein kann. R^{e} steht vorzugsweise für Wasserstoff, Alkyl, Cycloalkyl, Heterocycloalkyl, Aryl oder Hetaryl. Der Ausdruck Alkyl umfasst auch substituierte Alkylreste. Substituierte Alkylgruppen können in Abhängigkeit von der Länge der Alkylkette einen oder mehrere (z. B. 1, 2, 3, 4, 5 oder mehr als 5) Substituenten aufweisen. Diese sind vorzugsweise unabhängig voneinander ausgewählt unter Cycloalkyl, Heterocycloalkyl, Aryl, Hetaryl, Halogen, Hydroxy, Thiol, COOH, Carboxylat, SO₃H, Sulfonat, NE¹E², Nitro und Cyano, wobei E¹ und E² unabhängig voneinander für Wasserstoff, Alkyl, Cycloalkyl, Heterocycloalkyl, Aryl oder Hetaryl stehen. Halogensubstituenten sind vorzugsweise Fluor, Chlor oder Brom.

Carboxylat und Sulfonat stehen für ein Derivat einer Carbonsäurefunktion bzw. einer Sulfonsäurefunktion, insbesondere für ein Metallcarboxylat oder -sulfonat, eine Carbonsäureester- oder Sulfonsäureesterfunktion oder eine Carbonsäure- oder Sulfonsäureamidfunktion. Cycloalkyl-, Heterocycloalkyl-, Aryl- und Hetarylsubstituenten der Alkylgruppen können ihrerseits unsubstituiert oder substituiert sein; geeignete Substituenten sind die nachfolgend für diese Gruppen genannten.

Die vorstehenden Ausführungen zu Alkyl gelten auch für die Alkylteile in Alkoxy, Alkylamino, Alkylthio, Alkylsulfinyl, Alkylsulfonyl, etc.

Durch Aryl substituierte Alkylreste ("Arylalkyl") weisen wenigstens eine, wie nachfolgend definierte, unsubstituierte oder substituierte Arylgruppe auf. Dabei kann die Alkylgruppe in "Arylalkyl" wenigstens einen weiteren Substituenten tragen und/oder durch eine oder mehrere nicht benachbarte Gruppen, die ausgewählt sind unter -O-, -S-, -NR^{e}-, -CO- und/oder -SO₂- unterbrochen sein. Arylalkyl steht vorzugsweise für Phenyl-C₁-C₁₀-alkyl, besonders bevorzugt für Phenyl-C₁-C₄-alkyl, z. B. für Benzyl, 1-Phenethyl, 2-Phenethyl, 1-Phenprop-1-yl, 2-Phenprop-1-yl, 3-Phenprop-1-yl, 1-Phenbut-1-yl, 2-Phenbut-1-yl, 3-Phenbut-1-yl, 4-Phenbut-1-yl, 1-Phenbut-2-yl, 2-Phenbut-2-yl, 3-Phenbut-2-yl, 4-Phenbut-2-yl, 1-(Phenmeth)-eth-1-yl, 1-(Phenmethyl)-1-(methyl)-eth-1-yl oder -(Phenmethyl)-1-(methyl)-prop-1-yl; vorzugsweise für Benzyl und 2-Phenethyl.

Der Ausdruck "Alkenyl" umfasst im Sinne der vorliegenden Erfindung geradkettige und verzweigte Alkenylgruppen, die in Abhängigkeit von der Kettenlänge eine oder mehrere Doppelbindungen (z. B. 1, 2, 3, 4 oder mehr als 4) tragen können. Bevorzugt sind C₂-C₁₈-, besonders bevorzugt C₂-C₁₂-Alkenylgruppen. Der Ausdruck "Alkenyl" umfasst auch substituierte Alkenylgruppen, welche einen oder mehrere (z. B. 1, 2, 3, 4 5 oder mehr als 5) Substituenten tragen können. Geeignete Substituenten sind z. B. ausgewählt unter Cycloalkyl, Heterocycloalkyl, Aryl, Hetaryl, Halogen, Hydroxy, Thiol, COOH, Carboxylat, SO₃H, Sulfonat, NE³E⁴, Nitro und Cyano, wobei E³ und E⁴ unabhängig voneinander für Wasserstoff, Alkyl, Cycloalkyl, Heterocycloalkyl, Aryl oder Hetaryl stehen.

Alkenyl steht dann beispielsweise für Ethenyl, 1-Propenyl, 2-Propenyl, 1-Butenyl, 2-Butenyl, 3-Butenyl, 1-Pentenyl, 2-Pentenyl, 3-Pentenyl, 4-Pentenyl, 1-Hexenyl, 2-Hexenyl, 3-Hexenyl, 4-Hexenyl, 5-Hexenyl, Penta-1,3-dien-1-yl, Hexa-1,4-dien-1-yl, Hexa-1,4-dien-3-yl, Hexa-1,4-dien-6-yl, Hexa-1,5-dien-1-yl, Hexa-1,5-dien-3-yl, Hexa-1,5-dien-4-yl, Hepta-1,4-dien-1-yl, Hepta-1,4-dien-3-yl, Hepta-1,4-dien-6-yl, Hepta-1,4-dien-7-yl, Hepta-1,5-dien-1-yl, Hepta-1,5-dien-3-yl, Hepta-1,5-dien-4-yl, Hepta-1,5-dien-7-yl, Hepta-1,6-dien-1-yl, Hepta-1,6-dien-3-yl, Hepta-1,6-dien-4-yl, Hepta-1,6-dien-5-yl, Hepta-1,6-dien-2-yl, Octa-1,4-dien-1-yl, Octa-1,4-dien-2-yl, Octa-1,4-dien-3-yl, Octa-1,4-dien-6-yl, Octa-1,4-dien-7-yl, Octa-1,5-dien-1-yl, Octa-1,5-dien-3-yl, Octa-1,5-dien-4-yl, Octa-1,5-dien-7-yl, Octa-1,6-dien-1-yl, Octa-1,6-dien-3-yl, Octa-1,6-dien-4-yl, Octa-1,6-dien-5-yl, Octa-1,6-dien-2-yl, Deca-1,4-dienyl, Deca-1,5-dienyl, Deca-1,6-dienyl, Deca-1,7-dienyl, Deca-1,8-dienyl, Deca-2,5-dienyl, Deca-2,6-dienyl, Deca-2,7-dienyl, Deca-2,8-dienyl und dergleichen. Die Ausführungen zu Alkenyl gelten auch für die Alkenylgruppen in Alkenyloxy, Alkenylthio, etc.

Der Ausdruck "Alkinyl" umfasst unsubstituierte oder substituierte Alkinylgruppen, die eine oder mehrere nicht benachbarte Dreifachbindungen aufweisen, wie Ethinyl, 1-Propinyl, 2-Propinyl, 1-Butinyl, 2-Butinyl, 3-Butinyl, 1-Pentinyl, 2-Pentinyl, 3-Pentinyl, 4-Pentinyl, 1-Hexinyl, 2-Hexinyl, 3-Hexinyl, 4-Hexinyl, 5-Hexinyl, und dergleichen. Die Ausführungen zu Alkinyl gelten auch für die Alkinylgruppen in Alkinyloxy, Alkinylthio, etc. Substituierte Alkinyle tragen vorzugsweise einen oder mehrere (z. B. 1, 2, 3, 4, 5 oder mehr als 5) der zuvor für Alkyl genannten Substituenten.

Der Ausdruck "Cycloalkyl" umfasst im Rahmen der vorliegenden Erfindung unsubstituierte als auch substituierte Cycloalkylgruppen, vorzugsweise C₃-C₈-Cycloalkylgruppen wie Cyclopropyl, Cyclobutyl, Cyclopentyl, Cyclohexyl, Cycloheptyl oder Cyclooctyl, insbesondere C₅-C₈-Cycloalkyl. Substituierte Cycloalkylgruppen können einen oder mehrere (z. B. 1, 2, 3, 4, 5 oder mehr als 5) Substituenten aufweisen. Diese sind vorzugsweise unabhängig voneinander ausgewählt unter Alkyl sowie den zuvor für die Alkylgruppen genannten Substituenten. Die Cycloalkylgruppen tragen im Falle einer Substitution vorzugsweise eine oder mehrere, beispielsweise eine, zwei, drei, vier oder fünf C₁-C₆-Alkylgruppen.

Beispiele für bevorzugte Cycloalkylgruppen sind Cyclopentyl, 2- und 3-Methylcyclopentyl, 2- und 3-Ethylcyclopentyl, Cyclohexyl, 2-, 3- und 4-Methylcyclohexyl, 2-, 3- und 4-Ethylcyclohexyl, 3- und 4-Propylcyclohexyl, 3- und 4-Isopropylcyclohexyl, 3- und 4-Butylcyclohexyl, 3- und 4-sec.-Butylcyclohexyl, 3- und 4-tert.-Butylcyclohexyl, Cycloheptyl, 2-, 3- und 4-Methylcycloheptyl, 2-, 3- und 4-Ethylcycloheptyl, 3- und 4-Propylcycloheptyl, 3- und 4-Isopropylcycloheptyl, 3- und 4-Butylcycloheptyl, 3- und 4-sec.-Butylcycloheptyl, 3- und 4-tert.-Butylcycloheptyl, Cyclooctyl, 2-, 3-, 4- und 5-Methylcyclooctyl, 2-, 3-, 4- und 5-Ethylcyclooctyl, 3-, 4- und 5-Propylcyclooctyl.

Der Ausdruck Cycloalkenyl umfasst unsubstituierte und substituierte einfach ungesättigte Kohlenwasserstoffgruppen mit 3 bis 8, vorzugsweise 5 bis 6 Kohlenstoffringgliedern, wie Cyclopenten-1-yl, Cyclopenten-3-yl, Cyclohexen-1-yl, Cyclohexen-3-yl, Cyclohexen-4-yl und dergleichen. Geeignete Substituenten sind die zuvor für Cycloalkyl genannten.

Der Ausdruck Bicycloalkyl umfasst vorzugsweise bicyclische Kohlenwasserstoffreste mit 5 bis 10 C-Atomen wie Bicyclo[2.2.1]hept-1-yl, Bicyclo[2.2.1]hept-2-yl, Bicyclo[2.2.1]hept-7-yl, Bicyclo[2.2.2]oct-1-yl, Bicyclo[2.2.2]oct-2-yl, Bicyclo[3.3.0]octyl, Bicyclo[4.4.0]decyl und dergleichen.

Der Ausdruck "Aryl" umfasst im Rahmen der vorliegenden Erfindung ein- oder mehrkernige aromatische Kohlenwasserstoffreste, die unsubstituiert oder substituiert sein können. Aryl steht vorzugsweise für unsubstituiertes oder substituiertes Phenyl, Naphthyl, Indenyl, Fluorenyl, Anthracenyl, Phenanthrenyl, Naphthacenyl, Chrysenyl, Pyrenyl, etc., und besonders bevorzugt für Phenyl oder Naphthyl. Substituierte Aryle können in Abhängigkeit von der Anzahl und Größe ihrer Ringsysteme einen oder mehrere (z. B. 1, 2, 3, 4, 5 oder mehr als 5) Substituenten aufweisen. Diese sind vorzugsweise unabhängig voneinander ausgewählt unter Alkyl, Alkoxy, Cycloalkyl, Heterocycloalkyl, Aryl, Hetaryl, Halogen, Hydroxy, Thiol, COOH, Carboxylat, SO₃H, Sulfonat, NE⁵E⁶, Nitro und Cyano, wobei E⁵ und E⁶ unabhängig voneinander für Wasserstoff, Alkyl, Cycloalkyl, Heterocycloalkyl, Aryl oder Hetaryl stehen. Halogensubstituenten sind vorzugsweise Fluor, Chlor oder Brom. Besonders bevorzugt steht Aryl für Phenyl, das im Falle einer Substitution im Allgemeinen 1, 2, 3, 4 oder 5, vorzugsweise 1, 2 oder 3 Substituenten tragen kann.

Aryl, das einen oder mehrere Reste trägt, steht beispielsweise für 2-, 3- und 4-Methylphenyl, 2,4-, 2,5-, 3,5- und 2,6-Dimethylphenyl, 2,4,6-Trimethylphenyl, 2-, 3-und 4-Ethylphenyl, 2,4-, 2,5-, 3,5- und 2,6-Diethylphenyl, 2,4,6-Triethylphenyl, 2-, 3-und 4-Propylphenyl, 2,4-, 2,5-, 3,5- und 2,6- Dipropylphenyl, 2,4,6-Tripropylphenyl, 2-, 3- und 4-Isopropylphenyl, 2,4-, 2,5-, 3,5- und 2,6-Diisopropylphenyl, 2,4,6-Triisopropylphenyl, 2-, 3- und 4-Butylphenyl, 2,4-, 2,5-, 3,5- und 2,6-Dibutylphenyl, 2,4,6-Tributylphenyl, 2-, 3- und 4-Isobutylphenyl, 2,4-, 2,5-, 3,5- und 2,6-Diisobutylphenyl, 2,4,6-Triisobutylphenyl, 2-, 3- und 4-sec-Butylphenyl, 2,4-, 2,5-, 3,5- und 2,6-Di-sec-butylphenyl, 2,4,6-Tri-sec-butylphenyl, 2-, 3- und 4-tert.-Butylphenyl, 2,4-, 2,5-, 3,5- und 2,6-Di-tert.-butylphenyl und 2,4,6-Tri-tert.-butylphenyl; 2-, 3- und 4-Methoxyphenyl, 2,4-, 2,5-, 3,5- und 2,6-Dimethoxyphenyl, 2,4,6-Trimethoxyphenyl, 2-, 3- und 4-Ethoxyphenyl, 2,4-, 2,5-, 3,5- und 2,6-Diethoxyphenyl, 2,4,6-Triethoxyphenyl, 2-, 3- und 4-Propoxyphenyl, 2,4-, 2,5-, 3,5- und 2,6-Dipropoxyphenyl, 2-, 3- und 4-Isopropoxyphenyl, 2,4-, 2,5-, 3,5- und 2,6-Diisopropoxyphenyl und 2-, 3- und 4-Butoxyphenyl; 2-, 3- und 4-Cyanophenyl.

Der Ausdruck "Heterocycloalkyl" umfasst im Rahmen der vorliegenden Erfindung nicht-aromatische, ungesättigte oder vollständig gesättigte, cycloaliphatische Gruppen mit im Allgemeinen 5 bis 8 Ringatomen, vorzugsweise 5- oder 6 Ringatomen, in denen 1, 2 oder 3 der Ringkohlenstoffatome durch Heteroatome, ausgewählt unter Sauerstoff, Stickstoff, Schwefel und einer Gruppe -NRe- ersetzt sind und das unsubstituiert ist oder mit einer oder mehreren, beispielsweise 1, 2, 3, 4, 5 oder 6 C₁-C₆-Alkylgruppen substituiert ist. Beispielhaft für solche heterocycloaliphatischen Gruppen seien Pyrrolidinyl, Piperidinyl, 2,2,6,6-Tetramethyl-piperidinyl, Imidazolidinyl, Pyrazolidinyl, Oxazolidinyl, Morpholidinyl, Thiazolidinyl, Isothiazolidinyl, Isoxazolidinyl, Piperazinyl-, Tetrahydrothiophenyl, Dihydrothien-2-yl, Tetrahydrofuranyl, Dihydrofuran-2-yl, Tetrahydropyranyl, 1,2-Oxazolin-5-yl, 1,3-Oxazolin-2-yl und Dioxanyl genannt.

Der Ausdruck "Heteroaryl" umfasst im Rahmen der vorliegenden Erfindung unsubstituierte oder substituierte, heteroaromatische, ein- oder mehrkernige Gruppen, vorzugsweise die Gruppen Pyridyl, Chinolinyl, Acridinyl, Pyridazinyl, Pyrimidinyl, Pyrazinyl, Pyrrolyl, Imidazolyl, Pyrazolyl, Indolyl, Purinyl, Indazolyl, Benzotriazolyl, 1,2,3-Triazolyl, 1,3,4-Triazolyl und Carbazolyl, wobei diese heterocycloaromatischen Gruppen im Falle einer Substitution im Allgemeinen 1, 2 oder 3 Substituenten, tragen können. Die Substituenten sind vorzugsweise ausgewählt unter C₁-C₆-Alkyl, C₁-C₆-Alkoxy, Hydroxy, Carboxy, Halogen und Cyano.

Stickstoffhaltige 5- bis 7-gliedrige Heterocycloalkyl- oder Heteroarylreste, die gegebenenfalls weitere unter Sauerstoff und Schwefel ausgewählte Heteroatome enthalten, umfassen beispielsweise Pyrrolyl, Pyrazolyl, Imidazolyl, Triazolyl, Pyrrolidinyl, Pyrazolinyl, Pyrazolidinyl, Imidazolinyl, Imidazolidinyl, Pyridinyl, Pyridazinyl, Pyrimidinyl, Pyrazinyl, Triazinyl, Piperidinyl, Piperazinyl, Oxazolyl, Isooxazolyl, Thiazolyl, Isothiazolyl, Indolyl, Chinolinyl, Isochinolinyl oder Chinaldinyl.

Halogen steht für Fluor, Chlor, Brom oder Iod.

Konkrete Beispiele für die in den folgenden Formeln genannten Reste R^{a} und R^{b} sind im Einzelnen:
Methyl, Ethyl, n-Propyl, Isopropyl, n-Butyl, Isobutyl, sec.-Butyl, tert.-Butyl, n-Pentyl, n-Hexyl, n-Heptyl, n-Octyl, n-Nonyl, n-Decyl, n-Undecyl, n-Dodecyl, n-Tridecyl, n-Tetradecyl, n-Hexadecyl, n-Octadecyl und n-Eicosyl, 2-Methoxyethyl, 2-Ethoxyethyl, 2-Propoxyethyl, 2-Butoxyethyl, 3-Methoxypropyl, 3-Ethoxypropyl, 3-Propoxypropyl, 3-Butoxypropyl, 4-Methoxybutyl, 4-Ethoxybutyl, 4-Propoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 2- und 4-Butoxybutyl, 4,8-Dioxadecyl, 3,6,9-Trioxadecyl, 3,6,9-Trioxaundecyl, 3,6,9-Trioxadodecyl, 3,6,9,12-Tetraoxatridecyl und 3,6,9,12-Tetraoxatetradecyl;
2-Methylthioethyl, 2-Ethylthioethyl, 2-Propylthioethyl, 2-Butylthio-ethyl, 3-Methylthiopropyl, 3-Ethylthiopropyl, 3-Propylthiopropyl, 3-Butylthiopropyl, 4-Methylthiobutyl, 4-Ethylthiobutyl, 4-Propylthiobutyl, 3,6-Dithiaheptyl, 3,6-Dithiaoctyl, 4,8-Dithianonyl, 3,7-Dithiaoctyl, 3,7-Di-thianonyl, 2- und 4-Butylthiobutyl, 4,8-Dithiadecyl, 3,6,9-Trithiadecyl, 3,6,9-Trithia-undecyl, 3,6,9-Trithiadodecyl, 3,6,9,12-Tetrathiatridecyl und 3,6,9,12-Tetrathiatetradecyl;
2-Monomethyl- und 2-Monoethylaminoethyl, 2-Dimethylaminoethyl, 2- und 3-Dimethyl-aminopropyl, 3-Monoisopropylaminopropyl, 2- und 4-Monopropylaminobutyl, 2- und 4-Dimethylaminobutyl, 6-Methyl-3,6-diazaheptyl, 3,6-Dimethyl-3,6-diazaheptyl, 3,6-Di-azaoctyl, 3,6-Dimethyl-3,6-diazaoctyl, 9-Methyl-3,6,9-triazadecyl, 3,6,9-Trimethyl-3,6,9-triazadecyl, 3,6,9-Triazaundecyl, 3,6,9-Trimethyl-3,6,9-triazaundecyl, 12-Methyl-3,6,9,12-tetraazatridecyl und 3,6,9,12-Tetramethyl-3,6,9,12-tetraazatridecyl;
(1-Ethylethyliden)aminoethylen, (1-Ethylethyliden)aminopropylen, (1-Ethylethyliden)-aminobutylen, (1-Ethylethyliden)aminodecylen und (1-Ethylethyliden)aminododecylen;
Propan-2-on-1-yl, Butan-3-on-1-yl, Butan-3-on-2-yl und 2-Ethylpentan-3-on-1-yl;
2-Methylsulfinylethyl, 2-Ethylsulfinylethyl, 2-Propylsulfinylethyl, 2-Isopropylsulfinylethyl, 2-Butylsulfinylethyl, 2- und 3-Methylsulfinylpropyl, 2- und 3-Ethylsulfinylpropyl, 2- und 3-Propylsulfinylpropyl, 2- und 3-Butylsulfinylpropyl, 2- und 4-Methylsulfinylbutyl, 2- und 4-Ethylsulfinylbutyl, 2- und 4-Propylsulfinylbutyl und 4-Butylsulfinylbutyl;
2-Methylsulfonylethyl, 2-Ethylsulfonylethyl, 2-Propylsulfonylethyl, 2-lsopropylsulfonylethyl, 2-Butylsulfonylethyl, 2- und 3-Methylsulfonylpropyl, 2- und 3-Ethylsulfonylpropyl, 2- und 3-Propylsulfonylpropyl, 2- und 3-Butylsulfonylproypl, 2-und 4-Methylsulfonylbutyl, 2- und 4-Ethylsulfonylbutyl, 2- und 4-Propylsulfonylbutyl und 4-Butylsulfonylbutyl;
Carboxymethyl, 2-Carboxyethyl, 3-Carboxypropyl, 4-Carboxybutyl, 5-Carboxypentyl, 6-Carboxyhexyl, 8-Carboxyoctyl, 10-Carboxydecyl, 12-Carboxydodecyl und 14-Carboxy-tetradecyl;
Sulfomethyl, 2-Sulfoethyl, 3-Sulfopropyl, 4-Sulfobutyl, 5-Sulfopentyl, 6-Sulfohexyl, 8-Sulfooctyl, 10-Sulfodecyl, 12-Sulfododecyl und 14-Sulfotetradecyl;
2-Hydroxyethyl, 2- und 3-Hydroxypropyl, 3- und 4-Hydroxybutyl und 8-Hydroxy-4-oxaoctyl;
2-Cyanoethyl, 3-Cyanopropyl, 3- und 4-Cyanobutyl;
2-Chlorethyl, 2- und 3-Chlorpropyl, 2-, 3- und 4-Chlorbutyl, 2-Bromethyl, 2- und 3-Brompropyl und 2-, 3- und 4-Brombutyl;
2-Nitroethyl, 2- und 3-Nitropropyl und 2-, 3- und 4-Nitrobutyl;
Methoxy, Ethoxy, Propoxy, Butoxy, Pentoxy und Hexoxy;
Methylthio, Ethylthio, Propylthio, Butylthio, Pentylthio und Hexylthio;
Ethinyl, 1- und 2-Propinyl, 1-, 2- und 3-Butinyl, 1-, 2-, 3- und 4-Pentinyl, 1-, 2-, 3-, 4-und 5-Hexinyl, 1-, 2-, 3-, 4-, 5-, 6-, 7-, 8- und 9-Decinyl, 1-, 2-, 3-, 4-, 5-, 6-, 7-, 8-, 9-, 10- und 11-Dodecinyl und 1-, 2-, 3-, 4-, 5-, 6-, 7-, 8-, 9-, 10-, 11-, 12-, 13-, 14-, 15-, 16-und 17-Octadecinyl;
Ethenyl, 1- und 2-Propenyl, 1-, 2- und 3-Butenyl, 1-, 2-, 3- und 4-Pentenyl, 1-, 2-, 3-, 4-und 5-Hexenyl, 1-, 2-, 3-, 4-, 5-, 6-, 7-, 8- und 9-Decenyl, 1-, 2-, 3-, 4-, 5-, 6-, 7-, 8-, 9-, 10- und 11-Dodecenyl und 1-, 2-, 3-, 4-, 5-, 6-, 7-, 8-, 9-, 10-, 11-, 12-, 13-, 14-, 15-, 16-und 17-Octadecenyl;
Methylamino, Ethylamino, Propylamino, Butylamino, Pentylamino, Hexylamino, Dicyclopentylamino, Dicyclohexylamino, Dicycloheptylamino, Diphenylamino und Dibenzylamino;
Formylamino, Acetylamino, Propionylamino und Benzoylamino;
Carbamoyl, Methylaminocarbonyl, Ethylaminocarbonyl, Propylaminocarbonyl, Butylaminocarbonyl, Pentylaminocarbonyl, Hexylaminocarbonyl, Heptylaminocarbonyl, Octylaminocarbonyl, Nonylaminocarbonyl, Decylaminocarbonyl und Phenylaminocarbonyl;
Aminosulfonyl, N-Dodecylaminosulfonyl, N,N-Diphenylaminosulfonyl, und N,N-Bis(4-Chlorphenyl)aminosulfonyl;
Methoxycarbonyl, Ethoxycarbonyl, Propoxycarbonyl Hexoxycarbonyl, Dodecyloxycarbonyl, Octadecyloxycarbonyl, Phenoxycarbonyl, (4-tert-Butyl-phenoxy)carbonyl und (4-Chlorphenoxy)carbonyl;
Methoxysulfonyl, Ethoxysulfonyl, Propoxysulfonyl, Butoxysulfonyl, Hexoxysulfonyl, Dodecyloxysulfonyl, Octadecyloxysulfonyl, Phenoxysulfonyl, 1- und 2-Naphthyloxysulfonyl, (4-tert.-Butylphenoxy)-sulfonyl und (4-Chlorphenoxy)sulfonyl;
Diphenylphosphino, Di-(o-tolyl)phosphino und Diphenylphosphinoxido;
Fluor, Chlor, Brom und Iod;
Phenylazo, 2-Napthylazo, 2-Pyridylazo und 2-Pyrimidylazo;
Cyclopropyl, Cyclobutyl, Cyclopentyl, 2- und 3-Methylcyclopentyl, 2- und 3-Ethylcyclopentyl, Cyclohexyl, 2-, 3- und 4-Methylcyclohexyl, 2-, 3- und 4-Ethylcyclohexyl, 3- und 4-Propylcyclohexyl, 3- und 4-Isopropylcyclohexyl, 3- und 4-Butylcyclohexyl, 3- und 4-sec.-Butylcyclohexyl, 3- und 4-tert.-Butylcyclohexyl, Cycloheptyl, 2-, 3- und 4-Methyl-cycloheptyl, 2-, 3- und 4-Ethylcycloheptyl, 3- und 4-Propylcycloheptyl, 3- und 4-Iso-propylcycloheptyl, 3- und 4-Butylcycloheptyl, 3- und 4-sec.-Butylcycloheptyl, 3-und 4-tert.-Butylcycloheptyl, Cyclooctyl, 2-, 3-, 4- und 5-Methylcyclooctyl, 2-, 3-, 4- und 5-Ethylcyclooctyl und 3-, 4- und 5-Propylcyclooctyl; 3- und 4-Hydroxycyclohexyl, 3- und 4-Nitrocyclohexyl und 3- und 4-Chlorcyclohexyl;
1-, 2- und 3-Cyclopentenyl, 1-, 2-, 3- und 4-Cyclohexenyl, 1-, 2- und 3-Cycloheptenyl und 1-, 2-, 3- und 4-Cyclooctenyl;
2-Dioxanyl, 1-Morpholinyl, 1-Thiomorpholinyl, 2- und 3-Tetrahydrofuryl, 1-, 2- und 3-Pyrrolidinyl, 1-Piperazyl, 1-Diketopiperazyl und 1-, 2-, 3- und 4-Piperidyl;
Phenyl, 2-Naphthyl, 2- und 3-Pyrryl, 2-, 3- und 4-Pyridyl, 2-, 4- und 5-Pyrimidyl, 3-, 4-und 5-Pyrazolyl, 2-, 4- und 5-Imidazolyl, 2-, 4- und 5-Thiazolyl, 3-(1,2,4-Triazyl), 2-(1,3,5-Triazyl), 6-Chinaldyl, 3-, 5-, 6- und 8-Chinolinyl, 2-Benzoxazolyl, 2-Benzothiazolyl, 5-Benzothiadiazolyl, 2- und 5-Benzimidazolyl und 1- und 5-Isochinolyl;
1-, 2-, 3-, 4-, 5-, 6- und 7-Indolyl, 1-, 2-, 3-, 4-, 5-, 6- und 7-Isoindolyl, 5-(4-Methyliso-indolyl), 5-(4-Phenylisoindolyl), 1-, 2-, 4-, 6-, 7- und 8-(1,2,3,4-Tetrahydroisochinolinyl), 3-(5-Phenyl)-(1,2,3,4-tetrahydroisochinolinyl), 5-(3-Dodecyl-(1,2,3,4-tetrahydroiso-chinolinyl), 1-, 2-, 3-, 4-, 5-, 6-, 7- und 8-(1,2,3,4-Tetrahydrochinolinyl) und 2-, 3-, 4-, 5-, 6-, 7- und 8-Chromanyl, 2-, 4- und 7-Chinolinyl, 2-(4-Phenylchinolinyl) und 2-(5-Ethyl-chinolinyl);
2-, 3- und 4-Methylphenyl, 2,4-, 3,5- und 2,6-Dimethylphenyl, 2,4,6-Trimethylphenyl, 2-, 3- und 4-Ethylphenyl, 2,4-, 3,5- und 2,6-Diethylphenyl, 2,4,6-Triethylphenyl, 2-, 3- und 4-Propylphenyl, 2,4-, 3,5- und 2,6-Dipropylphenyl, 2,4,6-Tripropylphenyl, 2-, 3- und 4-Isopropylphenyl, 2,4-, 3,5- und 2,6-Diisopropylphenyl, 2,4,6-Triisopropylphenyl, 2-, 3-und 4-Butylphenyl, 2,4-, 3,5- und 2,6-Dibutylphenyl, 2,4,6-Tributylphenyl, 2-, 3- und 4-Isobutylphenyl, 2,4-, 3,5- und 2,6-Diisobutylphenyl, 2,4,6-Triisobutylphenyl, 2-, 3- und 4-sec.-Butylphenyl, 2,4-, 3,5- und 2,6-Di-sec.-butylphenyl und 2,4,6-Tri-sec.-butyl-phenyl; 2-, 3- und 4-Methoxyphenyl, 2,4-, 3,5- und 2,6-Dimethoxyphenyl, 2,4,6-Tri-methoxyphenyl, 2-, 3- und 4-Ethoxyphenyl, 2,4-, 3,5-und 2,6-Diethoxyphenyl, 2,4,6-Triethoxyphenyl, 2-, 3- und 4-Propoxyphenyl, 2,4-, 3,5-und 2,6-Dipropoxyphenyl, 2-, 3- und 4-Isopropoxyphenyl, 2,4- und 2,6-Diisopropoxyphenyl und 2-, 3- und 4-Butoxy-phenyl; 2-, 3- und 4-Chlorphenyl und 2,4-, 3,5- und 2,6-Dichlorphenyl; 2-, 3- und 4-Hydroxyphenyl und 2,4-, 3,5- und 2,6-Dihydroxyphenyl; 2-, 3- und 4-Cyanophenyl; 3- und 4-Carboxyphenyl; 3- und 4-Carboxamidophenyl, 3- und 4-N-Methylcarboxamido-phenyl und 3- und 4-N-Ethylcarboxamidophenyl; 3- und 4-Acetylaminophenyl, 3- und 4-Propionylaminophenyl und 3- und 4-Buturylaminophenyl; 3- und 4-N-Phenylaminophenyl, 3- und 4-N-(o-Tolyl)aminophenyl, 3- und 4-N-(m-Tolyl)aminophenyl und 3- und 4-(p-Tolyl)aminophenyl; 3- und 4-(2-Pyridyl)aminophenyl, 3- und 4-(3-Pyridyl)aminophenyl, 3- und 4-(4-Pyridyl)aminophenyl, 3- und 4-(2-Pyrimidyl)aminophenyl und 4-(4-Pyrimidyl)aminophenyl;
4-Phenylazophenyl, 4-(1-Naphthylazo)phenyl, 4-(2-Naphthylazo)phenyl, 4-(4-Naphthyl-azo)phenyl,4-(2-Pyriylazo)phenyl, 4-(3-Pyridylazo)phenyl, 4-(4-Pyridylazo)phenyl, 4-(2-Pyrimidylazo)phenyl, 4-(4-Pyrimidylazo)phenyl und 4-(5-Pyrimidylazo)phenyl;
Phenoxy, Phenylthio, 2-Naphthoxy, 2-Naphthylthio, 2-, 3- und 4-Pyridyloxy, 2-, 3- und 4-Pyridylthio, 2-, 4- und 5-Pyrimidyloxy und 2-, 4- und 5-Pyrimidylthio.

Bevorzugte fluorhaltige Reste R^{a} und R^{b} sind die Folgenden:

2,2,2-Trifluorethyl, 2,2,3,3,3-Pentafluorpropyl, 2,2-Difluorethyl, 2,2,3,3,4,4,4-Heptafluorbutyl, 2,2,3,3,3-Pentafluorpropyl, 1H, 1H-Pentadecafluoroctyl, 3-Brom-3,3-difluorpropyl, 3,3,3-Trifluorpropyl, 3,3,3-Trifluorpropyl, 1H, 1H,2H,2H-Perfluorodecyl, 3-(Perfluoroctyl)propyl, 4,4-Difluorbutyl-, 4,4,4-Trifluorbutyl, 5,5,6,6,6-Pentafluorhexyl, 2,2-Difluorpropyl, 2,2,2-Trifluor-1-phenylethylamino, 1-Benzyl-2,2,2-trifluorethyl, 2-Brom-2,2-difluorethyl, 2,2,2-Trifluor-1-pyridin-2-ylethyl, 2,2-Difluorpropyl, 2,2,2-Trifluor-1-(4-methoxyphenyl)ethylamino, 2,2,2-Trifluor-1-phenylethyl, 2,2-Difluor-1-phenylethyl, 1-(4-Brom-phenyl)-2,2,2-trifluorethyl, 3-Brom-3,3-difluorpropyl, 3,3,3-Trifluorpropylamin, 3,3,3-Trifluor-n-propyl, 1 H, 1H,2H,2H-Perfluordecyl, 3-(Perfluorctyl)propyl, Pentafluorphenyl, 2,3,5,6-Tetrafluorphenyl, 4-Cyano-(2,3,5,6)-Tetrafluorphenyl, 4-Carboxy-2,3,5,6-Tetrafluorphenyl, 2,4-Difluorphenyl, 2,4,5-Trifluorphenyl, 2,4,6-Trifluorphenyl, 2,5-Difluorphenyl, 2-Fluor-5-Nitrophenyl, 2-Fluor-5-trifluormethylphenyl, 2-Fluor-5-methylphenyl, 2,6-Difluorphenyl, 4-Carboxamido-2,3,5,6-tetrafluorphenyl, 2-Brom-4,6-difluorphenyl, 4-Brom-2-fluorphenyl, 2,3-Difluorphenyl, 4-Chlor-2-fluorphenyl, 2,3,4-Trifluorphenyl, 2-Fluor-4-iodphenyl, 4-Brom-2,3,5,6-tetrafluorphenyl, 2,3,6-Trifluorphenyl, 2-Brom-3,4,6-trifluorphenyl, 2-Brom-4,5,6-trifluorphenyl, 4-Brom-2,6-difluorphenyl, 2,3,4,5-Tetrafluorphenyl, 2,4-Difluor-6-nitrophenyl, 2-Fluor-4-nitrophenyl, 2-Chlor-6-fluorphenyl, 2-Fluor-4-methylphenyl, 3-Chlor-2,4-difluorphenyl, 2,4-Dibrom-6-fluorphenyl, 3,5-Dichlor-2,4-difluorphenyl, 4-Cyano-1-Fluorphenyl, 1-Chlor-4-fluorphenyl, 2-Fluor-3-trifluormethylphenyl, 2-Trifluormethyl-6-fluorphenyl, 2,3,4,6-Tetrafluorphenyl, 3-Chlor-2-fluorphenyl, 5-Chlor-2-fluorphenyl, 2-Brom-4-chlor-6-fluorphenyl, 2,3-Dicyano-4,5,6-trifluorphenyl, 2,4,5-Trifluor-3-carboxyphenyl, 2,3,4-Trifluor-6-carboxyphenyl, 2,3,5-Trifluorphenyl, 4-Trifluormethyl2,3,5,6-tetrafluorphenyl, 1-Fluor-5-carboxyphenyl, 2-Chlor-4,6-Difluorphenyl, 6-Brom-3-chlor-2,4-difluorphenyl, 2,3,4-Trifluor-6-nitrophenyl, 2,5-Difluor-4-cyanophenyl, 2,5-Difluor-4-trifluormethylphenyl, 2,3-Difluor-6-nitrophenyl, 4-Trifluormethyl-2,3-difluorphenyl, 2-Brom-4,6-difluorphenyl, 4-Brom-2-fluorphenyl, 2-Nitrotetrafluorphenyl, 2,2',3,3',4',5,5',6,6'-Nonafluorbiphenyl, 2-Nitro-3,5,6-trifluorphenyl, 2-Brom-6-fluorphenyl, 4-Chlor-2-fluor-6-iodphenyl, 2-Fluor-6-carboxyphenyl, 2,4-Difluor-3-trifluorphenyl, 2-Fluor-4-trifluorphenyl, 2-Fluor-4-carboxyphenyl, 4-Brom-2,5-difluorphenyl, 2,5-Dibrom-3,4,6-trifluorphenyl, 2-Fluor-5-methylsulphonylpenyl, 5-Brom-2-fluorphenyl, 2-Fluor-4-hydroxymethylphenyl, 3-Fluor-4-brommethylphenyl, 2-Nitro-4-trifluormethylphenyl, 4-Trifluormethylphenyl, 2-Brom-4-trifluormethylphenyl, 2-Brom-6-Chlor-4-(trifluormethyl)phenyl, 2-Chlor-4-trifluormethylphenyl, 3-Nitro-4-(trifluormethyl)phenyl, 2,6-Dichlor-4-(trifluormethyl)phenyl, 4-Trifluorphenyl, 2,6-Dibrom-4-(trifluormethyl)phenyl, 4-Trifluormethyl-2,3,5,6-tetrafluorphenyl, 3-Fluor-4-trifluormethylphenyl, 2,5-Difluor-4-trifluormethylphenyl, 3,5-Difluor-4-trifluormethylphenyl, 2,3-Difluor-4-trifluormethylphenyl, 2,4-Bis(trifluormethyl)phenyl, 3-Chlor-4-trifluormethylphenyl, 2-Brom-4,5-di(trifluormethyl)phenyl, 5-Chlor-2-nitro-4-(trifluormethyl)phenyl, 2,4,6-Tris(trifluormethyl)phenyl, 3,4-Bis(trifluormethyl)phenyl, 2-Fluor-3-trifluormethylphenyl, 2-lod-4-trifluormethylphenyl, 2-Nitro-4,5-bis(trifluormethyl)phenyl, 2-Methyl-4-(trifluormethyl)phenyl, 3,5-Dichlor-4-(trifluormethyl)phenyl, 2,3,6-Trichlor-4-(trifluormethyl)phenyl, 4-(Trifluormethyl)benzyl, 2-Fluor-4-(trifluormethyl)benzyl, 3-Fluor-4-(trifluormethyl)benzyl, 3-Chlor-4-(trifluormethyl)benzyl, 4-Fluorphenethyl, 3-(Trifluormethyl)phenethyl, 2-Chlor-6-fluorphenethyl, 2,6-Dichlorphenethyl, 3-Fluorphenethyl, 2-Fluorphenethyl, (2-Trifluormethyl)phenethyl, 4-Fluorphenethyl, 3-Fluorphenethyl, 4-Trifluormethylphenethyl, 2,3-Difluorphenethyl, 3,4-Difluorphenethyl, 2,4-Difluorphenethyl, 2,5-Difluorphenethyl, 3,5-Difluorphenethyl, 2,6-Difluorphenethyl,4-(4-Fluorphenyl)phenethyl, 3,5-Di(trifluormethyl)phenethyl, Pentafluorphenethyl, 2,4-Di(trifluormethyl)phenethyl, 2-Nitro-4-(trifluormethyl)phenethyl, (2-Fluor-3-trifluormethyl)phenethyl, (2-Fluor-5-trifluormethyl)phenethyl, (3-Fluor-5-trifluormethyl)phenethyl, (4-Fluor-2-trifluormethyl)phenethyl, (4-Fluor-3-trifluormethyl)phenethyl, (2-Fluor-6-trifluormethyl)phenethyl, (2,3,6-Trifluor)phenethyl, (2,4,5-Trifluor)phenethyl, (2,4,6-Trifluor)phenethyl, (2,3,4-Trifluor)phenethyl, (3,4,5-Trifluor)phenethyl, (2,3,5-Trifluor)phenethyl, (2-Chlor-5-fluor)phenethyl, (3-Fluor-4-trifluormethyl)phenethyl, (2-Chlor-5-trifluormethyl)phenethyl, (2-Fluor-3-chlor-5-trifluormethyl)phenethyl, (2-Fluor-3-chlor)phenethyl, (4-Fluor-3-chlor)phenethyl, (2-Fluor-4-chlor)phenethyl, (2,3-Difluor-4-methyl)phenethyl-, 2,6-Difluor-3-chlorphenethyl, (2,6-Difluor-3-methyl)phenethyl, (2-Trifluormethyl-5-chlor)phenethyl, (6-Chlor-2-fluor-5-methyl)phenethyl, (2,4-Dichlor-5-fluor)phenethyl, 5-Chlor-2-fluorphenethyl, (2,5-Difluor-6-chlor)phenethyl, (2,3,4,5-Tetrafluor)phenethyl, (2-Fluor-4-trifluormethyl)phenethyl, 2,3-(Difluor-4-trifluormethyl)phenethyl, (2,5-Di(trifluormethyl))phenethyl, 2-Fluor-3,5-dibromphenethyl, (3-Fluor-4-nitro)phenethyl, (2-Brom-4-trifluormethyl)phenethyl, 2-(Brom-5-fluor)phenethyl, (2,6-Difluor-4-brom)phenethyl, (2,6-Difluor-4-chlor)phenethyl, (3-Chlor-5-fluor)phenethyl, (2-Brom-5-trifluormethyl)phenethyl und dergleichen.

Des Weiteren bevorzugt sind Verbindungen der Formel I, wobei n für 3 steht und 1, 2, 3 oder 4 der Reste Rⁿ¹, Rⁿ², Rⁿ³ und Rⁿ⁴ für Fluor stehen.

Des Weiteren bevorzugt sind Verbindungen der Formel 1, wobei n für 4 steht und 1, 2, 3, 4, 5 oder 6 der Reste Rⁿ¹, Rⁿ², Rⁿ³ und Rⁿ⁴ für Fluor stehen.

Rylentetracarbonsäuredianhydride werden im Folgenden als Verbindungen I.A bezeichnet. Rylentetracarbonsäurediimide werden im Folgenden als Verbindungen I.B bezeichnet, wobei Verbindungen I.Ba keine zusätzliche verbrückende Gruppe X aufweisen und Verbindungen I.Bb eine solche zusätzliche verbrückende Gruppe X aufweisen.

Bezüglich der Bedeutungen der Substituenten R^{a} und R^{b} in den zuvor genannten Verbindungen wird auf die eingangs gemachten Ausführungen Bezug genommen.

Besonders bevorzugt steht wenigstens einer der Reste R^{a} und R^{b} für einen elektronenziehend substituierten Rest.

In einer speziellen Ausführungsform steht wenigstens einer der Reste R^{a} und R^{b} für einen ein- oder mehrfach mit Fluor substituierten Rest. Besonders bevorzugt stehen sowohl R^{a} als auch R^{b} für einen ein- oder mehrfach mit Fluor substituierten Rest. Bezüglich geeigneter fluorierter Reste wird ebenfalls auf die eingangs gemachten Ausführungen Bezug genommen.

In einer weiteren speziellen Ausführungsform sind die Reste R^{a} und R^{b} gleich.

Eine weitere erfindungsgemäße Ausführungsform sind Verbindungen der allgemeinen Formeln I.Bb 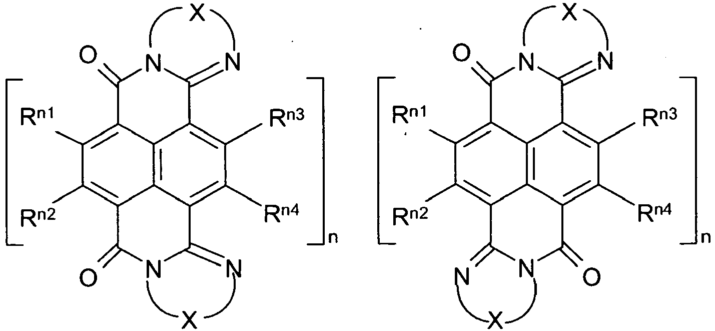 wobei
n und Rⁿ¹, Rⁿ², Rⁿ³ und Rⁿ⁴ die zuvor angegebenen Bedeutungen besitzen und
X für eine zweiwertige verbrückende Gruppe mit 2 bis 5 Atomen zwischen den flankierenden Bindungen steht.

Bevorzugt stehen die verbrückenden Gruppen X gemeinsam mit der N-C=N-Gruppe, an die sie gebunden sind für einen 5- bis 8-gliedrigen Heterocyclus steht, der gegebenenfalls ein-, zwei- oder dreifach mit Cycloalkyl, Heterocycloalkyl, Aryl und/oder Hetaryl anelliert ist, wobei die anellierten Gruppen unabhängig voneinander je einen, zwei, drei oder vier Substituenten, ausgewählt unter Alkyl, Alkoxy, Cycloalkyl, Aryl, Halogen, Hydroxy, Thiol, COOH, Carboxylat, SO₃H, Sulfonat, NE¹E², Alkylen-NE¹E³, Nitro und Cyano tragen können, wobei E¹ und E² unabhängig voneinander für Wasserstoff, Alkyl, Cycloalkyl, Heterocycloalkyl, Aryl oder Hetaryl stehen, und/oder X einen, zwei oder drei Substituenten, die ausgewählt sind unter gegebenenfalls substituiertem Alkyl, gegebenenfalls substituiertem Cycloalkyl und gegebenenfalls substituiertem Aryl, aufweisen kann und/oder X durch 1, 2 oder 3 gegebenenfalls substituierte Heteroatome unterbrochen sein kann.

Vorzugsweise sind die verbrückenden Gruppen X ausgewählt unter Gruppen der Formeln (III.a) bis (III.d) 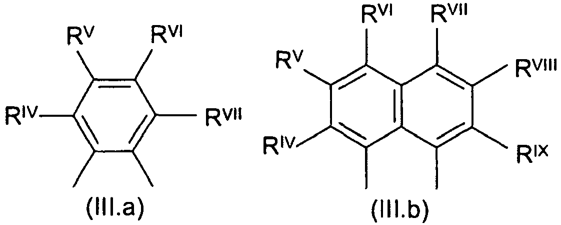 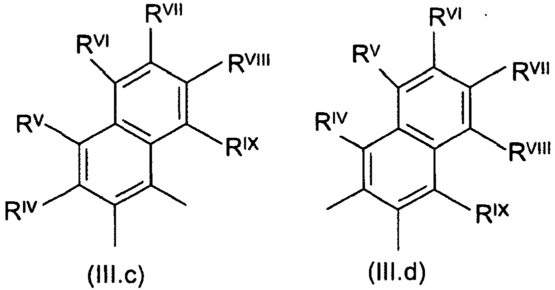 worin
R^{IV}, R^{V}, R^{VI}, R^{VII}, R^{VIII} und R^{IX} unabhängig voneinander für Wasserstoff, Alkyl, Alkoxy, Cycloalkyl, Cycloalkoxy, Heterocycloalkyl, Heterocycloalkoxy, Aryl, Aryloxy, Hetaryl, Hetaryloxy, Halogen, Hydroxy, Thiol, COOH, Carboxylat, SO₃H, Sulfonat, NE¹E², Alkylen-NE¹E³, Nitro, Alkoxycarbonyl, Acyl oder Cyano stehen, wobei E¹ und E² unabhängig voneinander für Wasserstoff, Alkyl, Cycloalkyl, Heterocycloalkyl, Aryl oder Hetaryl stehen.

In einer speziellen Ausführung stehen in den Gruppen (III.a) bis (III.d) die Reste R^{IV}, R^{V}, R^{VI}, R^{VII}, R^{VIII} und R^{IX} für Wasserstoff.

Im Folgenden werden einige besonders bevorzugte erfindungsgemäße Verbindungen wiedergegeben:

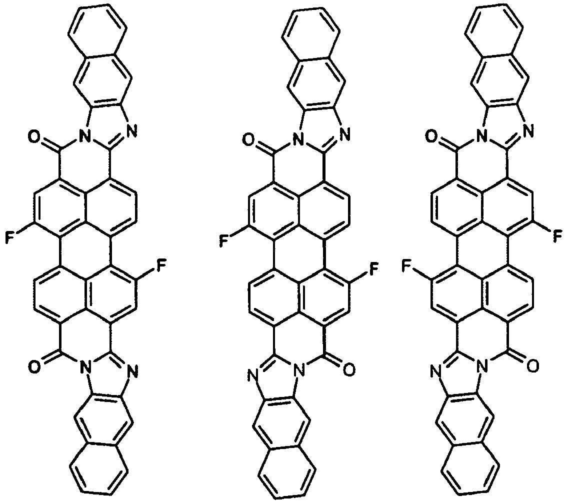

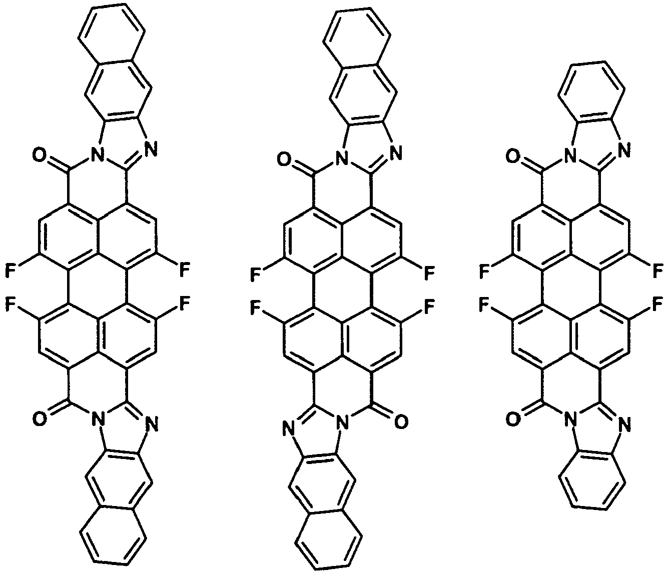

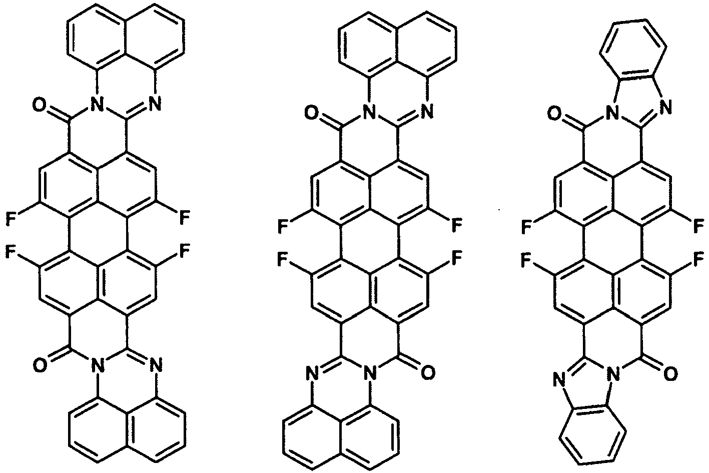

Die Herstellung der erfindungsgemäßen Verbindungen der allgemeinen Formel I kann ausgehen von bekannten Verbindungen mit gleichem Rylengrundgerüst, die anstelle von Fluoratomen andere Halogenatome, wie Br und Cl, insbesondere Br tragen. Dabei wurde speziell gefunden, dass man in Abhängigkeit von der Wahl der Reaktionsbedingungen Rylenverbindungen, die wenigstens ein Bromatom als Substituenten am Aromatengerüst aufweisen,einer vollständigen Substitution der Halogenatome durch Fluoratome, oder einer teilweisen Substitution der Halogenatome durch Fluoratome bei gleichzeitiger teilweiser Substitution Wasserstoff unterziehen kann.

Ein weiterer Gegenstand der Erfindung ist daher ein Verfahren zur Herstellung von Verbindungen der Formel I 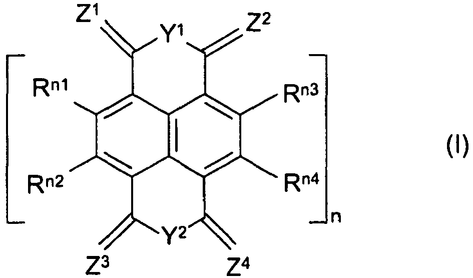 wobei n, Rⁿ¹, Rⁿ², Rⁿ³ und Rⁿ⁴, Y¹, Y², Z¹, Z², Z³ und Z⁴ wie zuvor definiert sind, bei dem man eine Verbindung der Formel I, bei der wenigstens einer der Reste Rⁿ¹, Rⁿ², Rⁿ³ und Rⁿ⁴, R¹, R², R³ und R⁴ für Cl oder Br steht einer Umsetzung mit einem Alkalifluorid unter wasserfreien Bedingungen unterzieht.

Unter "im Wesentlichen wasserfreien Bedingungen" wird im Rahmen der Erfindung ein Gesamtwassergehalt, bezogen auf auf alle an der Reaktion beteiligten Komponenten (Edukte, Lösungsmittel, Komplexbildner, etc.), von höchstens 2 Gew.-%, bevorzugt von höchstens 1 Gew.-%, insbesondere von höchstens 0,1 Gew.-%, verstanden. Zur Erzielung der wasserfreien Reaktionsbedingungen können die an der Umsetzung beteiligten Komponenten einer Trocknung nach üblichen, dem Fachmann bekannten Verfahren unterzogen werden.

Zur Erzielung bestimmter gemischt halogenierter Verbindungen kann es vorteilhaft sein, die nach der Fluorierung erhaltenen Verbindungen einer Halogenierung mit elementarem Cl₂ und/oder Br₂ zu unterziehen.

Geeignete Verfahrensbedingungen zur aromatischen nucleophilen Substitution von Bromatomen oder Chloratomen durch Fluoratome (Halo-Dehalogenierung) sind prinzipiell bekannt. Geeignete Bedingungen zur Halo-Dehalogenierung sind z. B. in J. March, Advanced Organic Chemistry, 4. Auflage, Verlag John Wiley & Sons (1992), S. 659 sowie in der DE-A-32 35 526 beschrieben.

In einer ersten Ausführungsform handelt es sich bei der Umsetzung um einen Austausch der Bromatome durch Fluoratome, gegebenenfalls mit einer teilweisen Dehalogenierung. Zur Einfügung der Fluorgruppen wird vorzugsweise ein Alkalifluorid, insbesondere KF, NaF oder CsF, eingesetzt. Es werden vorzugsweise 1 bis 30 Äquivalente kaliumfluorid je Äquivalent Rylenverbindung eingesetzt.

Bevorzugte Lösungsmittel für den Halogenaustausch sind aprotisch polare Lösungsmittel, wie Dimethylformamid, N-Methylpyrrolidon, (CH₃)₂SO, Dimethylsulfon, N,N'-Dimethylimidazolidinon oder Sulfolan. Besonders bevorzugt wird Sulfolan als Lösungsmittel eingesetzt. Vorzugsweise werden die Lösungsmittel vor ihrem Einsatz einer Trocknung zur Entfernung von Wasser nach üblichen, dem Fachmann bekannten Methoden unterzogen.

Zum Halogenaustausch kann zusätzlich ein Komplexbildner, wie z. B. ein Kronenether eingesetzt werden. Dazu zählen z. B. [12]Krone-4, [15]Krone-5, [18]Krone-6, [21]Krone-7, [24]Krone-8, etc. Die Auswahl des Komplexbildners erfolgt nach seiner Befähigung zur Komplexierung der Alkalimetalle der zum Halogenaustausch eingesetzten Alkalihalogenide. Wird zur Einfügung der Fluorgruppen KF eingesetzt, so wird als Komplexbildner bevorzugt [18]Krone-6 eingesetzt. Es werden vorzugsweise 0,1 bis 10 Äquivalente Kronenether je Äquivalent Rylenverbindung eingesetzt.

Weitere geeignete Phasentransferkatalysatoren sind beispielsweise ausgewählt unter 2-Azaalleniumverbindungen, Carbophosphazeniumverbindungen, Aminophosphoniumverbindungen und Diphosphazeniumverbindungen. A. Pleschke, A. Marhold, M. Schneider, A. Kolomeitsev und G. V. Röschenthaler geben im Journal of Fluorine Chemistry 125, 2004, 1031-1038 eine Übersicht über weitere geeignete Phasentransferkatalysatoren. Auf die Offenbarung dieses Dokuments wird Bezug genommen. In einer bevorzugten Ausführung werden 2-Azaalleniumverbindungen, wie (N,N-Dimethylimidazolidino)-tetramethylguanidiniumchlorid eingesetzt. Die Einsatzmenge dieser Phasentransferkatalysatoren beträgt vorzugsweise 0,1 bis 20 Gew.-%, bevorzugt 1 bis 10 Gew.-%, bezogen auf das Gewicht der eingesetzten Rylenverbindung.

Es ist ebenfalls möglich, wenigstens einen Kronenether in Kombination mit wenigstens einem davon verschiedenen Phasentransferkatalysator einzusetzen.

Die Reaktionstemperaturen liegen vorzugsweise bei 100 bis 200 °C, besonders bevorzugt bei 120 bis 160 °C. Die Reaktionszeit liegt vorzugsweise bei 0,5 bis 48 Stunden.

Bei der Umsetzung mit Alkalifluoriden in einem wasserfreien, aprotischen, polaren Lösungsmittel kann neben einem Halogenaustausch im Allgemeinen auch zu einem gewissen Grad eine Dehalogenierung eintreten. Die dabei erhaltenen Gemische können im Folgenden einer Auftrennung unterzogen werden, bei Anhydriden und in organischen Lösungsmitteln schwer löslichen Imiden z. B. durch fraktionierte Kristallisation aus H₂SO₄. In organischen Lösungsmitteln besser lösliche Verbindungen lassen sich durch Umkristallisieren oder Säulenchromatographie auftrennen.

Es wurde gefunden, dass bei der Umsetzung mit Alkalifluoriden unter im Wesentliche wasserfreien Bedingungen auch eine Dehydrofluorierung erfolgen kann, d. h. zusätzlich zu dem/den Chlor- oder Bromatom(en) werden auch aromatische Wasserstoffatome durch Fluoratome substituiert.

In einer beispielhaften Ausführung des erfindungsgemäßen Verfahrens wird ein Dibromperylenbisimid oder ein Gemisch aus Dibromperylenbisimiden einem Halogenaustausch gemäß folgendem Schema unterzogen, wobei die entsprechenden Difluorperylenbisimide (I.Ba) erhalten werden.

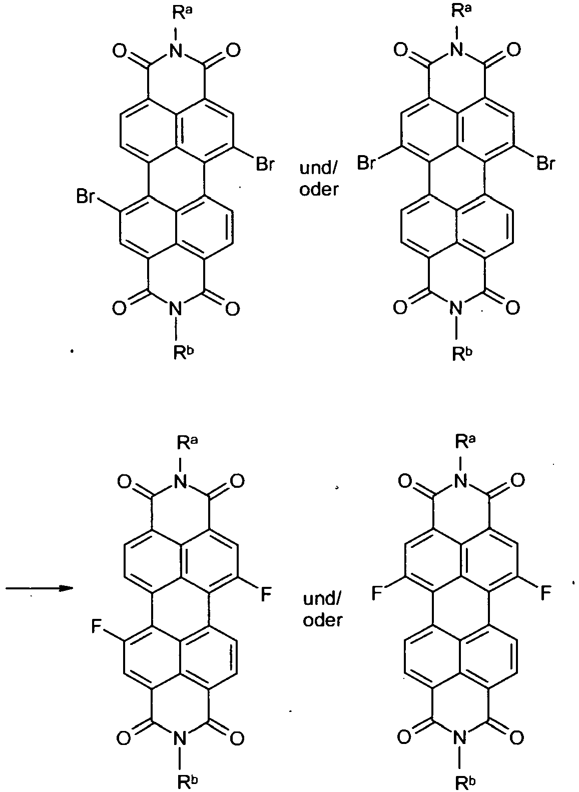

Dabei haben R^{a} und R^{b} die zuvor angegebenen Bedeutungen. Vorzugsweise haben R^{a} und R^{b} die gleiche Bedeutung und stehen beispielsweise beide für C₁-C₁₂-Alkyl, wie Methyl, Ethyl, n-Propyl, Isopropyl, n-Butyl, Isobutyl, sec.-Butyl, tert.-Butyl, n-Pentyl, n-Hexyl, n-Heptyl, n-Octyl, n-Nonyl, n-Decyl, n-Undecyl oder n-Dodecyl, Cycloalkyl, wie Cyclopentyl oder Cyclohexyl, oder Aryl, wie Phenyl, 2-, 3- und 4-Methylphenyl, 2,4-, 2,5-, 3,5- und 2,6-Dimethylphenyl, 2,4,6-Trimethylphenyl, 2-, 3- und 4-Ethylphenyl, 2,4-, 2,5-, 3,5- und 2,6-Diethylphenyl, 2,4,6-Triethylphenyl, 2-, 3- und 4-Propylphenyl, 2,4-, 2,5-, 3,5- und 2,6-Dipropylphenyl,etc.

Zur Einfügung der Fluorgruppen nach dieser Ausführung wird vorzugsweise ein Alkalifluorid, insbesondere KF, eingesetzt.

Bevorzugt wird zum Halogenaustausch nach dieser Ausführung ein Kronenether, beim Einsatz von KF speziell [18]Krone-6, oder ein anderer Phasentransferkatalysator, bevorzugt eine 2-Azaalleniumverbindungen, insbesondere (N,N-Dimethylimidazolidino)-tetramethylguanidiniumchlorid (CNC⁺), eingesetzt. Besonders bevorzugt wird Sulfolan als Lösungsmittel eingesetzt.

In einer weiteren beispielhaften Ausführung des erfindungsgemäßen Verfahrens wird ein Tetrachlorperylenbisimid einem Halogenaustausch gemäß folgendem Schema unterzogen, wobei die entsprechenden Tetrafluorperylenbisimide (I.Ba) erhalten werden.

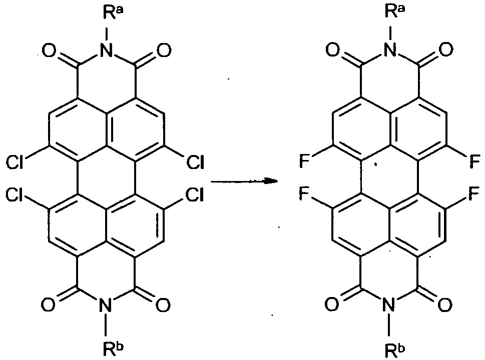

Dabei haben R^{a} und R^{b} die zuvor angegebenen Bedeutungen. Vorzugsweise haben R^{a} und R^{b} die gleiche Bedeutung und stehen beispielsweise beide für C₁-C₁₂-Alkyl, wie Methyl, Ethyl, n-Propyl, Isopropyl, n-Butyl, Isobutyl, sec.-Butyl, tert.-Butyl, n-Pentyl, n-Hexyl, n-Heptyl, n-Octyl, n-Nonyl, n-Decyl, n-Undecyl oder n-Dodecyl, oder Aryl, wie Phenyl, 2-, 3- und 4-Methylphenyl, 2,4-, 2,5-, 3,5- und 2,6-Dimethylphenyl, 2,4,6-Trimethylphenyl, 2-, 3- und 4-Ethylphenyl, 2,4-, 2,5-, 3,5- und 2,6-Diethylphenyl, 2,4,6-Triethylphenyl, 2-, 3- und 4-Propylphenyl, 2,4-, 2,5-, 3,5- und 2,6-Dipropylphenyl,etc.

Zur Einfügung der Fluorgruppen nach dieser Ausführung wird vorzugsweise ein Alkalifluorid, insbesondere KF, eingesetzt.

Zum Halogenaustausch nach dieser Ausführung kann ein Kronenether, beim Einsatz von KF speziell [18]Krone-6, eingesetzt werden. Bevorzugt wird eine 2-Azaalleniumverbindungen, insbesondere (N,N-Dimethylimidazolidino)-tetramethylguanidiniumchlorid (CNC⁺), eingesetzt. Besonders bevorzugt wird Sulfolan als Lösungsmittel eingesetzt.

Für einen Einsatz der Produkte als Halbleiter kann es von Vorteil sein, die Produkte einer weiteren Aufreinigung zu unterziehen. Dazu zählen beispielsweise säulenchromatographische Verfahren, wobei die Produkte z. B. in einem halogenierten Kohlenwasserstoff, wie Methylenchlorid oder einem Toluoll oder Petrolether/Essigester-Gemisch gelöst, einer Auftrennung bzw. Filtration an Kieselgel unterzogen werden. Des Weiteren ist eine Reinigung durch Sublimation oder Kristallisation möglich.

Ein weiterer Gegenstand der Erfindung ist ein Verfahren zur Herstellung von Verbindungen der Formel (I.Ba) 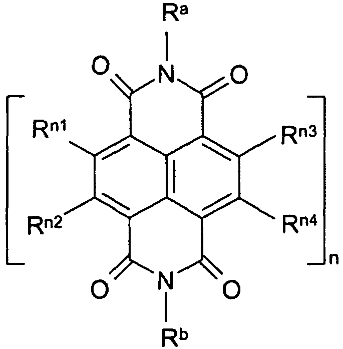 wobei n, Rⁿ¹, Rⁿ², Rⁿ³ und Rⁿ⁴ wie zuvor definiert sind, und
R^{a} und R^{b} unabhängig voneinander für Wasserstoff oder für unsubstituiertes oder substituiertes Alkyl, Alkenyl, Alkadienyl, Alkinyl, Cycloalkyl, Bicycloalkyl, Cycloalkenyl, Heterocycloalkyl, Aryl oder Heteroaryl stehen,
bei dem man
a1) ein Rylendianhydrid der Formel II, 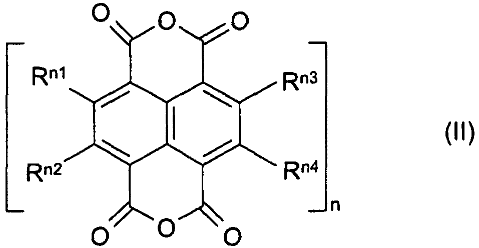 wobei wenigstens einer der Reste Rⁿ¹, Rⁿ², Rⁿ³ und Rⁿ⁴, R¹ für Cl oder Br steht und n für 3 oder 4 steht, einer Umsetzung mit einem Alkalifluorid unter im Wesentlichen wasserfreien Bedingungen, wie zuvor beschrieben, unterzieht,
b1) die in Schritt a1) erhaltene Verbindung einer Umsetzung mit einem Amin der Formel R^{a}-NH₂ und gegebenenfalls einem davon verschiedenen Amin der Formel R^{b}-NH₂ unterzieht.

Ein weiterer Gegenstand der Erfindung ist ein Verfahren zur Herstellung von Verbindungen der Formeln I.Bb 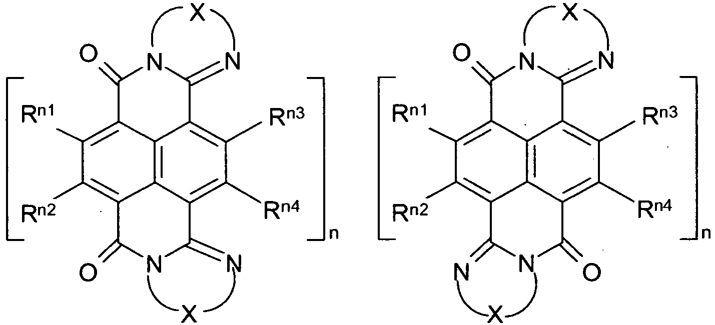 wobei n, X, Rⁿ¹, Rⁿ², Rⁿ³ und Rⁿ⁴ wie zuvor definiert sind, bei dem man
a2) ein Rylendianhydrid der Formel II, 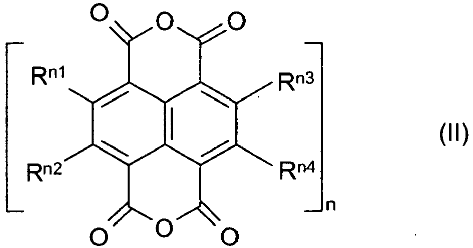 wobei wenigstens einer der Reste Rⁿ¹, Rⁿ², Rⁿ³ und Rⁿ⁴ für Cl oder Br steht und n für 2, 3 oder 4 steht, einer Umsetzung mit einem Alkalifluorid unter im Wesentlichen wasserfreien Bedingungen, wie zuvor beschrieben, unterzieht,
b2) die in Schritt a) erhaltene Verbindung einer Umsetzung mit einem Amin der Formel H₂N-X-NH₂ unterzieht.

### Schritte a1) und a2)

Geeignete Verfahrensbedingungen zur aromatischen nucleophilen Substitution von Bromatomen oder Chloratomen durch Fluoratome (sowie gegebenenfalls teilweise durch Wasserstoffatome) sind die zuvor beschriebenen, worauf hier Bezug genommen wird.

### Schritte b1) und b2)

Wird zur Imidisierung im Schritt b1) die in Schritt a1) erhaltene Verbindung einer Umsetzung mit einem Amin der Formel R^{a}-NH₂ und gegebenenfalls einem Amin der Formel R^{b}-NH₂ unterzogen, so resultiert wenigstens eine Verbindung der allgemeinen Formel (I.Ba) 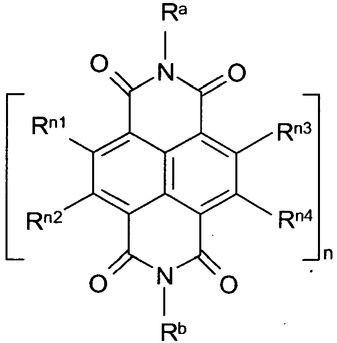 wobei R^{b} auch die gleiche Bedeutung wie R^{a} besitzen kann (falls zur Imidisierung nur ein Amin der Formel R^{a}-NH₂ eingesetzt wird.

Wird zur Imidisierung im Schritt b2) die in Schritt a2) erhaltene Verbindung einer Umsetzung mit einem Amin der Formel H₂N-X-NH₂ unterzogen, wobei X für eine zweiwertige verbrückende Gruppe mit 2 bis 5 Atomen zwischen den flankierenden Bindungen steht, so resultiert wenigstens eine Verbindung der allgemeinen Formeln (I.Bb)

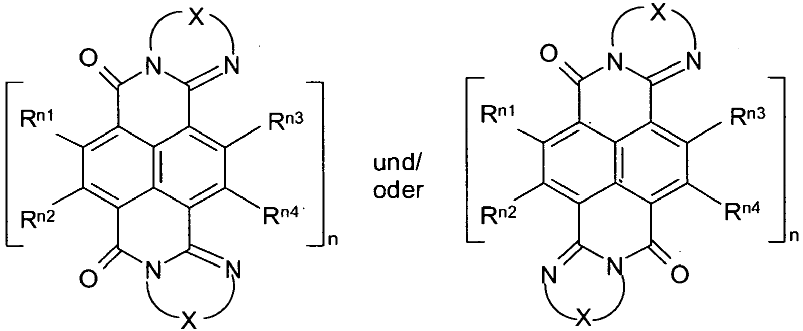

Die Imidierung der Carbonsäureanhydridgruppen in den Reaktionsschritten b1) und b2) ist prinzipiell bekannt und z. B. in der DE 10 2004 007 382 A1 beschrieben. Vorzugsweise erfolgt die Umsetzung des Dianhydrids mit dem primären Amin in Gegenwart eines aromatischen Lösungsmittels, wie Toluol, Xylol, Mesitylen, Phenol oder eines polaren aprotischen Lösungsmittels. Geeignete polare aprotische Lösungsmittel sind Stickstoffheterocyclen, wie Pyridin, Pyrimidin, Chinolin, Isochinolin, Chinaldin, N-Methylpiperidin, N-Methylpiperidon und N-Methylpyrrolidon. Zur Umsetzung mit einem aromatischen Diamin der Formel H₂N-X-NH₂ wird vorzugsweise ein Stickstoffheterocyclus oder Phenol als Lösungsmittel eingesetzt. Geeignete Katalysatoren sind die im Folgenden genannten. Bei Verwendung von Phenol als Lösungsmittel wird als Katalysator vorzugsweise Piperazin eingesetzt.

Die Reaktion kann in Gegenwart eines Imidierungskatalysators vorgenommen werden. Als Imidierungskatalysatoren eignen sich organische und anorganische Säuren, z. B. Ameisensäure, Essigsäure, Propionsäure und Phosphorsäure. Geeignete Imidierungskatalysatoren sind weiterhin organische und anorganische Salze von Übergangsmetallen, wie Zink, Eisen, Kupfer und Magnesium. Dazu zählen z. B. Zinkacetat, Zinkpropionat, Zinkoxid, Eisen(II)acetat, Eisen(III)chlorid, Eisen(II)sulfat, Kupfer(II)-acetat, Kupfer(II)oxid und Magnesiumacetat. Der Einsatz eines Imidierungskatalysators erfolgt vorzugsweise bei der Umsetzung aromatischer Amine und ist im Allgemeinen auch zur Umsetzung cycloaliphatischer Amine vorteilhaft. Bei der Umsetzung aliphatischer Amine, insbesondere kurzkettiger aliphatischer Amine, kann in der Regel auf den Einsatz eines Imidierungskatalysators verzichtet werden. Die Einsatzmenge des Imidierungskatalysators beträgt vorzugsweise 5 bis 80 Gew.-%, besonders bevorzugt 10 bis 75 Gew.-%, bezogen auf das Gesamtgewicht der zu amidierenden Verbindung.

Vorzugsweise liegt das Molmengenverhältnis von Amin zu Dianhydrid bei etwa 2:1 bis 10:1, besonders bevorzugt 2:1 bis 4:1, z. B. 2,2:1 bis 3:1.

Die zuvor als Imidierungskatalysatoren genannten organischen Säuren eignen sich auch als Lösungsmittel.

Die Reaktionstemperatur beträgt in den Schritten b1) bzw. b2) im Allgemeinen Umgebungstemperatur bis 200 °C, vorzugsweise 40 bis 160 °C. Die Umsetzung aliphatischer und cycloaliphatischer Amine erfolgt vorzugsweise in einem Temperaturbereich von etwa 60 °C bis 100 °C. Die Umsetzung aromatischer Amine erfolgt vorzugsweise in einem Temperaturbereich von etwa 120 bis 160 °C.

Vorzugsweise erfolgt die Umsetzung in den Reaktionsschritte b1) bzw. b2) unter einer Schutzgasatmosphäre, wie z. B. Stickstoff.

Die Reaktionsschritte b1) bzw. b2) können bei Normaldruck oder gewünschtenfalls unter erhöhtem Druck erfolgen. Ein geeigneter Druckbereich liegt im Bereich von etwa 0,8 bis 10 bar. Vorzugsweise beim Einsatz flüchtiger Amine (Siedepunkt etwa ≤ 180 °C) bietet sich der Einsatz unter erhöhtem Druck an.

Das bei der Reaktion in den Schritten b1) bzw. b2) entstehende Wasser kann durch Destillation nach dem Fachmann bekannten Verfahren abgetrennt werden.

In der Regel können die im Reaktionsschritt b1) bzw. b2) erhaltenen Diimide ohne weitere Reinigung verwendet werden. Für einen Einsatz der Produkte als Halbleiter kann es jedoch von Vorteil sein, die Produkte einer weiteren Aufreinigung zu unterziehen. Dazu zählen beispielsweise säulenchromatographische Verfahren, wobei die Produkte vorzugsweise in einem halogenierten Kohlenwasserstoff, wie Methylenchlorid, gelöst, einer Auftrennung bzw. Filtration an Kieselgel unterzogen werden.

Überraschenderweise wurde gefunden, dass sich fluorierte Diimide der Formel (I.Ba), 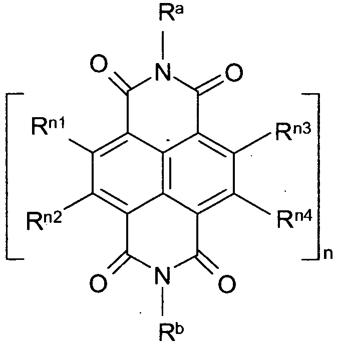 wobei einer oder mehrere (z. B. 2, 3 oder 4) der Reste Rⁿ¹, Rⁿ², Rⁿ³ und Rⁿ⁴ für Fluor stehen und die übrigen Reste Rⁿ¹, Rⁿ², Rⁿ³ und Rⁿ⁴ für Wasserstoff stehen, einer Umsetzung der Imidgruppen unter Bildung der entsprechenden Dianhydride (I.A) (einer Hydrolyse) unterziehen lassen. Überraschenderweise sind die Fluorgruppen dabei auch gegenüber basischen Hydrolysebedingungen stabil. Somit lassen sich fluorierte Dianhydride herstellen, die ansonsten nur schwer zugängig sind. Diese fluorierten Dianhydride können dann ihrerseits als Edukte für die Herstellung ansonsten nicht oder nur schwer zugängiger Diimide dienen.

Ein weiterer Gegenstand der Erfindung ist daher ein Verfahren zur Herstellung von Dianhydriden der Formel (I.A) 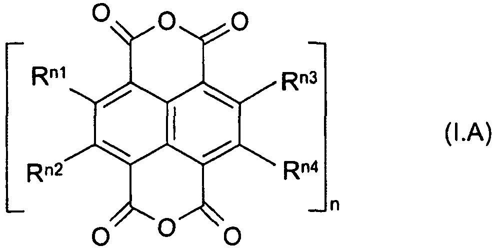 wobei
n für 3 oder 4 steht,
wenigstens einer der Reste Rⁿ¹, Rⁿ², Rⁿ³ und Rⁿ⁴ für Fluor steht,
die übrigen Reste Rⁿ¹, Rⁿ², Rⁿ³ und Rⁿ⁴ für Wasserstoff stehen,
bei dem man ein Diimid der Formel (I.Ba), 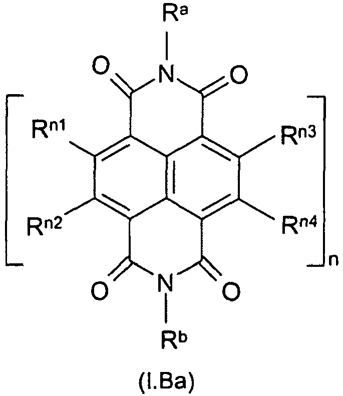 wobei R^{a} und R^{b} unabhängig voneinander für Wasserstoff oder für unsubstituiertes oder substituiertes Alkyl, Alkenyl, Alkadienyl, Alkinyl, Cycloalkyl, Bicycloalkyl, Cycloalkenyl, Heterocycloalkyl, Aryl oder Heteroaryl stehen,
einer zweistufigen Hydrolyse unterzieht, wobei die erste Stufe in Gegenwart einer Base und die zweite in Gegenwart einer Säure erfolgt.

In den Verbindungen der Formel (I.Ba) haben R^{a} und R^{b} vorzugsweise die gleiche Bedeutung und stehen beispielsweise beide für C₁-C₁₂-Alkyl, wie Methyl, Ethyl, n-Propyl, Isopropyl, n-Butyl, Isobutyl, sec.-Butyl, tert.-Butyl, n-Pentyl, n-Hexyl, n-Heptyl, n-Octyl, n-Nonyl, n-Decyl, n-Undecyl oder n-Dodecyl, oder Aryl, wie Phenyl, 2-, 3- und 4-Methylphenyl, 2,4-, 2,5-, 3,5- und 2,6-Dimethylphenyl, 2,4,6-Trimethylphenyl, 2-, 3- und 4-Ethylphenyl, 2,4-, 2,5-, 3,5- und 2,6-Diethylphenyl, 2,4,6-Triethylphenyl, 2-, 3- und 4-Propylphenyl, 2,4-, 2,5-, 3,5- und 2,6-Dipropylphenyl,etc.

Geeignete Basen für den ersten Schritt der Hydrolyse sind z. B. Alkalimetall- und Erdalkalimetallhydroxide, wie NaOH, KOH, Ca(OH)₂. Die Einsatzmenge der Base beträgt vorzugsweise 10 bis 500, besonders bevorzugt 30 bis 350 Mol-Äquivalente bezogen auf die zu hydrolysierende Verbindung.

Die Hydrolyse kann in Gegenwart von Wasser und/oder wenigstens einem Alkohol erfolgen. Geeignete Alkohole sind C₁- bis C₁₈-Alkanole, bevorzugt C₃- bis C₁₀-Alkanole, speziell C₄- bis C₆-Alkanole, wie Methanol, Ethanol, Propanol, Isopropanol, n-Butanol, tert.-Butanol, n-Pentanol oder n-Hexanol. Die Einsatzmenge des Alkohols beträgt vorzugsweise 10 bis 500 ml, insbesondere 30 bis 250 ml, pro Gramm zu hydrolysierender Verbindung.

Gewünschtenfalls kann die Hydrolyse zusätzlich in Gegenwart eines das Ausgangsdiimid solubilisierenden Lösungsmittels erfolgen. Geeignet sind aromatische Lösungsmittel, wie Toluol, Xylol, Mesitylen, Phenol oder polar aprotische Lösungsmittels, wie Pyridin, Pyrimidin, Chinolin, Isochinolin, Chinaldin und N-Methylpiperidin.

Für den ersten Schritt der Hydrolyse liegt die Reaktionstemperatur im Allgemeinen in einem Bereich von Umgebungstemperatur bis 200 °C, vorzugsweise 40 bis 180 °C, besonders bevorzugt 60 bis 150 °C.

Im Allgemeinen kann das Produkt des ersten Hydrolyseschritts ohne Isolierung für den zweiten Hydrolyseschritt eingesetzt werden. Dazu wird in der Regel eine ausreichende Menge einer Säure zu dem Reaktionsgemisch gegeben. Geeignete Säuren sind Mineralsäuren, wie HCl, H₂SO₄, H₃PO₄, und organische Säuren, vorzugsweise C₁- bis C₆-Carbonsäuren, wie Essigsäure, Propionsäure, etc.

Für den zweiten Schritt der Hydrolyse liegt die Reaktionstemperatur im Allgemeinen in einem Bereich von Umgebungstemperatur bis 200 °C, vorzugsweise 50 bis 180 °C, besonders bevorzugt 80 bis 150 °C.

Die Isolierung und gegebenenfalls Reinigung des erhaltenen Dianhydrids (I.A) erfolgt nach üblichen, dem Fachmann bekannten Verfahren. Dazu zählt z. B. das Ausfällen mit einem Fällungsmittel, wie einer ausreichenden Menge Wasser, Filtrieren und Trocknen.

Die fluorierten Dianhydride der Formel (I.A) 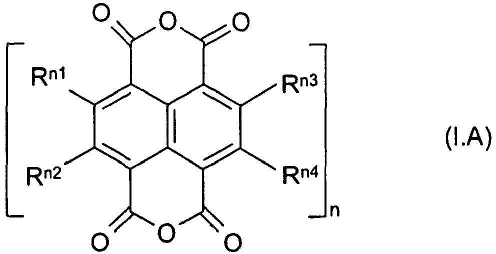 wobei n für 3 oder 4 steht, wenigstens einer der Reste Rⁿ¹, Rⁿ², Rⁿ³ und Rⁿ⁴ für Fluor steht und die übrigen Reste Rⁿ¹, Rⁿ², Rⁿ³ und Rⁿ⁴ für Wasserstoff stehen eignen sich in vorteilhafter Weise als Edukte zur Herstellung von ansonsten nicht oder nur schwer zugängigen Diimiden.

Ein weiterer Gegenstand der Erfindung ist daher ein Verfahren zur Herstellung von Verbindungen der Formel (I.Ba) 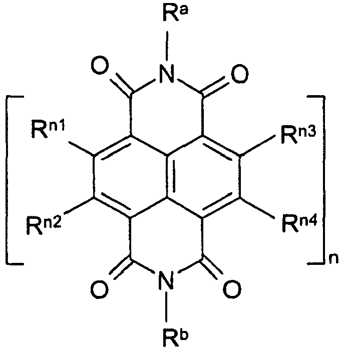 worin
n für 3 oder 4 steht,
wenigstens einer der Reste Rⁿ¹, Rⁿ², Rⁿ³ und Rⁿ⁴ für Fluor steht,
die übrigen Reste Rⁿ¹, Rⁿ², Rⁿ³ und Rⁿ⁴ für Wasserstoff stehen, und
R^{a} und R^{b} unabhängig voneinander für Wasserstoff oder für unsubstituiertes oder substituiertes Alkyl, Alkenyl, Alkadienyl, Alkinyl, Cycloalkyl, Bicycloalkyl, Cycloalkenyl, Heterocycloalkyl, Aryl oder Heteroaryl stehen, bei dem man ein Dianhydrid der Formel (I.A) 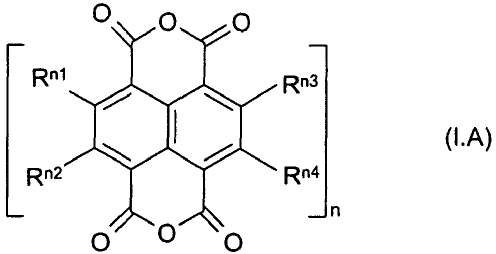 wobei
n für 3 oder 4 steht,
wenigstens einer der Reste Rⁿ¹, Rⁿ², Rⁿ³ und Rⁿ⁴ für Fluor steht,
die übrigen Reste Rⁿ¹, Rⁿ², Rⁿ³ und Rⁿ⁴ für Wasserstoff stehen,
einer Umsetzung mit einem Amin der Formel R^{a}-NH₂ und gegebenenfalls einem davon verschiedenen Amin der Formel R^{b}-NH₂ unterzieht.

Bezüglich geeigneter und bevorzugter Verfahrensbedingungen zur Umsetzung mit dem Amin der Formel R^{a}-NH₂ und gegebenenfalls dem Amin der Formel R^{b}-NH₂ wird auf die vorherigen Ausführungen zum Imidisierungsschritt b1), bzw. den Ausführungen zu den Imidisierungsschritten b1) und b2) in vollem Umfang Bezug genommen.

Ein weiterer Gegenstand der Erfindung ist ein Verfahren zur Herstellung von Verbindungen der Formeln I.Bb 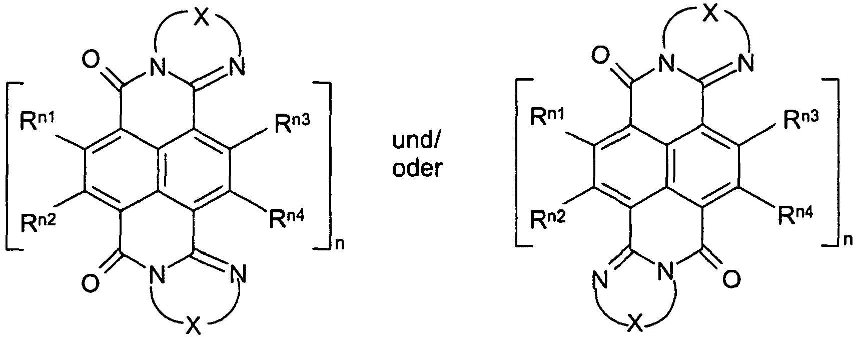 worin
n für 2, 3 oder 4 steht,
wenigstens einer der Reste Rⁿ¹, Rⁿ², Rⁿ³ und Rⁿ⁴ für Fluor steht,
die übrigen Reste Rⁿ¹, Rⁿ², Rⁿ³ und Rⁿ⁴ für Wasserstoff stehen, und
X für eine zweiwertige verbrückende Gruppe mit 2 bis 5 Atomen zwischen den flankierenden Bindungen steht,
bei dem man ein Dianhydrid der Formel (I.A) 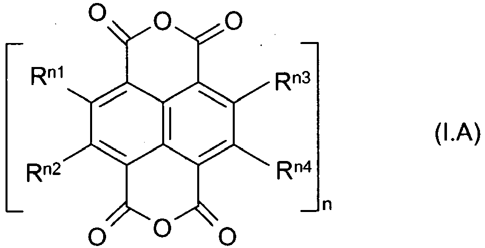 wobei
n für 2, 3 oder 4 steht,
wenigstens einer der Reste Rⁿ¹, Rⁿ², Rⁿ³ und Rⁿ⁴ für Fluor steht,
die übrigen Reste Rⁿ¹, Rⁿ², Rⁿ³ und Rⁿ⁴ für Wasserstoff stehen,
einer Umsetzung mit einem Amin der Formel H₂N-X-NH₂ unterzieht.

Bezüglich geeigneter und bevorzugter Verfahrensbedingungen zur Umsetzung mit dem Amin der Formel H₂N-X-NH₂ wird auf die vorherigen Ausführungen zum Imidisierungsschritt b2), bzw. den Ausführungen zu den Imidisierungsschritten b1) und b2) in vollem Umfang Bezug genommen.

Überraschenderweise wurde gefunden, dass sich fluorierte Diimide der Formel (I.Ba2), 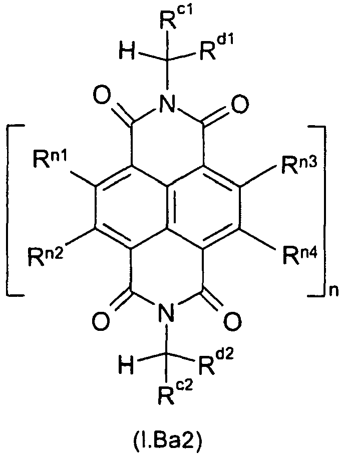 wobei einer oder mehrere (z. B. 2, 3 oder 4) der Reste Rⁿ¹, Rⁿ², Rⁿ³ und Rⁿ⁴ für Fluor stehen, die übrigen Reste Rⁿ¹, Rⁿ², Rⁿ³ und Rⁿ⁴ für Wasserstoff stehen, R^{c1} und R^{c2} unabhängig voneinander für Aryl stehen und R^{d1} und R^{d2} unabhängig voneinander für Alkyl stehen, leicht einer Umsetzung unter Erhalt von Diimiden unterziehen lassen, bei denen die Imidstickstoffe Wasserstoffatome tragen (I.BaH).

Ein weiterer Gegenstand der Erfindung ist daher ein Verfahren zur Herstellung von Verbindungen der Formel (I.BaH) 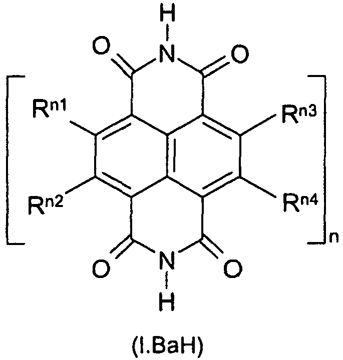 worin
n für 3 oder 4 steht,
wenigstens einer der Reste Rⁿ¹, Rⁿ², Rⁿ³ und Rⁿ⁴ für Fluor steht, und
die übrigen Reste Rⁿ¹, Rⁿ², Rⁿ³ und Rⁿ⁴ für Wasserstoff stehen,
bei dem man ein Diimid der Formel (I.Ba2), 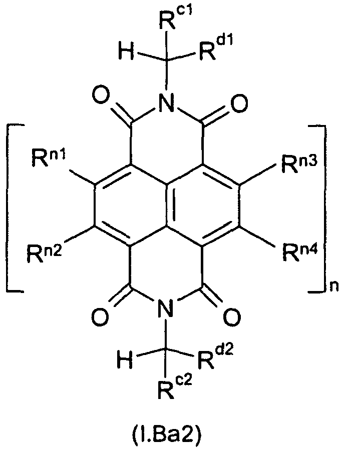 worin
n für 3 oder 4 steht,
wenigstens einer der Reste Rⁿ¹, Rⁿ², Rⁿ³ und Rⁿ⁴ für Fluor steht,
die übrigen Reste Rⁿ¹, Rⁿ², Rⁿ³ und Rⁿ⁴ für Wasserstoff stehen,
R^{c1} und R^{c2} unabhängig voneinander für Aryl stehen, und
R^{d1} und R^{d2} unabhängig voneinander für Alkyl stehen,
einer Umsetzung mit einer starken Lewis-Säure und einem Protonendonor unterzieht.

In den zuvor genannten Verbindungen der Formel (I.Ba2) haben R^{c1} und R^{c2} vorzugsweise die gleiche Bedeutung und stehen beispielsweise beide für Phenyl.

In den zuvor genannten Verbindungen der Formel (I.Ba2) haben R^{d1} und R^{d2} vorzugsweise die gleiche Bedeutung und stehen beispielsweise beide für C₁-C₁₂-Alkyl, besonders bevorzugt C₁-C₁₂-Alkyl, wie Methyl, Ethyl, n-Propyl, Isopropyl, n-Butyl, Isobutyl, sec.-Butyl, tert.-Butyl. Speziell stehen R^{d1} und R^{d1} beide für Methyl.

Als Lewis-Säure kommen kovalente Metallhalogenide und Halbmetallhalogenide, die eine Elektronenpaarlücke aufweisen, in Betracht. Derartige Verbindungen sind dem Fachmann bekannt, beispielsweise aus J.P. Kennedy, B. Ivan, "Designed Polymers by Carbocationic Macromolecular Engineering", Oxford University Press, New York, 1991. Sie sind in der Regel ausgewählt unter Halogen-Verbindungen des Titans, des Zinns, des Aluminiums, des Vanadiums oder des Eisens, sowie insbesondere den Halogeniden des Bors. Bevorzugte Lewis-Säuren sind Bortribromid, Bortrichlorid, Titantetrachlorid, Zinntetrachlorid, Aluminiumtrichlorid, Vanadiumpentachlorid, Eisentrichlorid, Alkylaluminiumdichloride und Dialkylaluminiumchloride. Eine besonders bevorzugte Lewis-Säure ist Titantetrachlorid.

Die Lewis-Säure wird in einer Menge eingesetzt, die ausrechend ist, beide Imidgruppen der Verbindung der Formel I.Ba2 umzusetzen. Das Molverhältnis von Lewis-Säure zu Verbindung der Formel I.Ba2 beträgt vorzugsweise 2:1 bis 10:1 und besonders bevorzugt 2,1:1 bis 5:1.

Die Umsetzung wird üblicherweise in einem Lösungsmittel durchgeführt. Als Lösungsmittel kommen solche in Frage, die gegenüber Lewis-Säuren stabil sind. Bevorzugte Lösungsmittel sind halogenierte Kohlenwasserstoffe, z. B. halogenierte aliphatische Kohlenwasserstoffe, vor allem Halogenalkane, wie Chlormethan, Dichlormethan, Trichlormethan, Chlorethan, 1,2-Dichlorethan, 1,1,1-Trichlorethan, 1-Chlorpropan, 2-Chlorpropan, 1-Chlorbutan und 2-Chlorbutan, und halogenierte aromatische Kohlenwasserstoffe, wie Chlorbenzol und Fluorbenzol. Geeignet sind auch Gemische der vorgenannten Lösungsmittel. Besonders bevorzugte Lösungsmittel sind die oben genannten Halogenalkane, vor allem Dichlormethan.

Es versteht sich von selbst, dass man die Umsetzung zunächst unter weitgehend aprotischen, insbesondere unter wasserfreien, Reaktionsbedingungen durchführt. Die Vorreinigung bzw. Vortrocknung der Lösungsmittel erfolgt in üblicher Weise, vorzugsweise durch Behandlung mit festen Trocknungsmitteln wie Molekularsieben oder vorgetrockneten Oxiden wie Aluminiumoxid, Siliciumdioxid, Calciumoxid oder Bariumoxid.

In der Regel wird man das erfindungsgemäße Verfahren bei Temperaturen im Bereich von 60 bis -140 °C, vorzugsweise im Bereich von 40 bis -80 °C, besonders bevorzugt im Bereich von 10 bis -40 °C durchführen.

Zum Freisetzen der Verbindungen (I.BaH) wird eine protische Verbindung (Protonendonor) zu dem Reaktionsgemisch gegeben. Der Protonendonor ist vorzugsweise ausgewählt unter Wasser, Alkoholen wie Methanol, Ethanol, n-Propanol, Isopropanol, n-Butanol, Isobutanol, sec-Butanol oder tert-Butanol, oder deren Mischungen mit Wasser.

Die Isolierung und gegebenenfalls Reinigung des erhaltenen Diimids (I.BaH) erfolgt nach üblichen, dem Fachmann bekannten Verfahren. Dazu zählt z. B. das Entfernen des für die Reaktion eingesetzten Lösungsmittels, z. B. durch Verdampfen. Das Verdampfen kann unter erhöhter Temperatur und/oder vermindertem Druck erfolgen. In einer geeigneten Ausführungsform wird das für die Reaktion mit der Lewis-Säure eingesetzte Lösungsmittel bereits vor der Zugabe des Protonendonors teilweise oder vollständig entfernt. Die Isolierung des Diimids (I.BaH) kann das Ausfällen mit einem geeigneten Fällungsmittel umfassen. Vorzugsweise wird zur Freisetzung des Diimids (I.BaH) ein Protonendonor eingesetzt, in dem die Verbindung (IBaH) nicht oder nur gering löslich ist. Solche Protonendonoren sind Wasser und Wasser/Alkohol-Gemische mit ausreichendem Wasseranteil. Die Isolierung des Diimids (I.BaH) kann weiterhin einen Filtrations- und/oder Trocknungsschritt umfassen.

Die zuvor genannten Verbindungen können einer weiteren Aufreinigung unterzogen werden. Dazu zählen beispielsweise säulenchromatographische Verfahren, Sublimation, Kristallisation oder eine Kombination mehrerer dieser Verfahren. Für einen Einsatz der Produkte als Halbleiter kann es von Vorteil sein, die Produkte einer weiteren Aufreinigung zu unterziehen. Die Säulenchromatographie sowie die Kristallistion erfolgen jeweils unter Einsatz eines geeigneten Lösungsmittels. Dazu zählen halogenierte Kohlenwasserstoffe, wie Methylenchlorid oder Chloroform, oder aromatische Lösungsmittel. Zur Kristallisation können auch aprotische Carbonsäureamide eingesetzt werden, gegebenenfalls in Kombination mit Wasser und/oder einem Alkohol, um die Löslichkeit zu verringern. In organischen Lösungsmitteln schlecht lösliche Verbindungen, z. B. Diimide, worin R^{a} und R^{b} für C₁- bis C₄-Alkyl stehen, sowie die Dianhydride können auch durch Fraktionierung aus Schwefelsäure gereinigt werden. Die Reinigung durch Sublimation kann unter Einsatz eines Temperaturgradienten erfolgen, z. B. in einer Dreizonensublimationsvorrichtung, wie im Folgenden näher beschrieben.

Die erfindungsgemäßen und nach dem erfindungsgemäßen Verfahren erhältlichen Verbindungen eignen sich besonders vorteilhaft als organische Halbleiter. Sie fungieren dabei in der Regel als n-Halbleiter. Werden die erfindungsgemäß eingesetzten Verbindungen der Formel (I) mit anderen Halbleitern kombiniert und ergibt sich aus der Lage der Energieniveaus, dass die anderen Halbleiter als n-Halbleiter fungieren, so können die Verbindungen (I) auch ausnahmsweise als p-Halbleiter fungieren. Dies ist z. B. bei der Kombination mit Cyano substituierten Perylentetracarboximiden der Fall. Die Verbindungen der Formel (I) zeichnen sich durch ihre Luftstabilität aus. Weiterhin verfügen sie über eine hohe Ladungstransportmobilität und haben ein hohes on/off-Verhältnis. Sie eignen sich in besonders vorteilhafter Weise für organische Feldeffekttransistoren. Die erfindungsgemäßen Verbindungen eignen sich vorteilhaft zur Herstellung von integrierten Schaltkreisen (ICs), für die bislang übliche n-Kanal MOSFET (metal oxide semiconductor field-effect transistor (MOSFET) zum Einsatz kommen. Dabei handelt es sich dann um CMOS analoge Halbleiterbausteine, z. B. für Microprozessoren, Microcontroller, statische RAM, und andere digitale logic circuits. Zur Herstellung von Halbleitermaterialien können die erfindungsgemäßen Verfahren nach einem der folgenden Verfahren weiterverarbeitet werden: Drucken (Offset, Flexo, Gravur, Screen, Inkjet, Elektrofotografie), Verdampfen, Lasertransfer, Fotolithografie, Dropcasting. Sie eignen sich insbesondere für einen Einsatz in Displays (speziell großflächigen und/oder flexiblen Displays) und RFID-Tags.

Die erfindungsgemäßen und nach dem erfindungsgemäßen Verfahren erhältlichen Verbindungen eignen sich weiterhin besonders vorteilhaft zur Datenspeicherung, in Dioden, speziell in OLEDs, in der Photovoltaik, als UV-Absorber, als optischer Aufheller, als unsichtbare Label und als Fluoreszenzlabel für Biomoleküle, wie Proteine, DNA, Zucker und Kombinationen davon.

Die erfindungsgemäßen und nach dem erfindungsgemäßen Verfahren erhältlichen Verbindungen eignen sich weiterhin besonders vorteilhaft als Fluoreszenzfarbstoff in einem auf Fluoreszenzonversion beruhenden Display; in einem lichtsammelnden Kunststoffteil, welches gegebenenfalls mit einer Solarzelle kombiniert ist; als Pigmentfabstoff in elektrophoretischen Displays; als Fluoreszenzfarbstoff in einer auf Chemolumineszenz basierenden Anwendung (z. B. in glow sticks).

Die erfindungsgemäßen und nach dem erfindungsgemäßen Verfahren erhältlichen Verbindungen eignen sich weiterhin besonders vorteilhaft als Fluoreszenzfarbstoff in einem auf Fluoreszenzkonversion beruhendem Display. Derartige Displays umfassen im Algemeinen ein transparentes Substrat, einen auf dem Substrat befindlichen Fluoreszenzfarbstoo und eine Strahlungsquelle. Übliche Strahlungsquellen senden blaues (color by blue) oder UV-Licht (color by uv) aus. Die Farbstoffe absorbieren entweder das blaue oder das UV-Licht und werden als Grünemitter eingesetzt. In diesen Displays wird z. B. das rote Licht erzeugt, indem der Rotemitter durch einen blaues oder UV-Licht absorbierenden Grünemitter angeregt wird. Geeignete color-by-blue-Displays sind z. B. in der WO 98/28946 beschrieben. Geeignete color-by-uv-Displays werden z. B. von W.A. Crossland, I.D. Sprigle und A.B. Davey in Photoluminescent LCDs (PL-LCD) using phosphors Cambridge University and Screen Technology Ltd., Cambridge, UK beschrieben.

Die erfindungsgemäßen und nach dem erfindungsgemäßen Verfahren erhältlichen Verbindungen eignen sich weiterhin besonders als Fluoreszenzemitter in OLEDs, in denen sie entweder durch Elektrolumineszenz oder durch einen entsprechenden Phosphoreszenzemitter über Förster Energietransfer (FRET) angeregt werden.

Die erfindungsgemäßen und nach dem erfindungsgemäßen Verfahren erhältlichen Verbindungen eignen sich weiterhin besonders in Displays, welche basierend auf einem elektrophoretischen Effekt über geladene Pigmentfarbstoffe Farben an- und ausschalten. Derartige elektrophoretische Displays sind z. B. in der US 2004/0130776 beschrieben.

Die erfindungsgemäßen und nach dem erfindungsgemäßen Verfahren erhältlichen Verbindungen eignen sich weiterhin besonders für den Einsatz in einem lichtsammelnden Kunststoffteil, welches großflächig Licht absorbiert und an seinen Kanten nach mehrfacher Brechung das Licht emittiert (sogenannt LISAs). Derartige LISAs können an den Kanten Solarzellen wie z. B. Siliciumsolarzellen oder organische Solarzellen aufweisen, die das konzentrierte Licht in elektrische Energie umwandeln. Eine Kombination lichtsammelnder Kunststoffe mit Solarzellen ist z. B. in der US 4,110,123 beschrieben.

Die erfindungsgemäßen und nach dem erfindungsgemäßen Verfahren erhältlichen Verbindungen eignen sich weiterhin besonders in Chemilumineszenzanwendungen. Dazu zählen so genannte "Glow Sticks". Zu deren Herstellung kann wenigstens eine Verbindung der Formel (I) z. B. in einem Alkylphthalat gelöst werden. Eine Anregung der Chemilumineszenz kann durch Mischung eines Oxalsäureesters mit Wasserstoffperoxid erfolgen, z. B. nachdem diese beiden zunächst separaten Komponenten durch Zerbrechen eines Glases gemischt werden. Die resultierende Reaktionsenergie führt zur Anregung und Fluoreszenz der Farbstoffe. Derartige Glow Sticks können als Notlicht, z. B. beim Angeln, in Seenotrettungswesten oder anderen Sicherheitsanwendungen eingesetzt werden.

Die erfindungsgemäßen und nach dem erfindungsgemäßen Verfahren erhältlichen Verbindungen eignen sich weiterhin besonders zum Laserschweißen oder zum Wärmemanagement.

Gegenstand der Erfindung sind weiterhin organische Feldeffekttransistoren, umfassend ein Substrat mit wenigstens einer Gate-Struktur, einer Source-Elektrode und einer Drain-Elektrode und wenigstens einer Verbindung der Formel I, wie zuvor definiert, als n-Halbleiter. Gegenstand der Erfindung sind weiterhin Substrate mit einer Vielzahl von organischen Feldeffekttransistoren, wobei zumindest ein Teil der Feldeffektransistoren wenigstens einer Verbindung der Formel I, wie zuvor definiert, als n-Halbleiter enthält. Gegenstand der Erfindung sind auch Halbleiterbausteine, die wenigstes ein solches Substrat umfassen.

Eine spezielle Ausführungsform ist ein Substrat mit einem Muster (Topographie) von organischen Feldeffekttransistoren, wobei jeder Transistor
- einen auf dem Substrat befindlichen organischen Halbleiter;
- eine Gate-Struktur zur Steuerung der Leitfähigkeit des leitenden Kanals; und
- leitfähige Source- und Drain-Elektroden an den beiden Enden des Kanals enthält, wobei der organische Halbleiter aus wenigstens einer Verbindung der Formel (I) besteht oder eine Verbindung der Formel (I) umfasst. Des Weiteren umfasst der organische Feldeffekttransistor in der Regel ein Dielektrikum.

Eine weitere spezielle Ausführungsform ist ein Substrat mit einem Muster von organischen Feldeffekttransistoren, wobei jeder Transistor einen integrierten Schaltkreis bildet oder Teil eines integrierten Schaltkreises ist und wobei zumindest ein Teil der Transistoren wenigstens eine Verbindung der Formel (I) umfasst.

Als Substrate eignen sich prinzipiell die dafür bekannten Materialien. Geeignete Substrate umfassen z. B. Metalle (vorzugsweise Metalle der Gruppen 8, 9, 10 oder 11 des Periodensystems, wie Au, Ag, Cu), oxidische Materialien (wie Glas, Quartz, Keramiken, SiO₂), Halbleiter (z. B. gedoptes Si, gedoptes Ge), Metalllegierungen (z. B. auf Basis von Au, Ag, Cu, etc.), Halbleiterlegierungen, Polymere (z. B. Polyvinylchlorid, Polyolefine, wie Polyethylen und Polypropylen, Polyester, Fluoropolymere, Polyamide, Polyimide, Polyurethane, Polyalkyl(meth)acrylate, Polystyrol und Mischungen und Komposite davon), anorganische Feststoffe (z. B. Ammoniumchlorid), Papier und Kombinationen davon. Die Substrate können flexibel oder unflexibel solid, mit gekrümmter oder planarer Geometrie sein, abhängig von der gewünschten Anwendung.

Ein typisches Substrat für Halbleiterbausteine umfasst eine Matrix (z. B. eine Quartz - oder Polymermatrix) und, optional, eine dielektrische Deckschicht.

Geeignete Dielektrika sind SiO₂, Polystyrol, Poly-α-methylstyrol, Polyolefine (wie Polypropylen, Polyethylen, Polyisobuten) Polyvinylcarbazol, fluorierte Polymere (z. B. Cytop, CYMM) Cyanopullane, Polyvinylphenol, Poly-p-xylol, Polyvinylchlorid oder thermisch oder durch Luftfeuchtigkeit vernetzbare Polymere. Spezielle Dielektrika sind "self assembled nanodielectrics", d. h. Polymere, welche aus SiCI-Funktionalitäten enthaltenden Monomeren wie z. B. Cl₃SiOSiCl₃, Cl₃Si-(CH₂)₆-SiCl₃ , Cl₃Si-(CH₂)₁₂-SiCl₃ erhalten und/oder welche durch Luftfeuchtigkeit oder durch Zugabe von Wasser in Verdünnung mit Lösungsmitteln vernetzt werden (siehe z. B. Faccietti Adv. Mat. 2005, 17, 1705-1725). An Stelle von Wasser können auch Hydroxylgruppen-haltige Polymere wie Polyvinylphenol oder Polyvinylalkohol oder Copolymere aus Vinylphenol und Styrol als Vernetzungskomponenten dienen. Es kann auch wenigstens ein weiteres Polymer während des Vernetzungsvorgangs zugegen sein, wie z. B. Polystyrol, welches dann mitvernetzt wird (siehe Facietti, US-Patentanmeldung 2006/0202195).

Das Substrat kann zusätzlich Elektroden aufweisen, wie Gate-, Drain- und Source-Elektroden von OFETs, die normalerweise auf dem Substrat lokalisiert sind (z. B. abgeschieden auf oder eingebettet in eine nichtleitende Schicht auf dem Dielektrikum). Das Substrat kann zusätzlich leitfähige Gate-Elektroden der OFETs enthalten, die üblicherweise unterhalb der dielektrischen Deckschicht (d. h. dem Gate-Dielektrikum) angeordnet sind.

Nach einer speziellen Ausführung befindet sich eine Isolatorschicht (gate insulating layer) auf wenigstes einem Teil der Substratoberfläche. Die Isolatorschicht umfasst wenigstens einen Isolator, der vorzugsweise ausgewählt ist unter anorganischen Isolatoren, wie SiO₂, SiN, etc., ferroelektrischen Isolatoren, wie Al₂O₃, Ta₂O₅, La₂O₅, TiO₂, Y₂O₃, etc., organischen Isolatoren, wie Polyimiden, Benzocyclobuten (BCB), Polyvinylalkoholen, Polyacrylaten, etc. und Kombinationen davon.

Geeignete Materialien für Source- und Drain-Elektroden sind prinzipiell elektrisch leitfähige Materialien. Dazu zählen Metalle, vorzugsweise Metalle der Gruppen 8, 9, 10 oder 11 des Periodensystems, wie Pd, Au, Ag, Cu, Al, Ni, Cr, etc. Geeignet sind weiterhin leitfähige Polymere, wie PEDOT (=Poly(3,4-ethylenedioxythiophene);PSS (= Poly(styrolsulfonat), Polyanilin, oberflächenmodifiziertes Gold, etc. Bevorzugte elektrisch leitfähige Materialien haben einen spezifischen Widerstand von weniger als 10 ⁻³, vorzugsweise weniger als 10 ⁻⁴, insbesondere weniger als 10 ⁻⁶ oder 10 ⁻⁷ Ohm x Meter.

Nach einer speziellen Ausführung befinden sich Drain- und Source-Elektroden zumindest teilweise auf dem organischen Halbleitermaterial. Selbstverständlich kann das Substrat weitere Komponenten umfassen, wie sie üblicherweise in Halbleitermaterialien oder ICs eingesetzt werden, wie Isolatoren, Widerstände, Kondensatoren, Leiterbahnen, etc.

Die Elektroden können nach üblichen Verfahren, wie Verdampfen, lithographische Verfahren oder einen anderen Strukturierungsprozess aufgebracht werden.

Die Halbleitermaterialien können auch mit geeigneten Hilfsmitteln (Polymere, Tenside) in disperser Phase durch Verdrucken verarbeitet werden.

In einer ersten bevorzugten Ausführungsform erfolgt die Abscheidung wenigstens einer Verbindung der allgemeinen Formel I (und gegebenenfalls weiterer Halbleitermaterialien) durch ein Gasphasenabscheidungsverfahren (Physical Vapor Deposition PVD). PVD-Verfahren werden unter Hochvakuumbedingungen durchgeführt und umfassen die folgenden Schritte: Verdampfen, Transport, Abscheidung. Überraschenderweise wurde gefunden, dass sich die Verbindungen der allgemeinen Formel I besonders vorteilhaft für einen Einsatz in einem PVD-Verfahren eignen, da sie sich im Wesentlichen nicht zersetzen und/oder unerwünschte Nebenprodukte bilden. Das abgeschiedene Material wird in hoher Reinheit erhalten. In einer speziellen Ausführung wird das abgeschiedene Material in Form von Kristallen erhalten oder enthält einen hohen kristallinen Anteil. Allgemein wird zur PVD wenigstens eine Verbindung der allgemeinen Formel I auf eine Temperatur oberhalb ihrer Verdampfungstemperatur erhitzt und durch Abkühlen unterhalb der Kristallisationstemperatur auf einem Substrat abgeschieden. Die Temperatur des Substrats bei der Abscheidung liegt vorzugsweise in einem Bereich von etwa 20 bis 250 °C, besonders bevorzugt 50 bis 200 °C. Überraschenderweise wurde gefunden, dass erhöhte Substrattemperaturen bei der Abscheidung der Verbindungen der Formel I vorteilhafte Auswirkungen auf die Eigenschaften der erzielten Halbleiterelemente haben können.

Die erhaltenen Halbleiterschichten weisen im Allgemeinen eine Dicke auf, die für einen ohmschen Kontakt zwischen Source- und Drain-Elektrode ausreicht. Die Abscheidung kann unter einer Inertatmosphäre, z. B. unter Stickstoff, Argon oder Helium erfolgen.

Die Abscheidung erfolgt üblicherweise bei Umgebungsdruck oder unter reduziertem Druck. Ein geeigneter Druckbereich beträgt etwa 10⁻⁷ bis 1,5 bar.

Vorzugsweise wird die Verbindung der Formel I auf dem Substrat in einer Dicke von 10 bis 1000 nm, besonders bevorzugt 15 bis 250 nm, abgeschieden. In einer speziellen Ausführung wird die Verbindung der Formel I zumindest teilweise in kristalliner Form abgeschieden. Hierfür eignet sich speziell das zuvor beschriebene PVD-Verfahren. Weiterhin ist es möglich, zuvor hergestellte organische Halbleiterkristalle einzusetzen. Geeignete Verfahren zur Gewinnung von derartigen Kristallen werden von R. A. Laudise et al. in "Physical Vapor Growth of Organic Semi-Conductors", Journal of Crystal Growth 187 (1998), Seiten 449-454, und in "Physical Vapor Growth of Centimetersized Crystals of α-Hexathiophene", Journal of Cystal Growth 1982 (1997), Seiten 416-427, beschrieben, worauf hier Bezug genommen wird.

In einer zweiten bevorzugten Ausführungsform erfolgt die Abscheidung wenigstens einer Verbindung der allgemeinen Formel I (und gegebenenfalls weiterer Halbleitermaterialien) durch eine Rotationsbeschichtung (spin coating). Überraschenderweise lassen sich die erfindungsgemäß verwendeten Verbindungen der Formel I somit auch in einem Nassverarbeitungsverfahren (wet processing) zur Herstellung von Halbleitersubstraten einsetzen. Die Verbindungen der Formel (I) sollten sich somit auch zur Herstellung von Halbleiterelementen, speziell OFETs oder auf der Basis von OFETs, durch ein Druckverfahren eignen. Es können dafür übliche Druckprozesse (Inkjet, Flexo, Offset, gravure; Tiefdruck, Nanoprint) verwendet werden. Bevorzugte Lösungsmittel für den Einsatz der Verbindungen der Formel (I) in einem Druckverfahren sind aromatische Lösungsmittel wie Toluol, Xylol, etc. Man kann zu diesen "Halbleitertinten" verdickend wirkende Substanzen, wie Polymere zusetzen, z. B. Polystyrol, etc. Dabei verwendet man als Dielektrikum die zuvor genannten Verbindungen.

In einer bevorzugten Ausführungsform handelt es sich bei dem erfindungsgemäßen Feldeffekttransistor um einen Dünnschichttransistor (thin film transistor, TFT). Gemäß einem üblichen Aufbau verfügt ein Dünnschichttransistor über eine auf dem Substrat befindliche Gate-Elektrode, eine auf dieser und dem Substrat befindliche Gate-Isolierschicht, eine auf der Gate-Isolierschicht befindliche Halbleiterschicht, eine ohmsche Kontaktschicht auf der Halbleiterschicht sowie über eine Source-Elektrode und eine Drain-Elektrode auf der ohmschen Kontaktschicht.

In einer bevorzugten Ausführungsform wird die Oberfläche des Substrats vor der Abscheidung wenigstens einer Verbindung der allgemeinen Formel I (und gegebenenfalls wenigstens eines weiteren Halbleitermaterials) einer Modifizierung unterzogen. Diese Modifizierung dient der Bildung von die Halbleitermaterialien bindenden Bereichen und/oder von Bereichen, auf denen keine Halbleitermaterialien abgeschieden werden können. Bevorzugt wird die Oberfläche des Substrats mit wenigstens einer Verbindung (C1) modifiziert, die geeignet ist, an die Oberfläche des Substrats sowie die Verbindungen der Formel (I) zu binden. In einer geeigneten Ausführungsform wird ein Teil der Oberfläche oder die komplette Oberfläche des Substrats mit wenigstens einer Verbindung (C1) beschichtet, um eine verbesserte Abscheidung wenigstens einer Verbindung der allgemeinen Formel I (und gegebenenfalls weiterer halbleitender Verbindungen) zu ermöglichen. Eine weitere Ausführungsform umfasst die Abscheidung eines Musters von Verbindungen der allgemeinen Formel (C1) auf dem Substrat nach einem entsprechenden Herstellungsverfahren. Dazu zählen die dafür bekannten Maskenprozesse sowie sogenannte "Patterning"-Verfahren, wie sie z. B. in der US 11/353934 beschrieben sind, worauf hier in vollem Umfang Bezug genommen wird.

Geeignete Verbindungen der Formel (C1) sind zu einer bindenden Wechselwirkung sowohl mit dem Substrat als auch mit wenigstens einer Halbleiterverbindung der allgemeinen Formel I befähigt. Der Begriff "bindende Wechselwirkung" umfasst die Bildung einer chemischen Bindung (kovalenten Bindung), ionischen Bindung, koordinativen Wechselwirkung, Van der Waals-Wechselwirkungen, z. B. Dipol-Dipol-Wechselwirkungen) etc. und Kombinationen davon. Geeignete Verbindungen der allgemeinen Formel (C1) sind:
- Silane, Phosphonsäuren, Carbonsäuren, Hydroxamsäuren, wie Alkyltrichlorsilane, z. B. n-(Octadecyl)trichlorsilan; Verbindungen mit Trialkoxysilan-Gruppen, z. B. Alkyltrialkoxysilane, wie n-Octadecyltrimethoxysilan, n-Octadecyltriethoxysilan, n-Octadecyltri-(n-propyl)oxysilan, n-Octadecyltri-(isopropyl)oxysilan; Tri-alkoxyaminoalkylsilane, wie Triethoxyaminopropylsilan und N[(3-triethoxysilyl)-propyl]-ethylen-diamin; Trialkoxyalkyl-3-glycidylethersilane, wie Triethoxypropyl-3-glycidylethersilan; Trialkoxyallylsilane wie Allyltrimethoxysilan; Trialkoxy-(isocyanatoalkyl)silane; Trialkoxysilyl(meth)acryloxyalkane und Trialkoxysilyl-(meth)acrylamidoalkane, wie 1-Triethoxysilyl-3-acryloxypropan.
- Amine, Phosphine und Schwefel enthaltende Verbindungen, speziell Thiole.

Bevorzugt ist die Verbindung (C1) ausgewählt unter Alkyltrialkoxysilanen, speziell n-Octadecyltrimethoxysilan, n-Octadecyltriethoxysilan; Hexaalkyldisilazanen, und speziell Hexamethyldisilazane (HMDS); C₈-C₃₀-Alkylthiolen, speziell Hexadecanthiol; Mercaptocarbonsäuren und Mercaptosulfonsäuren speziell Mercaptoessigsäure, 3-Mercaptopropionsäure, Mercaptobernsteinsäure, 3-Mercapto-1-propansulfonsäure und den Alkalimetall- und Ammoniumsalzen davon.

Es sind auch verschiedene Halbleiterarchitekturen mit den erfindungsgemäßen Halbleitern denkbar wie z. B. Top Contact, Top Gate, Bottom Contact, Bottom Gate, oder aber ein vertikaler Aufbau wie z. B. ein VOFET (Vertical organic field effect transistor) wie z. B. in US 2004/0046182 beschrieben.

Die Schichtdicken betragen bei Halbleitern z. B. 10 nm bis 5 µm, beim Dielektrikum 50 nm bis 10 µm, die Elektroden können z. B. 20 nm bis 1 µm dick sein. Die OFETs können auch zu anderen Bauteilen wie Ringoszillatoren oder Inverter kombiniert werden.

Ein weiterer Aspekt der Erfindung ist die Bereitstellung elektronischer Bauteile, die mehrere Halbleiterkomponenten umfassen, wobei es sich um n- und/oder p-Halbleiter handeln kann. Beispiele solcher Bauteile sind Feldeffekttransistoren (FETs), bipolare Flächentransistoren (bipolar junction transistors, BJTs), Tunneldioden, Wechselrichter, Licht-emittierende Bauteile, biologische und chemische Detectoren oder Sensoren, Temperatur-abhängige Detektoren, Photodetektoren wie Polarisations-sensitive Photodetektoren, Gatter, AND-, NAND-, NOT-, OR-, TOR-, und NOR-Gatter, Register, Schalter, Zeitbausteine, statische oder dynamische Speicher und andere dynamische oder sequentielle logische oder andere digitale Bauteile einschließlich programmierbarer Schaltungen.

Ein spezielles Halbleiterelement ist ein Inverter. In der digitalen Logic ist der Inverter ein Gatter, das ein Eingangssignal invertiert. Der Inverter wird auch als NOT-Gate bezeichnet. Reale Inverterschaltungen weisen einen Ausgangsstrom auf, der das Gegenteil zum Eingangsstrom darstellt. Übliche Werte sind z. B. (0, +5V) for TTL-Schaltungen. Die Leistungsfähigkeit eines digitalen Inverters gibt die Spannungtransferkurve (Voltage Transfer Curve VTC) wieder, d. h. der Auftrag von Inputstrom gegen Outputstrom. Idealerweise handelt es sich um eine Stufenfunktion und je näher sich die real gemessene Kurve einer solchen Stufe annähert, desto besser ist der Inverter. In einer speziellen Ausführung der Erfindung werden die Verbindungen der Formel (I) als organischer n-Halbleiter in einem Inverter eingesetzt.

Die erfindungsgemäßen und nach dem erfindungsgemäßen Verfahren erhältlichen Verbindungen der Formel (I) eignen sich weiterhin besonders vorteilhaft für einen Einsatz in der organischen Photovoltaik (OPV). Prinzipiell eignen sich diese Verbindungen auch für einen Einsatz in Farbstoff-sensibilisierten Solarzellen. Dabei kommen speziell Verbindungen der Formel (I) mit Anhydridfunktionen zum Einsatz. Bevorzugt ist jedoch ihr Einsatz in Solarzellen, die durch eine Diffusion von angeregten Zuständen gekennzeichnet sind. Dabei zeichnet sich eines oder beide der eingesetzten Halbleitermaterialien durch eine Diffusion von angeregten Zuständen aus. Geeignet ist auch die Kombination wenigstens eines Halbleitermaterials, durch eine Diffusion von angeregten Zuständen gekennzeichnet ist, mit Polymeren, die eine Leitung der angeregten Zustände entlang der Polymerkette zulassen. Derartige Solarzellen werden im Sinne der Erfindung als excitonische Solarzellen bezeichnet. Die Direktumwandlung von Solarenergie in elektrische Energie in Solarzellen beruht auf dem inneren Photoeffekt eines Halbleitermaterials, d. h. der Erzeugung von Elektron-Loch-Paaren durch Absorption von Photonen und der Trennung der negativen und positiven Ladungsträger an einem p-n-Übergang oder einem Schottky-Kontakt. Ein Exciton kann z. B. entstehen, wenn ein Photon in einen Halbleiter eindringt und ein Elektron zum Übergang aus dem Valenzband in das Leitungsband anregt. Um Strom zu erzeugen, muss der durch die absorbierten Photonen erzeugte angeregte Zustand jedoch einen p-n-Übergang erreichen, um ein Loch und ein Elektron zu erzeugen, welches dann zur Anode und Kathode fließt. Die so erzeugte Photospannung kann in einem äußeren Stromkreis einen Photostrom bewirken, durch den die Solarzelle ihre Leistung abgibt. Von dem Halbleiter können dabei nur solche Photonen absorbiert werden, die eine Energie aufweisen, die größer als seine Bandlücke ist. Die Größe der Halbleiterbandlücke bestimmt also den Anteil des Sonnenlichts, der in elektrische Energie umgewandelt werden kann. Solarzellen bestehen normalerweise aus zwei absorbierenden Materialien mit unterschiedlichen Bandlücken, um die Sonnenenergie möglichst effektiv zu nutzen. Die meisten organischen Halbleiter haben Excitonen-Diffusionslängen von bis zu 10 nm. Hier besteht weiterhin ein Bedarf an organischen Halbleitern, über die der angeregte Zustand über möglichst große Distanzen weitergeleitet werden kann. Überraschenderweise wurde nun gefunden, dass sich die zuvor beschriebenen Verbindungen der allgemeinen Formel I besonders vorteilhaft für einen Einsatz in excitonischen Solarzelle eignen.

Geeignete organische Solarzellen sind in der Regel schichtförmig aufgebaut und umfassen in der Regel zumindest die folgenden Schichten: Anode, photoaktive Schicht und Kathode. Diese Schichten befinden sich in der Regel auf einem dafür üblichen Substrat. Der Aufbau organischer Solarzellen ist z. B. in US 2005/0098726 A1 und US 2005/0224905 A1 beschrieben, worauf hier in vollem Umfang Bezug genommen wird.

Geeignete Substrate sind z. B. oxidische Materialien (wie Glas, Quartz, Keramik, SiO₂, etc.), Polymere (z. B. Polyvinylchlorid, Polyolefine, wie Polyethylen und Polypropylen, Polyester, Fluorpolymere, Polyamide, Polyurethane, Polyalkyl(meth)acrylate, Polystyrol und Mischungen und Komposite davon) und Kombinationen davon.

Als Elektroden (Kathode, Anode) eignen sich prinzipiell Metalle (vorzugsweise der Gruppen 8, 9, 10 oder 11 des Periodensystems, z. B. Pt, Au, Ag, Cu, Al, In, Mg, Ca), Halbleiter (z. B. dotiertes Si, dotiertes Ge, Indium-Zinn-Oxid (ITO), Gallium-Indium-Zinn-Oxid (GITO), Zink-Indium-Zinn-Oxid (ZITO), etc.), Metalllegierungen (z. B. auf Basis Pt, Au, Ag, Cu, etc., speziell Mg/Ag-Legierungen), Halbleiterlegierungen, etc. Bevorzugt wird als Anode ein gegenüber einfallendem Licht im Wesentlichen transparentes Material eingesetzt. Dazu zählt z. B. ITO, dotiertes ITO, ZnO, TiO₂, Ag, Au, Pt. Bevorzugt wird als Kathode ein das einfallende Licht im Wesentlichen reflektierendes Material eingesetzt. Dazu zählen z. B. Metallfilme, z. B. aus Al, Ag, Au, In, Mg, Mg/Al, Ca, etc.

Die photoaktive Schicht ihrerseits umfasst wenigstens eine oder besteht aus wenigstens einer Schicht, die als organisches Halbleitermaterial wenigsten eine Verbindung enthält, die ausgewählt ist unter Verbindungen der Formeln I und II, wie zuvor definiert. In einer Ausführung umfasst die photoaktive Schicht wenigstens ein organisches Akzeptormaterial. Zusätzlich zu der photoaktiven Schicht kann es eine oder mehrere weitere Schichten geben, z. B. eine Schicht mit Elektronen leitenden Eigenschaften (ETL, electron transport layer) und eine Schicht, die ein löcherleitendes Material (hole transport layer, HTL) enthält, die nicht absorbieren müssen, Excitonen und Löcher blockierende Schichten (z. B. excition blocking layers, EBL), die nicht absorbieren sollen, Multiplikatorschichten (multiplication layers). Geeignete Excitonen und Löcher blockierende Schichten sind z. B. in US 6,451,415 beschrieben.

Geeignete Excitonenblockerschichten sind z. B. Bathocuproine (BCP), 4,4',4"-Tris(N,-(3-methylphenyl)-N-phenylamino)triphenylamin (m-MTDATA) oder Polyethylendioxithiophen (PEDOT), wie in US 7,026,041 beschrieben.

Die erfindungsgemäßen excitonischen Solarzellen basieren auf photoaktiven Donor-Akzeptor-Heteroübergängen. Wird wenigstens eine Verbindung der Formel (I) als HTM eingesetzt, muss das entsprechende ETM so gewählt werden, dass nach Anregung der Verbindungen ein schneller Elektronenübergang auf das ETM stattfindet. Geeignete ETM sind z. B. C60 und andere Fullerene, Perylen-3,4:9,10-bis(dicarboximide) PTCDI, etc. Wird wenigstens eine Verbindung der Formel (I) als ETM eingesetzt, muss das komplementäre HTM so gewählt werden, dass nach Anregung der Verbindung ein schneller Löcherübertrag auf das HTM stattfindet. Der Heteroübergang kann flach ausgeführt werden (vgl. Two layer organic photovoltaic cell, C. W. Tang, Appl. Phys. Lett., 48 (2), 183-185 (1986) oder N. Karl, A. Bauer, J. Holzäpfel, J. Marktanner, M. Möbus, F. Stölzle, Mol. Cryst. Liq. Cryst., 252, 243-258 (1994).) oder als Volumen-Heteroübergang (bulk heterojunction bzw. interpenetrierenes Donor-akzeptor-Netzwerk, vgl. z. B. C. J. Brabec, N. S. Sariciftci, J. C. Hummelen, Adv. Funct. Mater., 11 (1), 15 (2001).) realisiert werden. Die photoaktive Schicht auf Basis eines Hetero-übergangs zwischen wenigstens eine Verbindung der Formel (I) und einem HTL oder ETL kann in Solarzellen mit MiM-, pin-, pn-, Mip- oder Min-Aufbau zum Einsatz kommen (M=Metall, p=p-dotierter organischer oder anorganischer Halbleiter, n=n-dotierter organischer oder anorganischer Halbleiter, i=intrinsisch leitfähiges System organischer Schichten, vgl. z. B. J. Drechsel et al., Org. Eletron., 5 (4), 175 (2004) oder Maennig et al., Appl. Phys. A 79, 1-14 (2004)). Sie kann auch in Tandemzellen, wie von P. Peumnas, A. Yakimov, S. R. Forrest in J. Appl. Phys, 93 (7), 3693-3723 (2003) (vgl. Patente US04461922, US06198091 und US06198092) beschrieben, verwendet werden. Sie kann auch in Tandemzellen aus zwei oder mehreren aufeinandergestapelten MiM-, pin-, Mip- oder Min-Dioden (vgl. Patentanmeldung DE 103 13 232.5) verwendet werden (J. Drechsel et al., Thin Solid Films, 451452, 515-517 (2004)).

Dünne Schichten der Verbindungen und aller anderer Schichten können durch Aufdampfen im Vakuum oder in Inertgasatmosphäre, durch Laserablation oder durch lösungs- oder dispersionsprozessierbare Verfahren wie Spin-Coating, Rakeln, Gießverfahren, Aufsprühen, Tauchbeschichtung oder Drucken (z. B. Inkjet, Flexo, Offset, gravure; Tiefdruck, Nanoimprint) hergestellt werden. Die Schichtdicken der M, n, i und p-Schichten betragen typischerweise 10 bis 1000 nm, bevorzugt 10 bis 400 nm.

Als Substrat werden z. B. Glas, Metall- oder Polymerfolien verwendet, die in der Regel mit einer transparenten, leitfähigen Schicht (wie z. B. SnO₂:F, SnO₂:In, ZnO:Al, Carbon-Nanotubes, dünne Metallschichten) beschichtet sind.

Neben den Verbindungen der allgemeinen Formel (I) eignen sich die folgenden Halbleitermaterialien für einen Einsatz in der organischen Photovoltaik:

Acene, wie Anthracen, Tetracen, Pentacen und substituierte Acene. Substituierte Acene umfassen wenigstens einen Substituenten, ausgewählt unter elektronschiebenden Substituenten (z. B. Alkyl, Alkoxy, Ester, Carboxylat oder Thioalkoxy), elektronziehenden Substituenten (z. B. Halogen, Nitro oder Cyano) und Kombinationen davon. Dazu zählen 2,9-Dialkylpentacene und 2,10-Dialkylpentacene, 2,10-Dialkoxypentacene, 1,4,8,11-Tetraalkoxypentacene und Rubren (5,6,11,12-tetraphenylnaphthacene). Geeignete substituierte Pentacene sind in US 2003/0100779 und US 6,864,396 beschrieben. Ein bevorzugtes Acen ist Ruben (5,6,11,12-tetraphenylnaphthacen).

Phthalocyanine, wie Hexadecachlorophthalocyanine und Hexadecafluorophthalocyanine, metallfreie und zweiwertige Metalle enthaltende, insbesondere die des Titanyloxy, Vanadyloxy, Eisens, Kupfers, Zinks, insbesondere Kupferphthalocyanin, Zinkphthalocyanin und metallfreies Phthalocyanin, Hexadecachlorokupferphthalocyanin, Hexadecachlorozinkphthalocyanin, metallfreies Hexadechlorophathlocyanin, Hexadecafluorokupferphthalocyanin, Hexadecafluorophthalocyanin oder metallfreies Hexadefluorophathlocyanin.

Porphyrine, wie z. B. 5,10,15,20-Tetra(3-pyridyl)porphyrin (TpyP).

Flüssigkristalline (LC-) Materialien, wie z. B. Hexabenzocoronene (HBC-PhC12) oder andere Corone, Coronendiimide, oder Triphenylene, wie 2,3,6,7,10,11-Hexahexylthiotriphenylen (HTT6) oder 2,3,6,7,10,11-Hexakis-(4-n-nonylphenyl)-triphenylen (PTP9), 2,3,6,7,10,11-hexakis-(undecyloxy)-triphenylene (HAT11). Besonders bevorzugt sind LCs, die diskotisch sind.

Thiophene, Oligothiophene und substituierte Derivate davon. Geeignete Oligothiophene sind Quaterthiophene, Quinquethiophene, Sexithiophene, α,ω-Di(C₁-C₈)-alkyloligothiophenes, wie α,ω-Dihexylquaterthiophene, α,ω-Dihexylquinquethiophene und α,ω-Dihexylsexithiophene, Poly(alkylthiophene), wie Poly(3-hexylthiophen), Bis(dithienothiophene), Anthradithiophene und Dialkylanthradithiophene, wie Dihexylanthradithiophen, Phenylene-Thiophen- (P-T-) Oligomere und Derivate davon, speziell α,ω-Alkyl-substituierte Phenylen-Thiophen-Oligomere.

Bevorzugte Thiophene, Oligothiophene und substituierte Derivate davon sind Poly-3-hexylthiophen oder Verbidungen des Typs α α,'-Bis (2,2-dicyanovinyl)quinquethiophene (DCV5T), P3HT, (3-(4-Octylphenyl)-2,2' bithiophen) (PTOPT), (PEOPT), (Poly(3-(2'-methoxy-5'-octylphenyl)thiophene)) (POMeOPT), Poly(3-octylthiophen) (P3OT), EHH-PpyPz, Copolymere PTPTB, BBL, F8BT, PFMO, siehe Brabec C., Adv. Mater., 2996, 18, 2884. (PCPDTBT) Poly[2,6-(4,4-bis-(2-ethylhexyl)-4H-cyclopenta[2,1-b;3,4-b']-dithiophene)-4,7-(2,1,3-benzothiadiazote).

Paraphenylenvinylen und Paraphenylenvinylen-enthaltende Oligomere oder Polymere wie z. B. Polyparaphenylenvinylen, MEH-PPV (Poly(2-methoxy-5-(2'-ethylhexyloxy)-1,4-phenylenevinylene, MDMO-PPV (Poly(2-methoxy-5-(3',7'-dimethyloctyloxy)-1,4-phenylene-vinylene)), PPV, CN-PPV (mit verschiedenen Alkoxy-Derivaten).

PPE-PPV-Hybridpolymere.

Polyfluorene und alternating polyfluorene Copolymere wie z. B. (mit 4,7-dithien-2'-yl-2,1,3-benzothiadiazole), auch F₈BT, PFB.

Polycarbazole, d. h. Carbazol enthaltende Oligomere und Polymere, wie (2,7) und (3,6).

Polyaniline, d. h. Anilin-enthaltende Oligomere und Polymere, wie (2,7) und (3,6).

Triarylamine, Polytriarylamine, Polycyclopentadiene, Polypyrrole, Polyfuran, Polysilole, Polyphosphole, TPD, CBP, Spiro-MeOTAD.

Fullerene, speziell C60 und seine Derivate wie PCBM (= [6,6]-Phenyl-C₆₁-buttersäuremethylester). In derartigen Zellen wäre das Fullerenderivat ein Lochleiter.

Kupfer(I)iodid, Kupfer(I)thiocyanat

p-n-Mischmaterialien, d. h. Donor und Akzeptor in einem Material, Polymer, Blockpolymere, Polymere mit C60s, C60-Azofarben, Triaden carotenoid-porphyrin-quinode LC Donor/Akzeptor-Systeme wie von Kelly in S. Adv. Mater. 2006, 18, 1754, beschrieben.

Alle zuvor genannten Halbleitermaterialien können auch dotiert sein. Beispiele von Dotierstoffen: Br₂, F₄-TCNQ, etc.

Die Erfindung wird anhand der folgenden, nicht einschränkenden Beispiele näher erläutert.

### Beispiele

Allgemeine Vorschrift zur Ermittlung der Transistorkenndaten

### I. Herstellung von Halbleitersubstraten mittels physical vapor deposition (PVD)

Als Substrate wurden n-dotierte Siliciumwafer (2,5 x 2,5 cm, Leitfähigkeit < 0,004 Ω⁻¹cm) mit thermisch abgeschiedener Oxidschicht als Dielektrikum (flächenbezogene Kapazität Cᵢ = 10 nF/cm²) eingesetzt. Die Oberflächen der Substrate wurden durch Waschen mit Aceton, gefolgt von Isopropanol, gereinigt. Anschließend modifiziert man die Oberflächen der Substrate durch Behandlung mit n-Octadecyltrimethoxysilan (OTS, C₁₈H₃₇Si(OCH₃)₃). Dazu wurden in einem Vakuumexsikkator einige Tropfen OTS (erhältlich von Aldrich Chem. Co.) auf die ggf. erwärmte Substratoberfläche (etwa 100 °C) gegeben. Der Exsikkator wurde unmittelbar anschließend evakuiert (25 mm Hg) und die Substrate 5 Stunden unter Vakuum belassen. Anschließend wurden die Substrate 15 Minuten bei 110 °C gebacken, mit Isopropanol gespült und in einem Luftstrom getrocknet. Die Verbindungen der Formel I wurden als dünne Filme mit einer Schichtdicke von etwa 40 nm mittels Vakuumabscheidung auf den Substratoberflächen aufgebracht. Die Auftragrate betrug 1,0 Ä/s bei 10⁻⁵ Torr. Zur Herstellung von Bauteilen mit Top-Kontakten wurden Source- und Drain-Elektroden aus Gold mittels Lochmaske auf den organischen Halbleiterfilmen abgeschieden. Die Kanallänge betrug 2000 µm und die Kanalbreite 200 µm. Die elektrischen Eigenschaften der OFETs wurden mittels Keithley 4200-SCS Halbleiter-Parameter-Analysator ermittelt.

### II. Herstellung von Halbleitersubstraten mittels Rotationsbeschichtung (spin coating)

Als Substrate wurden n-dotierte Siliciumwafer (2,5 x 2,5 cm, Leitfähigkeit < 0,004 Q ⁻¹cm) mit thermisch abgeschiedener Oxidschicht (300 nm) als Dielektrikum (flächenbezogene Kapazität Cᵢ = 10 nF/cm²) eingesetzt. Die Oberflächen der Substrate wurden durch Waschen mit Aceton, gefolgt von Isopropanol, gereinigt. Anschließend modifiziert man die Oberflächen der Substrate durch Behandlung mit n-Octadecyltrimethoxysilan (OTS, C₁₈H₃₇Si(OCH₃)₃), wie für das PVD-Verfahren beschrieben. Die Verbindungen der Formel I wurden als dünne Filme, mittels Rotationsbeschichtung (800 Upm, 30 s) auf den Wafern aufgebracht. Als Lösungsmittel wurde Dichlormethan oder Tetrahydrofuran eingesetzt.

### Beispiel 1 (nicht erfindungsgemäß)

Mischung aus 1,7-Difluor-N,N'-bis(2,6-diisopropylphenyl)perylen-3,4:9,10-tetracarbonsäurediimid und 1,6-Difluor-N,N'-bis(2,6-diisopropylphenyl)perylen-3,4:9,10-tetracarbonsäurediimid

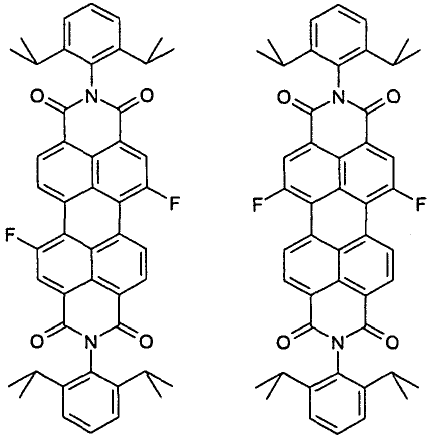

Eine Mischung aus 75 ml trockenem Sulfolan, 4,34 g (5 mmol), 1,7-Dibrom-N,N'-bis(2,6-diisopropylphenyl)-perylen-3,4-9,10-tetracarbonsäurediimid (das herstellungsbedingt noch ca. 25 % 1,6-Dibromisomer enthält) und 0,26 g (1 mmol) 18-Krone-6 wird auf 160 °C erhitzt. Bei dieser Temperatur gibt man 1,74 g (30 mmol) trockenes Kaliumfluorid dazu und hält das Reaktionsgemisch 90 Minuten bei dieser Temperatur. Anschließend kühlt man auf Raumtemperatur ab, gießt in 500 ml Wasser und filtriert. Der Niederschlag wird wiederholt mit Wasser gewaschen und im Vakuum getrocknet. Der Rückstand wird durch Chromatographie (Toluol/Essigester 50:1) gereinigt. Man erhält 2,34 (63 %) eines orangefarbenen Feststoffes.

R_{f}(Toluol/Essigester 10:1) = 0,56

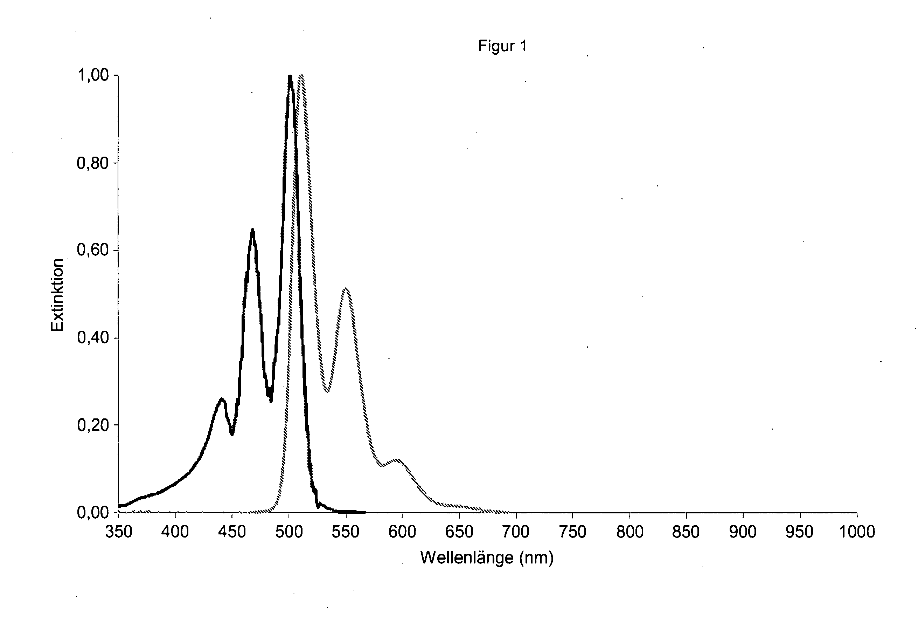

Figur 1 zeigt das Absorptions- und Fluoreszenzspektrum in Methylisobutyrat.

λₘₐₓ (CH₂Cl₂) = 511 nm/130 l·g⁻¹cm⁻¹ ,477 nm/75 l·g⁻¹cm⁻¹, 448 nm/26 l·g⁻¹cm⁻¹

Die Röntgendiffraktometriedaten von Toluolsolvaten des 1,7-Difluor-N,N'-bis(2,6-Diisopropylphenyl)perylen-3,4:9,10-tetracarbonsäurediimids wurden mit einem Bruker AXS CCD Detektor unter Verwendung monochromatischer CuK_{α}-Strahlung bestimmt. Die Asymmetrieeinheit umfasst zwei kristallographisch unabhängige Hälften des lmids und zwei Moleküle Toluol. Kristalldaten: 2C₄₀H₃₈F₄N₂O₄·4C₇H₈, rote Prismen, 0.30 x 0.10 x 0.07 mm, monoklin, Raumgruppe P2₁/c, a= 17.040(2), b= 11.320(1), c= 25.137(2) Å, *β* = 97.135(6), *V* = 4811.3(X)Å³, *Z=* 4, *ρ_{calc}* = 1.388 g/cm³, µ = 0.105 mm⁻¹, *F*-(000) = 1968, *T=* 100(2) K, *R₁* = 0.052, 4828 unabhängige Reflexe und 653 Parameter.

### Beispiel 2 (nicht erfindungsgemäß)

Mischung aus 1,7-Difluor-N,N'-biscyclohexyl-perylen-3,4:9,10-tetracarbonsäurediimid und 1,6-Difluor-N,N'-biscyclohexyl-perylen-3,4:9,10-tetracarbonsäurediimid

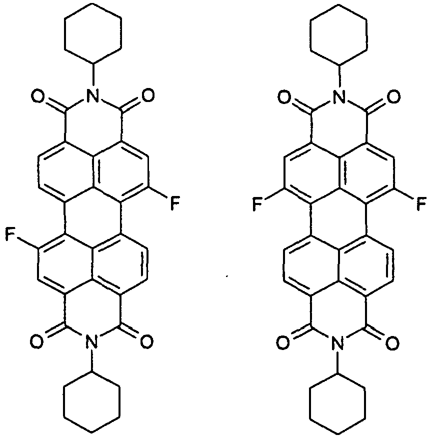

In 40 ml trockenes Sulfolan gibt man 37 mg (0,14 mmol) 18-Krone-6, 1,0 g (17,2 mmol) KF und 0,5 g (0,7 mmol) 1,7-Dibrom-N,N'-biscyclohexyl-perylen-3,4:9,10-tetracarbonsäurediimid (das herstellungsbedingt noch ca. 25 % 1,6-Isomer enthält). Das Gemisch wird auf 160 °C erwärmt und eine Stunde bei dieser Temperatur gerührt. Anschließend kühlt man auf Raumtemperatur ab und gießt das Reaktionsgemisch auf Kochsalzlösung. Der ausgefallene Niederschlag wird filtriert und mehrfach mit Wasser gewaschen und im Vakuum getrocknet. Das Produkt wird durch präparative Dünnschichtchromatographpie mit Dichlormethan gereinigt.
R_{f}(Toluol/Essigester 10:1) = 0,64

### Beispiel 3 (nicht erfindungsgemäß)

### 1,6,7,12-Tetrafluor-N,N'-bis(2,6-diisopropylphenyl)perylen-3,4:9,10-tetracarbonsäurediimid

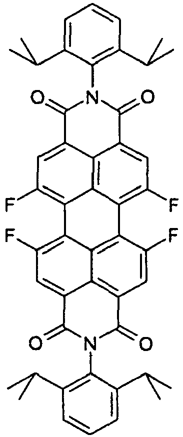

Eine Mischung aus 80 ml Sulfolan, 4,24 g (5 mmol) 1,6,7,12-Tetrachloro-N,N'-bis(2,6-diisopropylphenyl)perylen-3,4:9,10-tetracarbonsäurediimid, 0,52 g (2 mmol) 18-Krone-6 und 3,48 g (60 mmol) KF wird 16 Stunden auf 150 °C erhitzt. Das Reaktionsgemisch wird abgekühlt und durch Zugabe von 100 ml Ethanol ausgefällt, abfiltriert und der Rückstand mit Wasser gewaschen und getrocknet. Das Produkt wird durch präparative Dünnschichtchromatographie (Petrolether/Essigester 8:1) gereinigt.

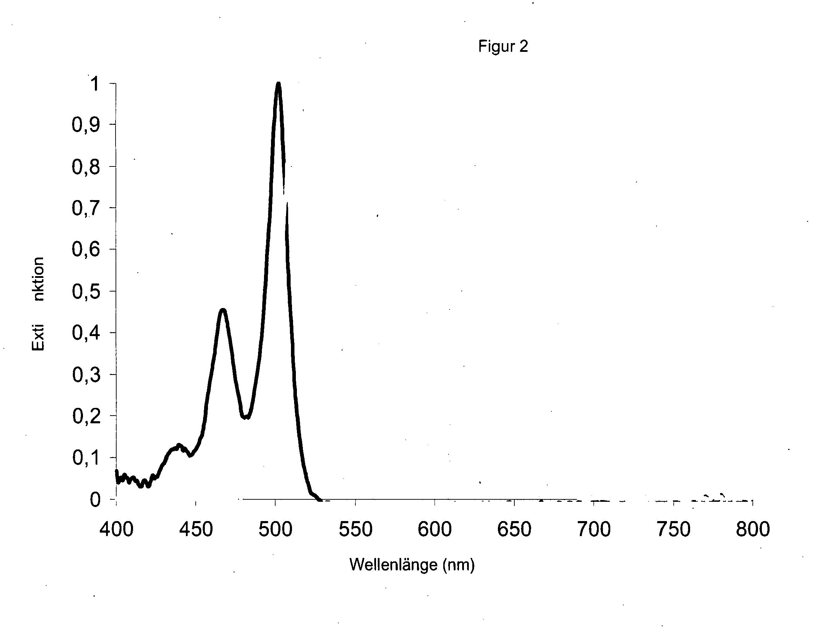

Figur 2 zeigt das UV-Spektrum des Produkts in Dichlormethan.
λ _{max abs} (CH₂Cl₂) = 440 nm, 467 nm, 502 nm
λ ₘₐₓ em (CH₂Cl₂) = 514 nm, 554 nm, 601 nm

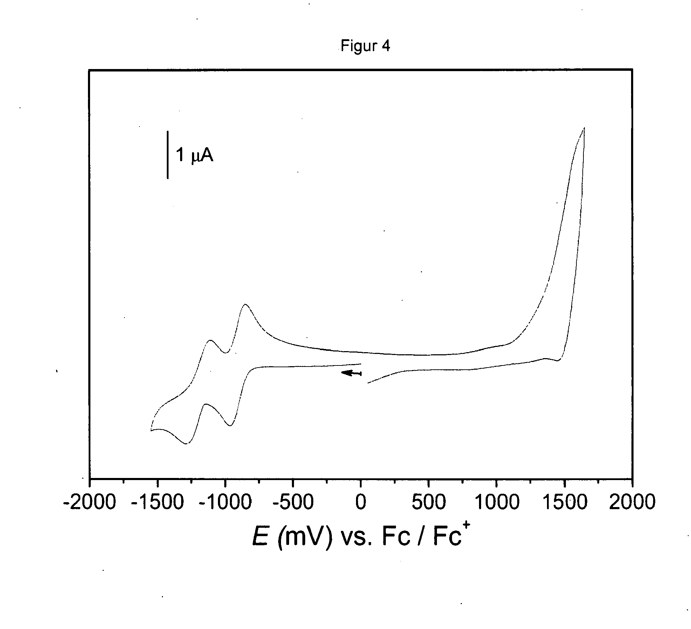

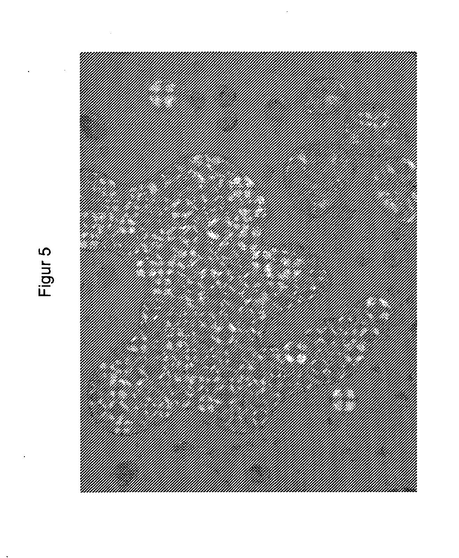

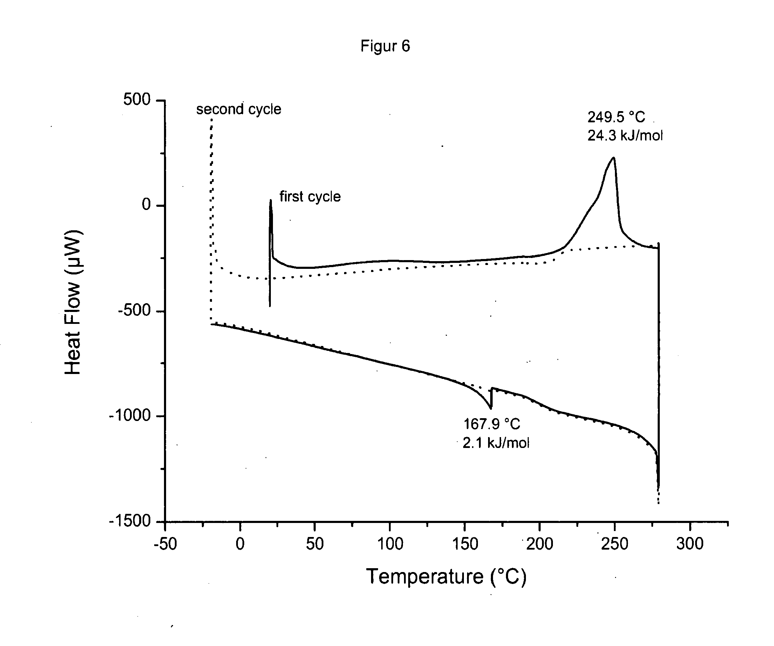

Das Cyclovoltamogramm des 1,6,7,12-Tetrafluor-N,N'-bis(2,6-diisopropylphenyl)-perylen-3,4:9,10-tetracarbonsäurediimids ist in Figur 4 abgebildet. Eine Polarisationsmikroskopaufnahme ist in Figur 5 abgebildet und die DSC-Kurve ist in Figur 6 abgebildet.

### Beispiel 4 (nicht erfindungsgemäß)

Mischung aus 1,7-Difluorperylen-3,4:9,10-tetracarbonsäurebisanhydrid und 1,6-Difluorperylen-3,4:9,10-tetracarbonsäurebisanhydrid

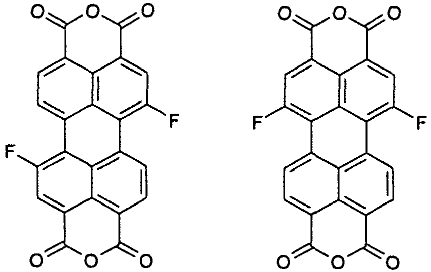

Mischung aus 50 ml Sulfolan, 1,9 g (7,2 mmol) 18 Krone 6 und 2,09 g (36 mmol) Kaliumfluorid wird auf 160 °C erhitzt und eine Suspension von 1,0 g (1,8 mmol) 1,7-Dibrom-perylen-3,4:9,10-tetracarbonsäurebisanhydrid (das herstellungsbedingt noch ca. 25 % 1,6-Isomer enthält) in 75 ml Sulfolan innerhalb von 30 Minuten zugetropft. Man hält das blaue Reaktionsgemisch eine Stunde bei 160 °C. Nach Abkühlen des Reaktionsgemischs gießt man auf Kochsalzlösung, filtriert und wäscht mehrfach mit Wasser. Gemäß massenspektroskopischer Untersuchung (MALDI) besteht das Produkt überwiegend aus Difluorperylentetracarbonsäurebisanhydrid. Die Verbindung wurde durch dreimalige Sublimation in einer Dreizonensublimationsapparatur gereinigt.

Es werden OFETs gemäß der allgemeinen Vorschrift für das PVD-Verfahren hergestellt. Die Ergebnisse sind in nachfolgend dargestellt.

| Substrattemperatur | | Raumtemperatur | 125 °C |
|---|---|---|---|
| nur unter Schutzgas gehandhabt | Mobilität (cm²/Vs) | 2,3 x 10⁻⁴ | 3,1 x 10⁻⁴ |
| | on/off-Verhältnis | 635 | 5284 |
| | Vₜₕ (V) | 17 | 24 |
| an der Atmosphäre gehandhabt | Mobilität (cm²/Vs) | 8,2 x 10⁻⁵ | 2,4 x 10⁻⁴ |
| | on/off-Verhältnis | 497 | 1449 |
| | Vth (V) | 20 | 28 |

### Beispiel 5 (nicht erfindungsgemäß)

Mischung aus 1,7-Difluor-N,N'-dicyclohexyl-perylen-3,4:9,10-tetracarbonsäurediimid und 1,6-Difluor-N,N'-dicyclohexyl-perylen-3,4:9,10-tetracarbonsäurediimid

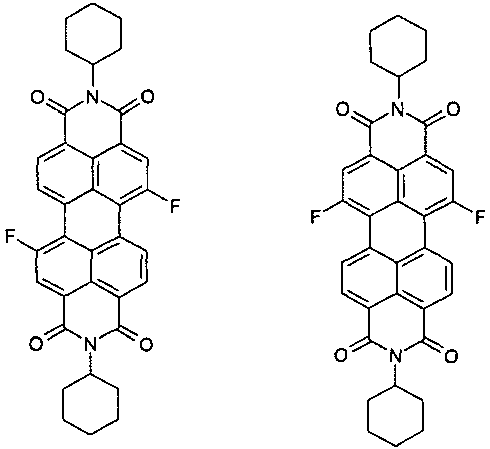

Eine Mischung aus 100 mg (0,14 mmol) 1,7-Dibrom-N,N'-dicylohexyl-perylene3,4:9,10-tetracarbonsäurediimid (enthält ca. 25 % 1,6 Isomer), 84 mg (1,4 mmol) KF und 1 mg (N,N-Dimethylimidazolidino)-tetramethylguanidiniumchlorid werden in 3 ml trockenem Sulfolan 6,5 Stunden auf 180 °C erhitzt. Das Reaktionsgemisch wird auf Raumtemperatur abgekühlt und durch Zugabe von 10 ml Wasser das Produkt ausgefällt. Der Niederschlag wird filtriert, mehrfach mit Wasser gewaschen und im Vakuum getrocknet. Nach Chromatographie mit Dichlormethan/n-Pentan 4:1 und 3:2 erhält man 38 mg (46 %) eines orangefarbenen Feststoffes.

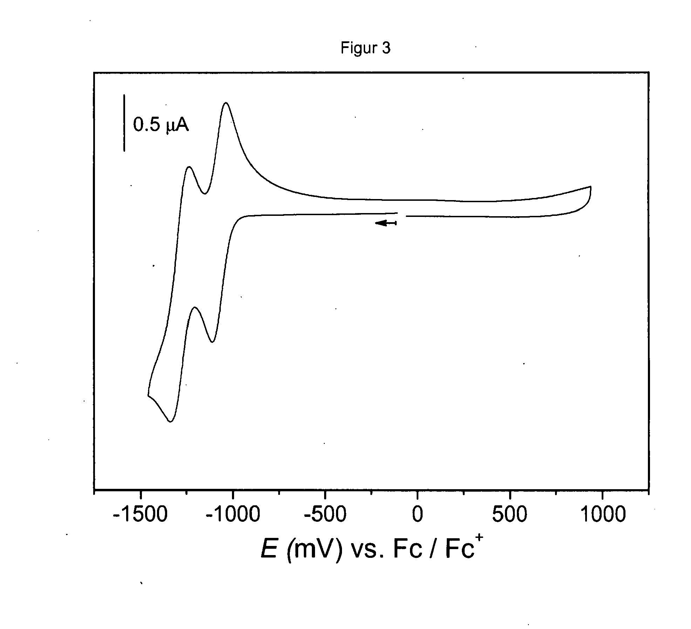

Das Cyclovoltamogramm des 1,7-Difluor-N,N'-biscyclohexyl-perylen-3,4:9,10-tetra-carbonsäurediimids ist in Fig. 3 abgebildet.

### Beispiel 6 (nicht erfindungsgemäß)

Mischung aus 1,7-Difluor-N,N'-dicyclohexyl-perylen-3,4:9,10-tetracarbonsäurediimid und 1,6-Difluor-N,N'-dicyclohexyl-perylen-3,4:9,10-tetracarbonsäurediimid durch Imidierung eines Difluorperylen-3,4:9,10-tetracarbonsäurebisanhydridgemischs

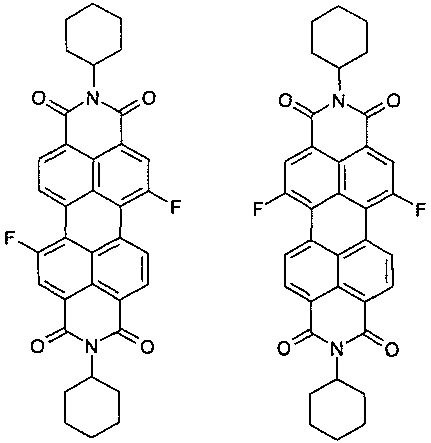

40 mg (93 µmol) 1,7-Difluoroperylen-3,4:9,10-tetracarbonsäurebisanhydrid (gemäß Beispiel 4, welches ca. 25 % 1,6-Difluorisomer enthält) werden mit 86,7 mg (874 µmol) Cyclohexylamin und 20,0 mg Zinkacetat und 5 ml Chinolin zwei Stunden auf 180 °C erhitzt. Das Reaktionsgemisch wird auf Raumtemperatur abgekühlt, in verdünnte Salzsäure gegossen, der Niederschlag abfiltriert, mit Wasser gewaschen und im Vakuum getrocknet. Der Rückstand wurde durch Chromatographie, wie in Beispiel 5 beschrieben, gereinigt.

### Beispiel 7 (nicht erfindungsgemäß)

Mischung aus 1,7-Difluor-N,N'-di-(n-octyl)-perylen-3,4:9,10-tetracarbonsäurediimid und 1,6-Difluor-N,N'-di-(n-octyl)-perylen-3,4:9,10-tetracarbonsäurediimid

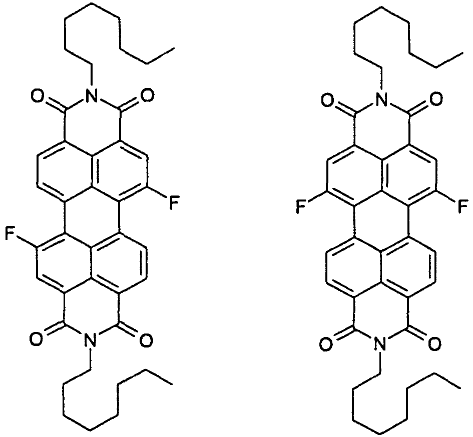

Eine Mischung aus 97 mg (0,13 mmol) 1,7-Dibrom-N,N'-di-(n-octyl)perylen (welches ca. 25 % 1,6 Isomer enthält), 85 mg (1,46 mmol) KF und 6 mg (N,N-Dimethylimidazolidino)-tetramethylguanidiniumchlorid (CNC⁺) werden in 2,9 g Sulfolan 16 Stunden auf 170 °C erhitzt. Das Reaktionsgemisch wird aufgearbeitet wie in Beispiel 5. Nach Chromatographie mit Chloroform/Pentan 7:3 und 3:2 und anschließender Kristallisation aus Toluol erhält man 22,8 mg (28 %) eines roten Feststoffs.

### Beispiel 8 (nicht erfindungsgemäß)

Mischung aus 1,7-Difluor-N,N'-di-(n-octyl)-perylen-3,4:9,10-tetracarbonsäurediimid und 1,6-Difluor-N,N'-di-(n-octyl)-perylen-3,4:9,10-tetracarbonsäurediimid durch Imidierung eines Difluorperylen-3,4:9,10-tetracarbonsäurebisanhydridgemischs

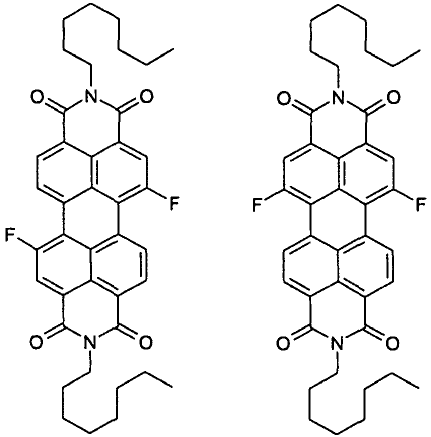

44,0 mg 1,7-Difluoroperylen-3,4:9,10-tetracarbonsäurebisanhydrid (gemäß Beispiel 4, welches ca. 25 % 1,6-Difluorisomer enthält) werden mit 78,1 mg (0,6 mmol) n-Octylamin, 20 mg Zink(II)-acetat und 5 ml Chinolin zwei Stunden auf 180 °C erhitzt. Das Reaktionsgemisch wird wie in Beispiel 6 aufgearbeitet. Nach Säulenchromatographie mit Chloroform n-Hexan 4:1 und 7:3 wird das Produkt erhalten.

### Beispiel 9 (nicht erfindungsgemäß)

### 1,6,7,12-Tetrafluor-N,N'-bis(n-octyl)perylen-3,4:9,10-tetracarbonsäurediimid

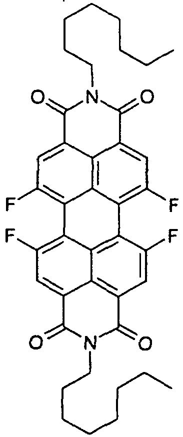

In Anlehnung an Beispiel 5 wird die Umsetzung von 1,6,7,12-Tetrachlor-N,N'-bis(n-octyl)perylen-3,4:9,10-tetrcarbonsäurediimid durchgeführt. Die Zielverbindung wird nach Säulenchromatographie (CH₂Cl₂ : n-Pentane) = 3:2 und 7:3 als orangefarbener Feststoff erhalten.

Die Röntgendiffraktometriedaten des 1,6,7,12-Tetrafluor-N,N'-bis(n-octyl)perylen-3,4:9,10-tetracarbonsäurediimids wurden mit einem Bruker AXS CCD Detektor unter Verwendung monochromatischer MoK_{α}-Strahlung bestimmt. Kristalldaten: C₄₀H₃₈F₄N₂O₄, orangefarbige Nadeln, 0.30 x 0.10 x 0.07 mm, triklin, Raumgruppe: P-1, *a* = 7.8097(4), *b* = 12.1717(7), *c* = 18.2517(11) Å, *α* = 75.6050(10), *β* = 80.7910(10), *Y* = 80.2050(10)°, *V* = 1643.34(16)Å³, *Z* = 2, *ρ_{calc}* = 1.388 g/cm³, µ = 0.105 mm⁻¹, *F-*(000) = 720, T= 193(2) K, *R₁* = 0.1295, *wR₂* = 0.2112, 6490 unabhängige Reflexe [2*θ* ≤ 52.16°] und 470 Parameter.

### Beispiel 10 (nicht erfindungsgemäß)

### N,N'-Bis(heptafluorbutyl)-1,7-difluor-3,4:9,10-tetracarbonsäurebisimid

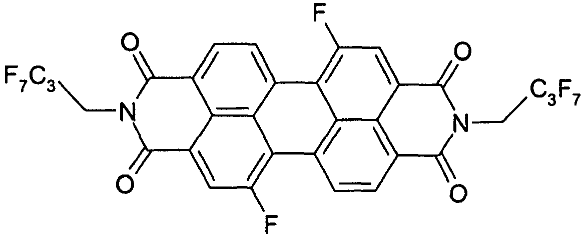

### 10.1 N,N'-Bis(heptafluorbutyl)-1,7-dibrom-3,4:9,10-tetracarbonsäurebisimid

460 mg (0,836 mmol) 1,7-Dibrom-3,4:9,10-tetracarbonsäurebisanhydrid (hergestellt wie beschrieben in F. Würther, V. Stepanenko, Z. Chen, C. R. Saha-Möller, N. Kocher, D. Stalke, J. Org. Chhhem. 2004, 69, 7933-7939) werden in 10 ml trockenem N-Methylpyrrolidon (NMP) 30 min in Ultraschallbad beschallt. Danach werden 0,532 g (2,68 mmol) 2,2,3,3,4,4,4-Heptafluorbutylamin in 7 ml N-Methylpyrrolidon und 0,34 g Essigsäure zugegeben. Die Reaktionsmischung wird 5 h auf 90 °C erhitzt. Nach Abkühlen auf Raumtemperatur wird auf 2 N HCl geschüttet und der ausfallende Feststoff abfiltriert. Nach Trocknen im Vakuum wird säulenchromatographisch (Dichlormethan) aufgereinigt, wobei 690 mg (94%) der Titelverbindung erhalten werden.

¹H-NMR (400 MHz, CDCl₃, TMS): δ = 5,04 (t, 4H, ³J (H,F) = 15,3 Hz), 8,78 (d, 2H, ³J = 8,1 Hz), 9,00 (s, 2H), 9,54 (d, 2H, ³J = 8,2 Hz).

### 10.2 N,N'-Bis(heptafluorbutyl)-1,7-difluor-3,4:9,10-tetracarbonsäurebisimid

40,0 mg (0,043 mmol) N,N'-Bis(heptafluorbutyl)-1,7-dibrom-3,4:9,10-tetracarbonsäurebisimid, 30 mg KF und 10 mg 18-Krone-6 in 1,5 ml Sulfolan werden 45 min auf 160 °C erhitzt. Das abgekühlte Gemisch wird auf Wasser gegeben und der ausfallende Feststoff abfiltriert. Der getrocknete Feststoff wird in Dichlormethan gelöst und säulenchromatographisch (Dichlormethan) gereinigt, wobei 11 mg (32%) der Titelverbindung erhalten werden.

¹H-NMR (400 MHz, CDCl₃, TMS): δ = 5,04 (t, 4H, ³J = 15,8 Hz), 8,62 (d, 2H, ³J = 13,5 Hz), 8,81 (d, 2H, ³J = 7,6 Hz), 9,23 (d, 2H, ³J = 5,3 Hz);

HR-MS (ESI (neg.-mode, Chloroform, Acetonitril)): 790,03903 (M), berechnet 790,03906 (C₃₂H₁₀F₁₆N₂O₄);

Cyclovoltamogramm (CH₂Cl₂, 0,1 M Tetrabutylammoniumhexafluorophosphat (TBAHFP), vs. Ferrocen):
E^{red}_{1/2} (PBI/PBI⁻)= -0.92 V
E^{red}_{1/2} (PBI⁻/PBI²⁻)= -1.14. V.

PBI = Bisperylenimid

Es werden OFETs gemäß der allgemeinen Vorschrift für das Spin-coating-Verfahren hergestellt. Lösungsmittel: Dichlormethan; Mobilität: 3,8 x 10⁻⁴ cm²/Vs.

Es werden OFETs gemäß der allgemeinen Vorschrift für das PVD-Verfahren hergestellt. Substrattemperatur: 125 °C, Mobilität: 0,223 cm²/Vs.

Setzt man als Edukt 1,7-Dibrom-perylen-3,4:9,10-tetracarbonsäurebisanhydrid, das noch etwa 15 % 1,6-Isomer enthält, ein, so erhält man ein Isomerengemisch aus N,N'-Bis(heptafluorbutyl)-1,7-difluor-3,4:9,10-tetracarbonsäurebisimid und N,N'-Bis(heptafluorbutyl)-1,6-difluor-3,4:9,10-tetracarbonsäurebisimid. Mit diesem Gemisch wurde eine Solarzelle hergestellt.

### Beispiel 11 (nicht erfindungsgemäß)

### N,N'-Bis(pentafluorphenyl)-1,7-difluor-3,4:9,10-tetracarbonsäurebisimid

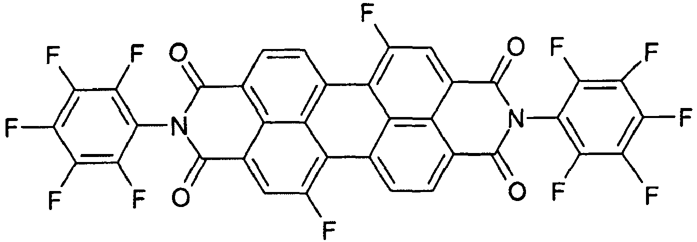

### 11.1 N,N'-Bis(pentafluorphenyl)-1,7dibrom-3,4:9,10-tetracarbonsäurebisimid

420 mg (0,76 mmol) 1,7-Dibrom-3,4:9,10-tetracarbonsäurebisanhydrid, 1,20 g (6,56 mmol) Pentafluoranilin und 200 mg Zink(II)-acetat werden 5 h auf 140 °C erhitzt. Nach Abkühlen auf Raumtemperatur wird das feste Gemisch in Dichlormethan gelöst und auf 2 N HCl geschüttet. Es wird mehrere Male mit Dichlormethan extrahiert, die organischen Phasen vereinigt, getrocknet und eingeengt. Es wird säulenchromatographisch (Dichlormethan) aufgereinigt, wobei 90 mg (13%) der Titelverbindung erhalten werden.

¹H-NMR (400 MHz, CDCl₃, TMS): δ = 8,12 (d, 2H, ³*J*= 8,1 Hz), 9,03 (s, 2H), 9,58 (d, 2H, ³*J*= 8,1 Hz);

HR-MS (ESI (neg.-mode, Chloroform, Acetonitril)): 877,85419 (M), berechnet 877,85401 (C₃₆H₆Br₂F₁₀N₂O₄).

### 11.2 N,N'-Bis(pentafluorphenyl)-1,7-difluor-3,4:9,10-tetracarbonsäurebisimid

128 mg (0,145 mmol) N,N'-Bis(pentafluorphenyl)-1,7-dibrom-3,4:9,10-tetracarbonsäurebisimid, 100 mg KF und 50 mg 18-Krone-6 werden in 2,5 ml Sulfolan 45 min auf 160 °C erhitzt. Das abgekühlte Gemisch wird auf Wasser gegeben und der ausfallende Feststoff abfiltriert. Der getrocknete Feststoff wird in Dichlormethan gelöst und säulenchromatographisch (Dichlormethan) aufgereinigt, wobei 50 mg (45 %) der Titelverbindung erhalten werden.

¹H-NMR (400 MHz, CDCl₃, TMS): δ = 8,66 (d, 2H, ³*J*= 13,3 Hz), 8,85 (d, 2H, ³*J*= 7,5 Hz), 9,29 (m, 2H);

HR-MS (ESI (neg.-mode, Chloroform)): 792,98290 (M+Cl⁻), berechnet 792,98300 (C₃₆H₆F₁₂N₂O₄Cl);

Cyclovoltamogramm (CH₂Cl₂, 0,1M TBAHFP, vs. Ferrocen):
E^{red}_{1/2} (PBI/PBI⁻)= -0.86 V
E^{red}_{1/2} (PBI⁻/PBI²⁻)= -1.08. V.

### Beispiel 12 (nicht erfindungsgemäß)

### N,N'-Di-1-phenylethyl-1,7-difluor-3,4:9,10-tetracarbonsäurebisimid

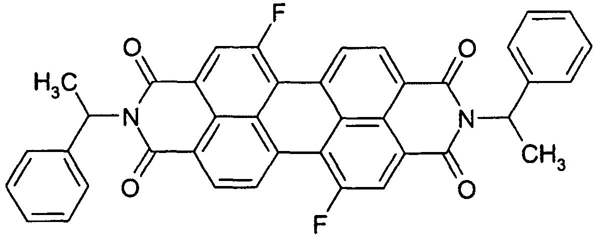

### 12.1 N,N'-Di-1-phenylethyl-1,7-dibrom-3,4:9,10-tetracarbonsäurebisimid

2,00 g (3,63 mmol) 1,7-Dibrom-3,4:9,10-tetracarbonsäurebisanhydrid werden in 8 ml destillierten Chinolin zusammen mit 0,5 ml Methylbenzylamin und 10 mg Zink(II)-acetat auf 130 °C erhitzt. Nach 3h wird auf Raumtemperatur abgekühlt und auf 200 ml 2 N HCl gegeben. Der ausfallende Feststoff wird abfiltriert, getrocknet und säulenchromatographisch aufgereinigt (Dichlormethan), wobei 1,31 g (1,74 mmol, 48 %) der Titelverbindung erhalten werden.

¹H-NMR (400 MHz, CDCl₃, TMS): δ = 2,02 (d, 6H, ³*J*= 7,2 Hz), 6,54 (q, 2H, ³*J*= 7,2 Hz), 7,2-7,3 (m, 2H), 7,34 (m, 4H), 7,51 (m, 4H), 8,67 (d, 2H, ³*J*= 8,1 Hz), 8,90 (s, 2H), 9,46 (d, 2H, ³*J*= 8,2 Hz);

HR-MS (apci (pos.-mode)): 755,0178 (M+H)⁺, berechnet 755,0176 (C₄₀H₂₄Br₂N₂O₄).

### 12.2 N,N'-Di-1-phenylethyl-1,7-difluor-3,4:9,10-tetracarbonsäurebisimid

651 mg (1,36 mmol) N,N'-Di-1-phenylethyl-1,7-dibrom-3,4:9,10-tetracarbonsäurebisimid, 650 mg KF und 200 mg 18-Krone-6 in 13 ml Sulfolan werden 1 h auf 160 °C erwärmt. Das abgekühlte Gemisch wird auf 200 ml Wasser gegeben und der ausfallende Feststoff abfiltriert und mit Wasser nachgewaschen. Der getrocknete Feststoff wird in Dichlormethan gelöst und säulenchromatographisch (Dichlormethan) gereinigt. Es werden 330 mg (0,52 mmol, 60 %) der Titelverbindung erhalten.

¹H-NMR (400 MHz, CDCl₃, TMS): δ = 2,04 (d, 6H, ³*J*= 7,2 Hz), 6,54 (q, 2H, ³*J*= 7,2 Hz), 7,2-7,3 (m, 2H), 7,34 (m, 4H), 7,51 (m, 4H), 8,46 (d, 2H, ³*J*= 13,7 Hz), 8,63 (d, 2H, ³*J*= 7,8 Hz), 9,04 (m, 2H);

HR-MS (apci (pos.-mode)): 635,1776 (M+H)⁺, berechnet 635,1777 (C₄₀H₂₅F₂N₂O₄).

### Beispiel 13 (nicht erfindungsgemäß)

### N,N'-Bis(2-ethylhexyl)-1,7-difluor-3,4:9,10-tetracarbonsäurebisimid

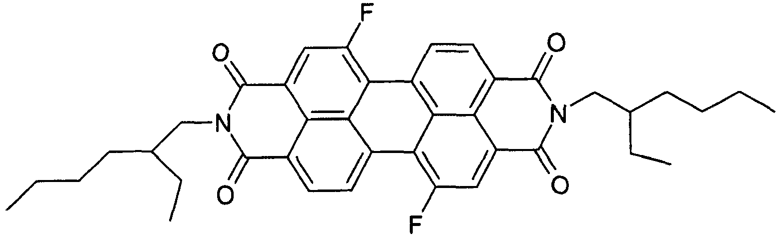

### 13.1 N,N'-Bis(2-ethylhexyl)-1,7-dibrom-3,4:9,10-tetracarbonsäurebisimid

2,00 g (3,63 mmol) 1,7-Dibrom-3,4:9,10-tetracarbonsäurebisanhydrid, 4 ml 2-Ethylhexylamin und 25 ml Propionsäure werden 3,5 h auf 142 °C erhitzt und danach auf 150 ml Wasser gegeben. Der ausfallende Feststoff wird abfiltriert, getrocknet und säulenchromatographisch (Dichlormethan) aufgereinigt, wobei 1,38 g (50 %) der Titelverbindung erhalten werden.

¹H-NMR (400 MHz, CDCl₃, TMS):

δ = 0,88-1,00 (m, 12H), 1,25-1,43 (m, 16H), 1,95 (m, 2H), 4,16 (m, 4H), 8,70 (d, 2H, ³J = 8,2 Hz), 8,94 (s, 2H), 9,50 (d, 2H, ³J = 8,1 Hz).

### 13.2 N,N'-Bis(2-ethylhexyl)-1,7-difluor-3,4:9,10-tetracarbonsäurebisimid

Die Titelverbindung wird analog zu dem in Beispiel 12.2 beschriebenen Verfahren unter Verwendung von N,N'-Bis(2-ethylhexyl)-1,7-dibrom-3,4:9,10-tetracarbonsäurebisimid anstelle von N,N'-Di-1-phenylethyl-1,7-difluor-3,4:9,10-tetracarbonsäurebisimid hergestellt, wobei jedoch 2 h auf 160 °C erwärmt wird. Die Titelverbindung wird in einer Ausbeute von 50 % erhalten.

### Beispiel 14 (nicht erfindungsgemäß)

### 1,7-Difluor-3,4:9,10-tetracarbonsäurebisanhydrid

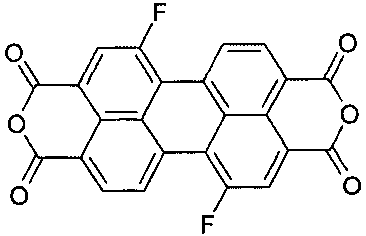

In Anlehnung an das in Beispiel 19 beschriebene Verfahren wird die Umsetzung von N,N'-Bis(2-ethylhexyl)-1,7-difluor-3,4:9,10-tetracarbonsäurebisimid aus Beispiel 13 durchgeführt. Ausbeute: 90 %.

### Beispiel 15 (nicht erfindungsgemäß)

### N,N'-Bis(heptafluorbutyl)-1,6,7,12-tetrafluor-3,4:9,10-tetracarbonsäurebisimid

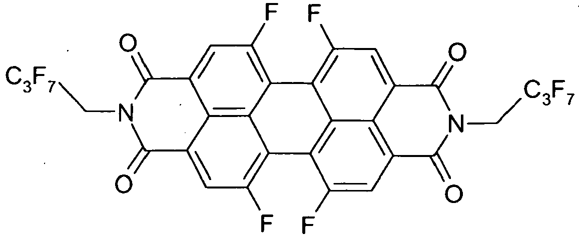

### 15.1 N,N'-Bis(heptafluorbutyl)-1,6,7,12-tetrachlor-3,4:9,10-tetracarbonsäurebisimid

1,00 g (1,89 mmol) 1,6,7,12-Tetrachlor-3,4:9,10-tetracarbonsäurebisanhydrid werden in 20 ml trockenem N-Methylpyrrolidon 30 min ins Ultraschallbad gestellt. Danach werden 1,06 g (5,35 mmol) 2,2,3,3,4,4,4-Heptafluorbutylamin in 15 ml N-Methylpyrrolidon und 0,68 g Essigsäure zugegeben. Die Reaktionsmischung wird 5 h auf 90 °C erhitzt. Nach Abkühlen auf Raumtemperatur wird auf 2n HCl geschüttet und der ausgefallene Feststoff abfiltriert. Nach Trocknen im Vakuum wird säulenchromatographisch (Dichlormethan) aufgereinigt, wobei 1,30 g (77 %) der Titelverbindung erhalten werden.

¹H-NMR (400 MHz, CDCl₃, TMS): δ =5,04 (t, 4H, ³J (H,F) = 15,7 Hz), 8,75 (s, 4H);

HR-MS (ESI (neg.-mode. Chloroform, Acetonitril)): 924,86968 (M+Cl⁻), berechnet 924,87086 (C₃₂H₈Cl₅F₁₄N₂O₄);

Cyclovoltamogramm (CH₂Cl₂, 0,1M TBAHFP, vs, Ferrocen):
E^{red}_{1/2} (PBI/PBI⁻) = -0,74 V
E^{red}_{1/2} (PBI⁻/PBI²⁻)= -0,95, V.

### 15.2 N,N'-Bis(heptafluorbutyl)-1,6,7,12-tetrafluor-3,4:9,10-tetracarbonsäurebisimid

700 mg (0,956 mmol) N,N'-Bis(heptafluorbutyl)-1,6,7,12-tetrachlor-3,4:9,10-tetra-carbonsäurebisimid , 1,09 g KF und 120 mg (N,N-Dimethylimidazolidino)-tetramethyl-guanidiniumchlorid in 50 ml Sulfolan werden 2 h auf 160 °C erhitzt. Das abgekühlte Gemisch wird auf 700 ml Wasser gegeben und der ausfallende Feststoff abfiltriert und mit Wasser nachgewaschen. Der getrocknete Feststoff wird in Dichlormethan gelöst und säulenchromatographisch (Pentan/Dichlormethan: 1/4) gereinigt. Ausbeute: 63 mg (8,6 %)

¹H-NMR (400 MHz, CDCl₃, TMS): δ = 5,04 (t, 4H, ³J = 15,3 Hz), 8,56 (t, 4H, ³J = 5,2 Hz);

HR-MS (ESI (neg.-mode, Chloroform, Acetonitril)): 860,9886 (M+Cl⁻), berechnet 860,9891 (C₃₂H₈F₁₈N₂O₄Cl);

Cyclovoltamogramm (CH₂Cl₂, 0,1M TBAHFP, vs. Ferrocen):
E^{red}_{1/2} (PBI/PBI⁻)= -0.87 V
E^{red}_{1/2} (PBI⁻/PBI²⁻)= -1.12 V.

Es werden OFETs gemäß der allgemeinen Vorschrift für das Spin-coating-Verfahren hergestellt. Lösungsmittel: Dichlormethan; Mobilität: 4,0 x 10⁻⁴ cm²/Vs.

Es werden OFETs gemäß der allgemeinen Vorschrift für das PVD-Verfahren hergestellt. Substrattemperatur: 125 °C, Mobilität 0,032 cm²/Vs.

### Beispiel 16 (nicht erfindungsgemäß)

### N,N'-Di-1-phenylethyl-1,6,7,12-tetrafluor-3,4:9,10-tetracarbonsäurebisimid

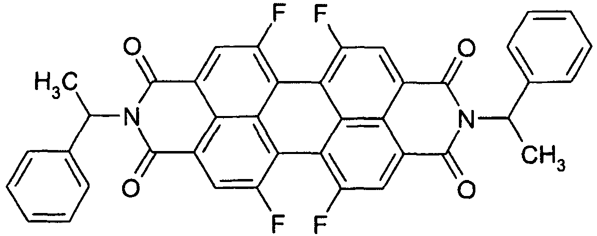

### 16.1 N,N'-Di-1-phenylethyl-1,6,7,12-tetrachlor-3,4:9,10-tetracarbonsäurebisimid

3,00 g (5,66 mmol) 1,6,7,12-Tetrachlor-3,4:9,10-tetracarbonsäurebisanhydrid, 65 ml destilliertes Chinolin, 7,5 ml (65,2 mmol) Methylbenzylamin und 1,00 g Zinkacetat werden auf 180 °C erhitzt. Nach 4 h wird auf Raumtemperatur abgekühlt und auf 300 ml 2 N HCl gegeben. Der ausfallende Feststoff wird abfiltriert und getrocknet. Nach säulenchromatographischer Aufreinigung (Dichlormethan) erhielt man 3,70 g (88 %) der Titelverbindung.

¹H-NMR (400 MHz, CDCl₃, TMS): δ = 2.01 (d, 6H, ³*J*= 7.2 Hz), 6.54 (q, 2H, ³*J*= 7.2 Hz), 7.2-7.3 (m, 2H), 7.34 (m, 4H), 7.51 (m, 4H), 8.64 (s, 4H);

HR-MS (apci (pos.-mode)): 735,0414 (M⁻), berechnet 735,0406 (C₄₀H₂₃Cl₄N₂O₄).

### 16.2 N,N'-Di-1-phenylethyl-1,6,7,12-tetrafluor-3,4:9,10-tetracarbonsäurebisimid

1,00 g (1,36 mmol) N,N'-Di-1-phenylethyl-1,6,7,12-tetrachlor-3,4:9,10-tetra-carbon-säurebisimid, 1,25 g KF und 100 mg (N,N-Dimethylimidazolidino)-tetramethyl-guanidiniumchlorid in 8 ml Sulfolan werden 5h auf 160 °C erhitzt. Das abgekühlte Gemisch wird auf 200 ml Wasser gegeben und der ausfallende Feststoff abfiltriert und mit Wasser nachgewaschen. Der getrocknete Feststoff wird in Dichlormethan gelöst und säulenchromatographisch (Pentan/Dichlormethan: 1/2) gereinigt. Ausbeute: 80 mg (8,8 %).

¹H-NMR (400 MHz, CDCl₃, TMS): δ = 2,01 (d, 6H, ³*J*= 7,0 Hz), 6,54 (q, 2H, ³*J*= 7,3 Hz), 7,26 (m, 2H), 7,34 (m, 4H), 7,51 (m, 4H), 8,44 (t, 4H, ³*J*= 5,3 Hz);

HR-MS (apci (neg.-mode)): 670,1532 (M-), berechnet 670,1521 (C₄₀H₂₂F₄N₂O₄).

### Beispiel 17 (nicht erfindungsgemäß)

### 1,6,7,12-Tetrafluor-3,4:9,10-tetracarbonsäurebisimid

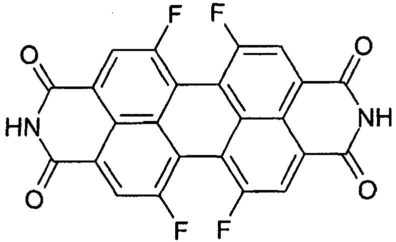

Eine Lösung von 70,0 mg (104 µmol) N,N'-Di-1-phenylethylamin-1,6,7,12-tetrafluor-3,4:9,10-tetracarbonsäurebisimid aus Beispiel 16 in 15 ml Dichlormethan wird auf 0 °C gekühlt. Dann werden langsam 200 µl BBr₃ zugetropft und die Lösung 3 h bei Raumtemperatur gerührt. Das Dichlormethan wird im Vakuum entfernt und der Rückstand mit einer Methanol/Wasser Mischung im Ultraschallbad aufgeschwemmt. Der ausgefallene Feststoff wird abfiltriert und mit Wasser und danach mit Dichlormethan gewaschen. Ausbeute: 45 mg (93 %).

HR-MS (EI (pos.-mode)): 462,0255 (M⁺), berechnet 462.0260 (C₂₄H₆F₄N₂O₄).

Es werden OFETs gemäß der allgemeinen Vorschrift für das PVD-Verfahren hergestellt. Substrattemperatur: 125 °C; Mobilität: 1,4 x 10⁻⁴ cm²/ Vs.

Es werden OFETS gemäß der allgemeinen Vorschrift für das Spin-coating Verfahren hergestellt. Lösungsmittel: Tetrahydrofuran; Mobilität: 1,11 x 10⁻⁶ cm²/ Vs.

### Beispiel 18 (nicht erfindungsgemäß)

### N,N'-2-Ethylhexyl-1,6,7,12-tetrafluor-3,4:9,10-tetracarbonsäurebisimid

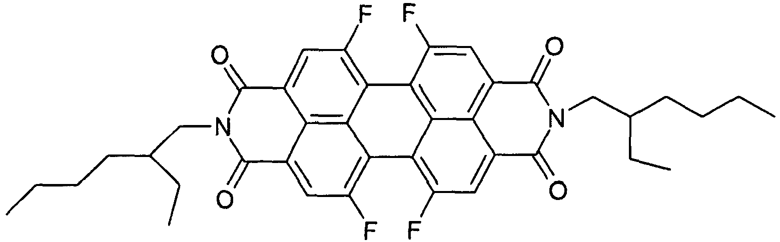

### 17.1 N,N'-2-Ethylhexyl-1,6,7,12-tetrachlor-3,4:9,10-tetracarbonsäurebisimid

3,00 g (5,66 mmol) 1,6,7,12-Tetrachlor-3,4:9,10-tetracarbonsäurebisanhydrid, 16 ml 2-Ethylhexylamin und 35 ml Propionsäure werden 1,5 h auf 142 °C erhitzt und danach auf 150 ml Wasser gegeben. Der ausfallende Feststoff wird abfiltriert, getrocknet und säulenchromatographisch (Dichlormethan) aufgereinigt, wobei 3,30 g (77 %) der Titelverbindung erhalten werden.

¹H-NMR (400 MHz, CDCl₃, TMS): δ =0,88-0,95 (m, 12H), 1,25-1,43 (m, 16H), 1,95 (m, 2H), 4,16 (m, 4H), 8,68 (s, 4H).

### 18.2 N,N'-2-Ethylhexyl-1,6,7,12-tetrafluor-3,4:9,10-tetracarbonsäurebisimid

300 mg (0,400 mmol) N,N'-2-Ethylhexyl-1,6,7,12-tetrachlor-3,4:9,10-tetracarbonsäurebisimid, 250 mg KF und 100 mg (N,N-Dimethylimidazolidino)-tetramethylguanidiniumchlorid in 2 ml Sulfolan werden 2 h auf 160 °C erhitzt. Das abgekühlte Gemisch wird auf 100 ml Wasser gegeben und der ausfallende Feststoff abfiltriert und mit Wasser gewaschen. Der getrocknete Feststoff wird in Dichlormethan gelöst und säulenchromatographisch (Hexan/Dichlormethan: 3/7) aufgereinigt. Ausbeute: 30 mg (8,8 %).

¹H-NMR (400 MHz, CDCl₃, TMS): δ = 0,88-0,97 (m, 12H), 1,25-1,43 (m, 16H), 1,95 (m, 2H), 4,16 (m, 4H), 8,49 (t, 4H, ³J = 5,2 Hz);

HR-MS (apci (neg.-mode)): 686,2783 (M⁻), berechnet 686,2773 (C₄₀H₃₈F₄N₂O₄).

### Beispiel 19 (nicht erfindungsgemäß)

### 1,6,7,12-Tetrafluor-3,4:9,10 tetracarbonsäurebisanhydrid

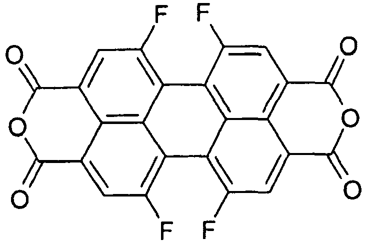

55 mg (0,08 mmol) N,N'-Ethylhexyl-1,6,7,12-tetrafluor-3,4:9,10-tetracarbonsäurebisimid aus Beispiel 18, 300 mg KOH, 200 µl Wasser und 10 ml Butanol werden 2 h auf 75 °C erhitzt. Danach wird das Gemisch auf 200 ml Essigsäure gegeben und 5 h bei etwa 100 °C gerührt. Dann wird mit Wasser verdünnt und das ausfallende Produkt wird abfiltriert und getrocknet. Das Massenspektrum zeigt neben der Titelverbindung eine geringe Verunreinigung mit einem einfach Hydroxy und dreimal Fluor substituierten Anhydrid. Ausbeute: 35 mg (94 %).

### MS (EI (pos.-mode)): 464,1 (M⁺), berechnet 464,0 (C₂₄H₄F₄O₆).

### Beispiel 20

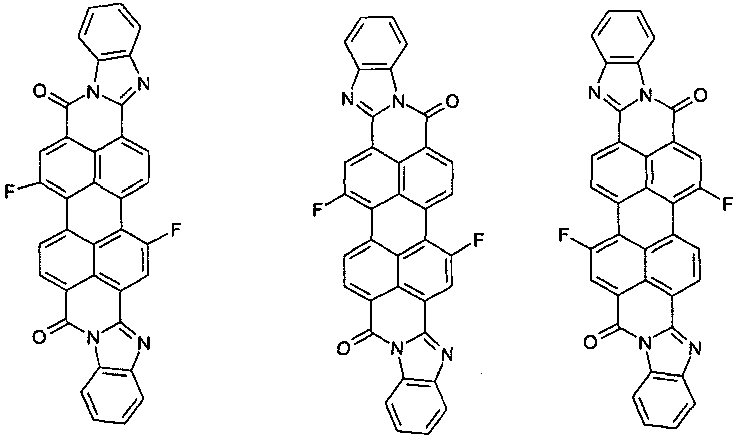

Eine Mischung aus 15 g Phenol, 1,01 g (9,3 mmol) 1,2-Phenylendiamin, 0,37 g (4,7 mmol) Pyrazin werden auf 120°C erwärmt. Danach wird bei dieser Temperatur 1,0 g (2,3 mmol) 1,7-Difluorperylen-3,4:9,10-tetracarbonsäurebisanhydrid aus Beispiel 4 zugegeben und das Gemisch auf 170 °C erwärmt. Nach 2-stündigem Rühren wird das Reaktionsgemisch auf Raumtemperatur abgekühlt und mit 50 ml vollentsalztem Wasser und 10 ml Methanol verdünnt. Der Feststoff wird abfiltriert, mit Ethanol gewaschen und im Vakuum getrocknet. Man erhält 1,21 g (91 %) eines schwarzen Feststoffes.

R_{f}(Trichloressigsäure Toluol 5:1) = 0,12, 0,36, 0,48. Die Verbindungen wurden durch dreimalige Sublimation in einer Dreizonensublimationsanlage gereinigt.

Es werden OFETs gemäß der allgemeinen Vorschrift für das PVD-Verfahren hergestellt. Die Ergebnisse sind nachfolgend dargestellt.

| Substrattemperatur | | Raumtemperatur | 125 °C |
|---|---|---|---|
| Innerhalb der Glovebox | Mobilität (cm²/Vs) | 4,1 x 10⁻⁵ | 6,2 x 10⁻⁵ |
| | Vₜₕ (V) | 52 | 14 |
| Außerhalb der Glovebox | Mobilität (cm²/Vs) | 3,3 x 10⁻⁵ | 5,1 x 10⁻⁵ |
| | Vₜₕ (V) | 64 | 47 |

### Beispiel 21

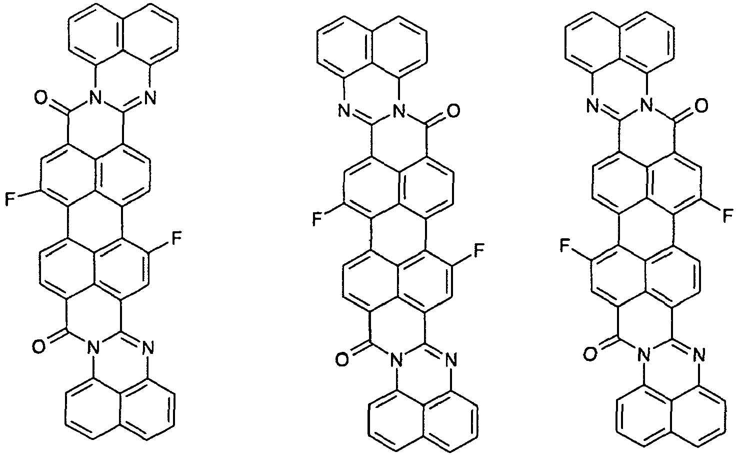

In Anlehnung an Beispiel 20 wird die Umsetzung von 1,7-Difluorperylen-3,4:9,10-tetracarbonsäurebisanhydrid aus Beispiel 4 durchgeführt, jedoch wird 1,8-Diaminonaphthalin anstelle von 1,2-Phenylendiamin eingesetzt. Man erhält ein schwarzes Pulver in 92%iger Ausbeute. R_{f}(Trichloressigsäure:Toluol 5:1) = 0,29, 0,47

### Beispiel 22 (nicht erfindungsgemäß)

Mischung aus 1,7-Difluor-N,N'-dimethyl-perylen-3,4:9,10-tetracarbonsäurediimid und 1,6-Difluor-N,N'-dimethyl-perylen-3,4:9,10-tetracarbonsäurediimid

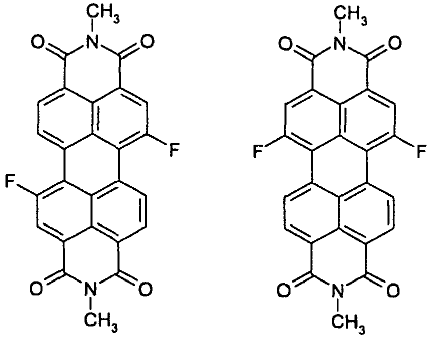

Eine Mischung aus 50 ml Wasser, 5,0 g (0,012 mol) einer Mischung aus 1,7-Difluorperylen-3,4:9,10-tetracarbonsäurebisanhydrid und 1,6-Difluorperylen-3,4:9,10-tetracarbonsäurebisanhydrid aus Beispiel 4 und 2,8 ml einer 41%igen Methylaminlösung (0,048 mol Methylamin) werden 22 Stunden bei Raumtemperatur gerührt. Der Niederschlag wird abfiltriert, mit Wasser gewaschen und im Vakuum getrocknet. Man erhält 4,82 g (89 %) eines braun-schwarzen Feststoffes.

UV-VIS (H₂SO₄) λ ₘₐₓ = 576 nm (89.5 l / cm·g), 544 nm (71.3 l /cm·g)

### Beispiel 23

### Mischung von fluorierten Terrylendiimiden

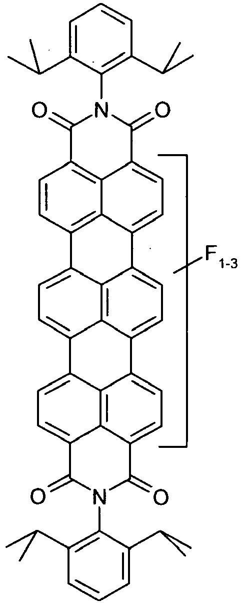

Eine Mischung aus 250 ml Sulfolan, 5,8 g (5 mmol) 1,6,9,14-Tetrabromterrylen-3,4-11,12-tetracarbonsäurediimid, 0,26 g (1 mmol) 18-Krone-6 und 2,32 g (40 mmol) Kaliumfluorid wird auf 120 °C erwärmt und mit weiteren 200 ml Sulfolan verdünnt. Nach dreistündigem Rühren bei 120°C wird auf 160 °C erwärmt und danach auf Raumtemperatur abgekühlt. Es werden weitere 1,16 g (20 mmol) Kaliumfluorid zugesetzt und die Mischung wird weitere 22 Stunden auf 160°C erhitzt. Nach Abkühlen auf Raumtemperatur werden 300 ml Wasser zugesetzt und die Reaktionsmischung weitere 16 Stunden gerührt. Der Niederschlag wird abfiltriert, mehrfach mit Wasser gewaschen und im Vakuum getrocknet. Man erhält in quantitativer Ausbeute ein blau-schwarzes Produkt. Gemäß massenspektroskopischer Untersuchung (MALDI) enthält das Produkt ein Gemisch der Titelverbindungen.

### Beispiel 24 (nicht erfindungsgemäß)

### 24.1

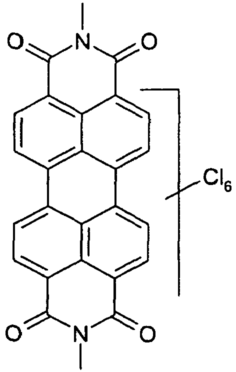

In Anlehnung an Sadrai Acta. Cryst. 1990, 46, 637-640 werden 20,9 g (0,05 mol) N,N'-Dimethylperylimid in 115 ml Chlorsulfonsäure bei 60 °C unter Zusatz von 1,0 g Iod innerhalb von drei Stunden mit 53.0 g Chlor behandelt. Das Reaktionsgemisch wird auf Eiswasser vorsichtig gefällt, abfiltriert und mit Wasser gewaschen. Man erhielt 29,2 g eines orangefarbenen Feststoffes, welcher gemäß massenspektroskopischer Untersuchung (MALDI) überwiegend aus der Hexachlorverbindung besteht.
Chlor-Gehalt: 34,7 %

### 24.2 Mischung aus

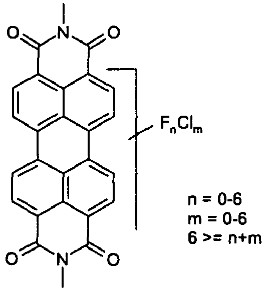

Eine Mischung aus 25 ml N-Methylpyrolidon, 2,3 g Kaliumfluorid (40 mmol), 0,3 g (11 mmol)18-Krone-6 und 1,2 g (2 mmol) des Gemischs aus 24.1 werden zwei Stunden auf 120 °C erwärmt. Das Reaktionsgemisch wird abgekühlt, auf Wasser gefällt, filtriert und gewaschen. Man erhält 1,06 g eines orangefarbenen Feststoffes, welcher säulenchromatographisch aufgetrennt wird. Es entsteht ein Gemisch aus teilchlorierten und teilfluorierten Verbindungen mit maximal 6 Halogenatomen und einer hexafluorierten Verbindung.

### Anwendungsbeispiele:

### Anwendungsbeispiel 1: Excitonische Solarzelle

Als Arbeitselektrode wurden mit Indium-dotiertem Zinnoxid (ITO) beschichtete Glasplatten der Abmessung 25 mm x 15 mm x 1 mm verwendet (Indium tin oxide coated glass slide, 30-60 ohm resistance, Sigma-Aldrich). Diese wurden nacheinander mit Glasreiniger, Aceton und Isopropanol im Ultraschallbad gereinigt und im Stickstoff-strom getrocknet. Anschließend wurde das ITO-Glas mit einer etwa 100 nm dicken PEDOT:PSS-Schicht durch Spin-Coating belegt. Dabei wurde PEDOT:PSS in wässriger Lösung verwendet (Baytron^{®}P VP Al 4083), die Spincoatingfrequenz auf 4000rpm und die Schleuderzeit auf 30s gesetzt. Danach wurde die Probe für 15 min bei 100 °C im Trockenschrank getrocknet.

Zur elektrischen Abisolation der Metallrückelektroden von der Arbeitselektrode wurden auf die PEDOT:PSS-Schicht an den Rändern längsseitig jeweils ein Streifen Polyimid (Pyrolin Polyimide Coating, Supelco) aufgetragen und 15 min bei 200 °C im Trockenschrank ausgehärtet.

Die aktiven organischen Schichten wurden in der folgenden Reihenfolge aufgedampft. Zuerst wurde als Donor Kupferphthalocyanin (CuPc, einfach gradientensublimiert), dann als Akzeptor ein Gemisch aus N,N'-Bis(heptafluorbutyl)-1,7-difluor-3,4:9,10-tetracarbonsäurebisimid und N,N'-Bis(heptafluorbutyl)-1,6-difluor-3,4:9,10-tetracarbonsäurebisimid aus Beispiel 10 (das Material wurde durch Säulenchromatografie gereinigt wie beschrieben) und schließlich als Pufferschicht Bathocuproin (BCP, Sensient) mit einer thermischen Verdampfung im Vakuum auf die PEDOT:PSS/Polyimid-Schicht aufgebracht. Es wurde bei einem Druck von 8*10⁻⁷ mbar gearbeitet. Die Verdampfung des CuPc fand bei einer Temperatur von 380 °C und einer Aufdampfrate von 0,2 bis 1,0 nm/s statt, das Gemisch aus N,N'-Bis(heptafluorbutyl)-1,7-difluor-3,4:9,10-tetracarbonsäurebisimid und N,N'-Bis(heptafluorbutyl)-1,6-difluor-3,4:9,10-tetracarbon-säurebisimid wurde bei 290 °C mit einer Rate von 0,2 bis 0,8 nm/s und BCP bei 180 °C mit 1,0 nm/s aufgedampft. Die entstandenen Schichtdicken betrugen 50 nm für die CuPc, 50 nm für das Gemisch aus N,N'-Bis(heptafluorbutyl)-1,7-difluor-3,4:9,10-tetracarbonsäurebisimid und N,N'-Bis(heptafluorbutyl)-1,6-difluor-3,4:9,10-tetra-carbonsäurebisimid und 20 nm für die BCP-Schicht.

Die Metallrückelektrode wurde durch thermische Metallverdampfung im Vakuum aufgebracht. Dazu wurde die Probe mit einer Maske versehen, um 8 voneinander getrennte runde Rückelektroden mit einem Durchmesser von 1 mm auf die aktive Region zu bedampfen, die jeweils mit einer etwa 3 mm x 2 mm großen Kontaktfläche über der Polyimidschicht verbunden sind. Als Metall wurde Ag verwendet, das mit einer Rate von 0.2 bis 1,0 nm/s bei einem Druck von ca. 5*10⁻⁵ mbar verdampft wurde, so dass eine Schichtdicke von 100 nm entstand.

Zur Bestimmung des Wirkungsgrads η wurde die jeweilige Strom/Spannungs-Kennlinie mit einem Source Meter Model 2400 (Keithley Instruments Inc.) unter Bestrahlung mit einem Halogen-Lampenfeld (Xenophot^{®} 64629; Osram) als Sonnensimulator gemessen.

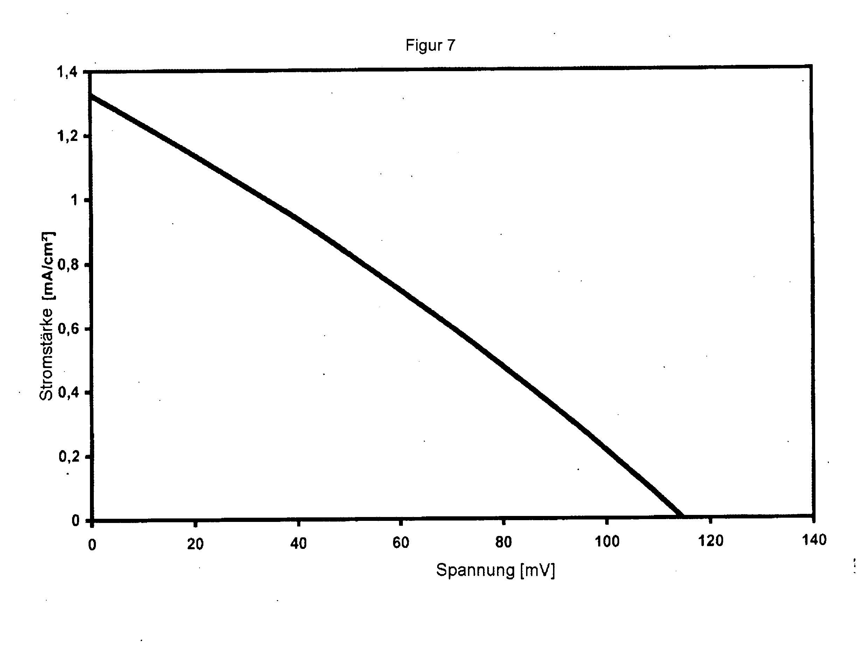

Die Strom/Spannungs-Kennlinie bei einer Beleuchtungsstärke von 100 mW/cm² ist in Figur 7 dargestellt. Der Kurzschluss-Strom betrug 1,37 mA/cm², die offene-KlemmenSpannung 114 mV und der Füllfaktor 29%. Dies ergibt einen Wirkungsgrad von 0,04 %, was eine photovoltaische Aktivität der Substanz belegt.

## Patentansprüche

1. Verwendung von Verbindungen der allgemeinen Formel I 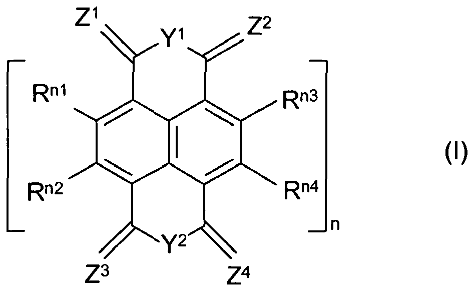 oder von Verbindungen der Formeln I.Bb 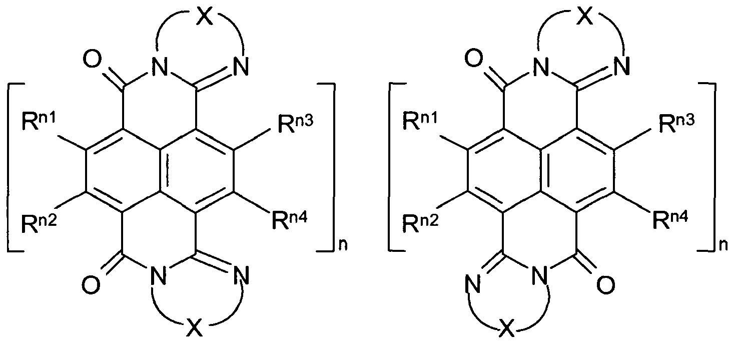 wobei
n in den Verbindungen der allgemeinen Formel I für 3 oder 4 steht und in den
Verbindungen der allgemeinen Formeln I.Bb für 2, 3 oder 4 steht, wenigstens einer der Reste Rⁿ¹, Rⁿ², Rⁿ³ und Rⁿ⁴ für Fluor steht,
gegebenenfalls wenigstens ein weiterer Rest Rⁿ¹, Rⁿ², Rⁿ³ und Rⁿ⁴ für einen Substituenten steht, der ausgewählt ist unter Cl und Br, und die übrigen Reste für Wasserstoff stehen,
X für eine zweiwertige verbrückende Gruppe mit 2 bis 5 Atomen zwischen
den flankierenden Bindungen steht,
Y¹ für O oder NR^{a} steht, wobei R^{a} für Wasserstoff oder einen Organylrest
steht,
Y² für O oder NR^{b} steht, wobei R^{b} für Wasserstoff oder einen Organylrest
steht,
Z¹, Z², Z³ und Z⁴ für O stehen,
als Halbleiter, insbesondere als n-Halbleiter.

2. Verwendung nach Anspruch 1 in der organischen Elektronik, insbesondere in organischen Feldeffekttransistoren, organischen Leuchtdioden oder in Solarzellen.

3. Verwendung nach Anspruch 1 in der organischen Photovoltaik, insbesondere in excitonischen Solarzellen.

4. Verwendung einer Verbindung der allgemeinen Formel I, wie in Anspruch 1 definiert, für optische Label, zur unsichtbaren Markierung von Produkten, als Fluoreszenzfarbstoffe, als Fluoreszenzlabel für Biomoleküle und als Pigmente.

5. Verwendung einer Verbindung der allgemeinen Formel I, wie in Anspruch 1 definiert, als Fluoreszenzfarbstoff in einem auf Fluoreszenzkonversion beruhenden Display; in einem lichtsammelnden Kunststoffteil, welches gegebenenfalls mit einer Solarzelle kombiniert ist; als Pigmentfarbstoff in elektrophoretischen Displays; als Fluoreszenzfarbstoff in einer auf Chemolumineszenz basierenden Anwendung.

6. Verfahren zur Herstellung von Verbindungen der Formel I 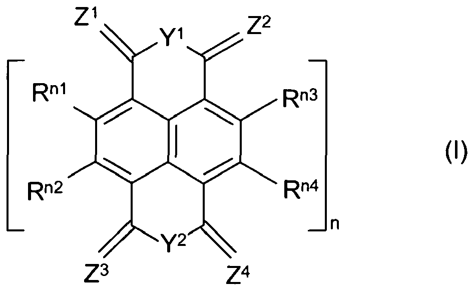 wobei n, Rⁿ¹, Rⁿ², Rⁿ³ und Rⁿ⁴, Y¹, Y², Z¹, Z², Z³ und Z⁴ wie in Anspruch 1 definiert sind, bei dem man eine Verbindung der Formel I, bei der wenigstens einer der Reste Rⁿ¹, Rⁿ², Rⁿ³ und Rⁿ⁴, R¹, R², R³ und R⁴ für Cl oder Br steht einer Umsetzung mit einem Alkalifluorid unter wasserfreien Bedingungen unterzieht.

7. Verfahren nach Anspruch 6, wobei zur Umsetzung mit dem Alkalifluorid zusätzlich ein Komplexbildner, vorzugsweise ein Kronenether, eingesetzt wird.

8. Verfahren nach einem der Ansprüche 6 oder 7, wobei zur Umsetzung mit dem Alkalifluorid zusätzlich ein Phasentransferkatalysator eingesetzt wird, der ausgewählt ist unter 2-Azaalleniumverbindungen, Carbophosphazeniumverbindungen, Aminophosphoniumverbindungen und Diphosphazeniumverbindungen.

9. Verfahren nach Anspruch 8, wobei als Phasentransferkatalysator (N,N-Dimethylimidazolidino)-tetramethylguanidiniumchlorid eingesetzt wird.

10. Verfahren zur Herstellung von Verbindungen der Formel I.Ba 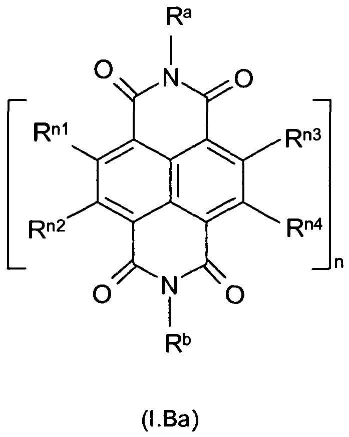 wobei n, Rⁿ¹, Rⁿ², Rⁿ³ und Rⁿ⁴ wie in Anspruch 1 definiert sind, und
R^{a} und R^{b} unabhängig voneinander für Wasserstoff oder für unsubstituiertes oder
substituiertes Alkyl, Alkenyl, Alkinyl, Cycloalkyl, Bicycloalkyl, Cycloalkenyl, Heterocycloalkyl, Aryl oder Heteroaryl stehen,
worin
der Ausdruck Alkyl auch Alkylreste umfasst, deren Kohlenstoffketten durch eine
oder mehrere nicht benachbarte Gruppen, die ausgewählt sind unter -O-, -S-, -NR^{e}-, -CO-, -SO- und/oder -SO²- unterbrochen sein kann, worin Re für Wasserstoff, Alkyl, Cycloalkyl, Heterocycloalkyl, Aryl oder Hetaryl steht,
substituierte Alkylgruppen in Abhängigkeit von der Länge der Alkylkette einen
oder mehrere Substituenten aufweisen, die unabhängig voneinander ausgewählt sind unter Cycloalkyl, Heterocycloalkyl, Aryl, Hetaryl, Halogen, Hydroxy, Thiol, COOH, Carboxylat, SO₃H, Sulfonat, NE¹E², Nitro und Cyano, wobei E¹ und E² unabhängig voneinander für Wasserstoff, Alkyl, Cycloalkyl, Heterocycloalkyl, Aryl oder Hetaryl stehen,
der Ausdruck "Alkenyl" geradkettige und verzweigte Alkenylgruppen umfasst, die
in Abhängigkeit von der Kettenlänge eine oder mehrere Doppelbindungen tragen können,
substituierte Alkenylgruppen einen oder mehrere Substituenten tragen können,
die unabhängig voneinander ausgewählt sind unter Cycloalkyl, Heterocycloalkyl, Aryl, Hetaryl, Halogen, Hydroxy, Thiol, COOH, Carboxylat, SO₃H, Sulfonat, NE³E⁴, Nitro und Cyano, wobei E³ und E⁴ unabhängig voneinander für Wasserstoff, Alkyl, Cycloalkyl, Heterocycloalkyl, Aryl oder Hetaryl stehen,
substituierte Alkinyle einen oder mehrere der zuvor für Alkyl genannten Substituenten tragen,
substituierte Cycloalkylgruppen einen oder mehrere Substituenten aufweisen, die
unabhängig voneinander ausgewählt sind unter Alkyl sowie den zuvor für die Alkylgruppen genannten Substituenten,
der Ausdruck Bicycloalkyl bicyclische Kohlenwasserstoffreste mit 5 bis 10 C-Atomen umfaßt,
der Ausdruck Heterocycloalkyl nicht-aromatische, ungesättigte oder vollständig
gesättigte, cycloaliphatische Gruppen mit 5 bis 8 Ringatomen umfaßt, in denen 1, 2 oder 3 der Ringkohlenstoffatome durch Heteroatome, ausgewählt unter Sauerstoff, Stickstoff, Schwefel und einer Gruppe -NRe- ersetzt sind, und das unsubstituiert ist oder mit einer oder mehreren C₁-C₆-Alkylgruppen substituiert ist,
substituierte Aryle in Abhängigkeit von der Anzahl und Größe ihrer Ringsysteme
einen oder mehrere Substituenten aufweisen, die unabhängig voneinander ausgewählt sind unter Alkyl, Alkoxy, Cycloalkyl, Heterocycloalkyl, Aryl, Hetaryl, Halogen, Hydroxy, Thiol, COOH, Carboxylat, SO₃H, Sulfonat, NE⁵E⁶, Nitro und Cyano, wobei E⁵ und E⁶ unabhängig voneinander für Wasserstoff, Alkyl, Cycloalkyl, Heterocycloalkyl, Aryl oder Hetaryl stehen,
die Substituenten von Heteroaryl ausgewählt sind unter C₁-C₆-Alkyl, C₁-C₆-Alkoxy, Hydroxy, Carboxy, Halogen und Cyano,
bei dem man
a1) ein Rylendianhydrid der Formel II, 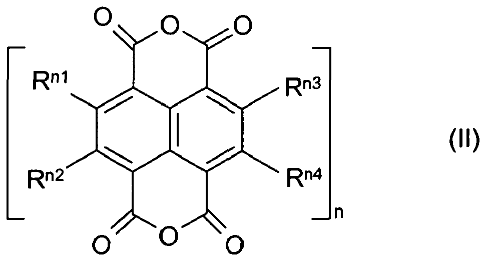 wobei wenigstens einer der Reste Rⁿ¹, Rⁿ², Rⁿ³ und Rⁿ⁴ für Cl oder Br steht und n für 3 oder 4 steht, einer Umsetzung mit einem Alkalifluorid unter im Wesentlichen wasserfreien Bedingungen unterzieht,
b1) die in Schritt a1) erhaltene Verbindung einer Umsetzung mit einem Amin
der Formel R^{a}-NH₂ und gegebenenfalls einem davon verschiedenen Amin der Formel R^{b}-NH₂ unterzieht.

11. Verfahren zur Herstellung von Verbindungen der Formeln I.Bb 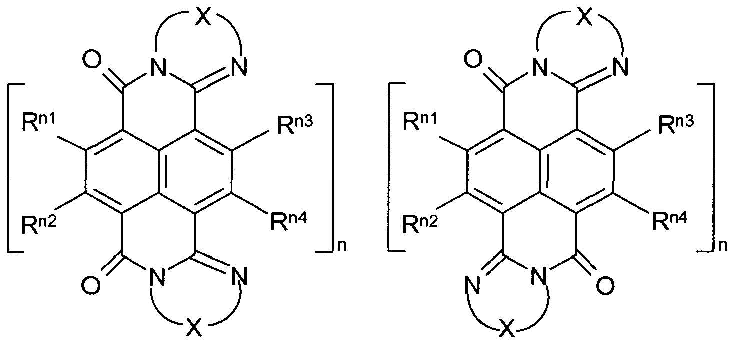 wobei n, X, Rⁿ¹, Rⁿ², Rⁿ³ und Rⁿ⁴ wie in Anspruch 1 definiert sind, bei dem man
a2) ein Rylendianhydrid der Formel II, 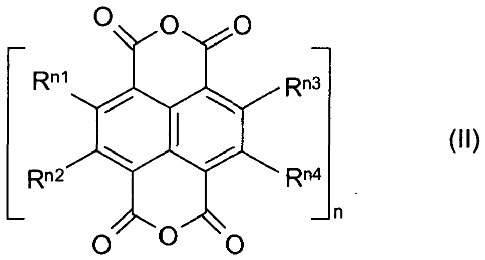 wobei wenigstens einer der Reste Rⁿ¹, Rⁿ², Rⁿ³ und Rⁿ⁴ für Cl oder Br steht und n für 2, 3 oder 4 steht, einer Umsetzung mit einem Alkalifluorid unter im Wesentlichen wasserfreien Bedingungen unterzieht,
b2) die in Schritt a2) erhaltene Verbindung einer Umsetzung mit einem Amin
der Formel H₂N-X-NH₂ unterzieht.

12. Verfahren zur Herstellung von Dianhydriden der Formel (I.A) 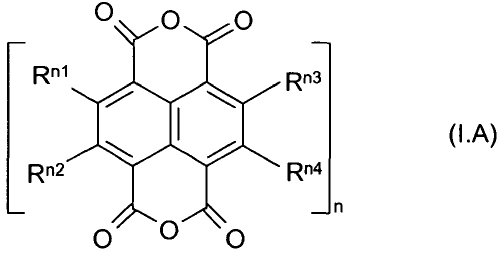 wobei
n für 3 oder 4 steht,
wenigstens einer der Reste Rⁿ¹, Rⁿ², Rⁿ³ und Rⁿ⁴ für Fluor steht, die übrigen Reste Rⁿ¹, Rⁿ², Rⁿ³ und Rⁿ⁴ für Wasserstoff stehen,
bei dem man ein Diimid der Formel (I.Ba), 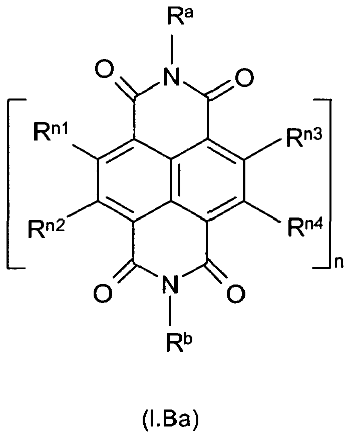 wobei R^{a} und R^{b} unabhängig voneinander für Wasserstoff oder für unsubstituiertes oder substituiertes Alkyl, Alkenyl, Alkinyl, Cycloalkyl, Bicycloalkyl, Cycloalkenyl, Heterocycloalkyl, Aryl oder Heteroaryl stehen,
worin
der Ausdruck Alkyl auch Alkylreste umfasst, deren Kohlenstoffketten durch
eine oder mehrere nicht benachbarte Gruppen, die ausgewählt sind unter -O-, -S-, -NR^{e}-, -CO-, -SO- und/oder -SO₂- unterbrochen sein kann, worin Re für Wasserstoff, Alkyl, Cycloalkyl, Heterocycloalkyl, Aryl oder Hetaryl steht,
substituierte Alkylgruppen in Abhängigkeit von der Länge der Alkylkette einen oder mehrere Substituenten aufweisen, die unabhängig voneinander ausgewählt sind unter Cycloalkyl, Heterocycloalkyl, Aryl, Hetaryl, Halogen, Hydroxy, Thiol, COOH, Carboxylat, SO₃H, Sulfonat, NE¹E², Nitro und Cyano, wobei E¹ und E² unabhängig voneinander für Wasserstoff, Alkyl, Cycloalkyl, Heterocycloalkyl, Aryl oder Hetaryl stehen,
der Ausdruck "Alkenyl" geradkettige und verzweigte Alkenylgruppen umfasst, die in Abhängigkeit von der Kettenlänge eine oder mehrere Doppelbindungen tragen können,
substituierte Alkenylgruppen einen oder mehrere Substituenten tragen können, die unabhängig voneinander ausgewählt sind unter Cycloalkyl, Heterocycloalkyl, Aryl, Hetaryl, Halogen, Hydroxy, Thiol, COOH, Carboxylat, SO₃H, Sulfonat, NE³E⁴, Nitro und Cyano, wobei E³ und E⁴ unabhängig voneinander für Wasserstoff, Alkyl, Cycloalkyl, Heterocycloalkyl, Aryl oder Hetaryl stehen,
substituierte Alkinyle einen oder mehrere der zuvor für Alkyl genannten
Substituenten tragen,
substituierte Cycloalkylgruppen einen oder mehrere Substituenten aufweisen, die unabhängig voneinander ausgewählt sind unter Alkyl sowie den zuvor für die Alkylgruppen genannten Substituenten,
der Ausdruck Bicycloalkyl bicyclische Kohlenwasserstoffreste mit 5 bis 10 C-Atomen umfaßt,
der Ausdruck Heterocycloalkyl nicht-aromatische, ungesättigte oder vollständig gesättigte, cycloaliphatische Gruppen mit 5 bis 8 Ringatomen umfaßt, in denen 1, 2 oder 3 der Ringkohlenstoffatome durch Heteroatome, ausgewählt unter Sauerstoff, Stickstoff, Schwefel und einer Gruppe -NR^{e}- ersetzt sind, und das unsubstituiert ist oder mit einer oder mehreren C₁-C₆-Alkylgruppen substituiert ist,
substituierte Aryle in Abhängigkeit von der Anzahl und Größe ihrer Ringsysteme einen oder mehrere Substituenten aufweisen, die unabhängig voneinander ausgewählt sind unter Alkyl, Alkoxy, Cycloalkyl, Heterocycloalkyl, Aryl, Hetaryl, Halogen, Hydroxy, Thiol, COOH, Carboxylat, SO₃H, Sulfonat, NE⁵E⁶, Nitro und Cyano, wobei E⁵ und E⁶ unabhängig voneinander für Wasserstoff, Alkyl, Cycloalkyl, Heterocycloalkyl, Aryl oder Hetaryl stehen,
die Substituenten von Heteroaryl ausgewählt sind unter C₁-C₆-Alkyl, C₁-C₆-Alkoxy, Hydroxy, Carboxy, Halogen und Cyano,
einer zweistufigen Hydrolyse unterzieht, wobei die erste Stufe in Gegenwart einer Base und die zweite in Gegenwart einer Säure erfolgt.

13. Verfahren zur Herstellung von Verbindungen der Formel (I.Ba) 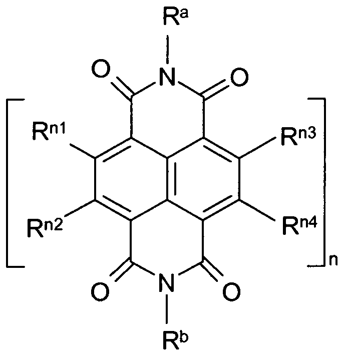 worin
n für 3 oder 4 steht,
wenigstens einer der Reste Rⁿ¹, Rⁿ², Rⁿ³ und Rⁿ⁴ für Fluor steht, die übrigen Reste Rⁿ¹, Rⁿ², Rⁿ³ und Rⁿ⁴ für Wasserstoff stehen, und
R^{a} und R^{b} unabhängig voneinander für Wasserstoff oder für unsubstituiertes oder substituiertes Alkyl, Alkenyl, Alkinyl, Cycloalkyl, Bicycloalkyl, Cycloalkenyl, Heterocycloalkyl, Aryl oder Heteroaryl stehen,
worin
der Ausdruck Alkyl auch Alkylreste umfasst, deren Kohlenstoffketten durch
eine oder mehrere nicht benachbarte Gruppen, die ausgewählt sind unter -O-, -S-, -NR^{e}-, -CO-, -SO- und/oder -SO₂- unterbrochen sein kann, worin Re für Wasserstoff, Alkyl, Cycloalkyl, Heterocycloalkyl, Aryl oder Hetaryl steht,
substituierte Alkylgruppen in Abhängigkeit von der Länge der Alkylkette einen oder mehrere Substituenten aufweisen, die unabhängig voneinander ausgewählt sind unter Cycloalkyl, Heterocycloalkyl, Aryl, Hetaryl, Halogen, Hydroxy, Thiol, COOH, Carboxylat, SO₃H, Sulfonat, NE¹E², Nitro und Cyano, wobei E¹ und E² unabhängig voneinander für Wasserstoff, Alkyl, Cycloalkyl, Heterocycloalkyl, Aryl oder Hetaryl stehen,
der Ausdruck "Alkenyl" geradkettige und verzweigte Alkenylgruppen umfasst, die in Abhängigkeit von der Kettenlänge eine oder mehrere Doppelbindungen tragen können,
substituierte Alkenylgruppen einen oder mehrere Substituenten tragen können, die unabhängig voneinander ausgewählt sind unter Cycloalkyl, Heterocycloalkyl, Aryl, Hetaryl, Halogen, Hydroxy, Thiol, COOH, Carboxylat, SO₃H, Sulfonat, NE³E⁴, Nitro und Cyano, wobei E³ und E⁴ unabhängig voneinander für Wasserstoff, Alkyl, Cycloalkyl, Heterocycloalkyl, Aryl oder Hetaryl stehen,
substituierte Alkinyle einen oder mehrere der zuvor für Alkyl genannten
Substituenten tragen,
substituierte Cycloalkylgruppen einen oder mehrere Substituenten aufweisen, die unabhängig voneinander ausgewählt sind unter Alkyl sowie den zuvor für die Alkylgruppen genannten Substituenten,
der Ausdruck Bicycloalkyl bicyclische Kohlenwasserstoffreste mit 5 bis 10 C-Atomen umfaßt,
der Ausdruck Heterocycloalkyl nicht-aromatische, ungesättigte oder vollständig gesättigte, cycloaliphatische Gruppen mit 5 bis 8 Ringatomen umfaßt, in denen 1, 2 oder 3 der Ringkohlenstoffatome durch Heteroatome, ausgewählt unter Sauerstoff, Stickstoff, Schwefel und einer Gruppe -NRe- ersetzt sind, und das unsubstituiert ist oder mit einer oder mehreren C₁-C₆-Alkylgruppen substituiert ist,
substituierte Aryle in Abhängigkeit von der Anzahl und Größe ihrer Ringsysteme einen oder mehrere Substituenten aufweisen, die unabhängig voneinander ausgewählt sind unter Alkyl, Alkoxy, Cycloalkyl, Heterocycloalkyl, Aryl, Hetaryl, Halogen, Hydroxy, Thiol, COOH, Carboxylat, SO₃H, Sulfonat, NE⁵E⁶, Nitro und Cyano, wobei E⁵ und E⁶ unabhängig voneinander für Wasserstoff, Alkyl, Cycloalkyl, Heterocycloalkyl, Aryl oder Hetaryl stehen,
die Substituenten von Heteroaryl ausgewählt sind unter C₁-C₆-Alkyl, C₁-C₆-Alkoxy, Hydroxy, Carboxy, Halogen und Cyano,
bei dem man ein Dianhydrid der Formel (I.A) 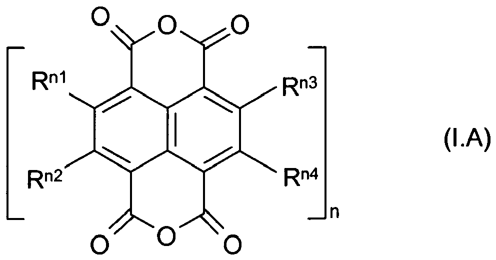 wobei
n für 3 oder 4 steht,
wenigstens einer der Reste Rⁿ¹, Rⁿ², Rⁿ³ und Rⁿ⁴ für Fluor steht, die übrigen Reste Rⁿ¹, Rⁿ², Rn³ und Rⁿ⁴ für Wasserstoff stehen, einer Umsetzung mit einem Amin der Formel R^{a}-NH₂ und gegebenenfalls einem davon verschiedenen Amin der Formel R^{b}-NH₂ unterzieht.

14. Verfahren zur Herstellung wenigstens einer Verbindung der Formeln I.Bb 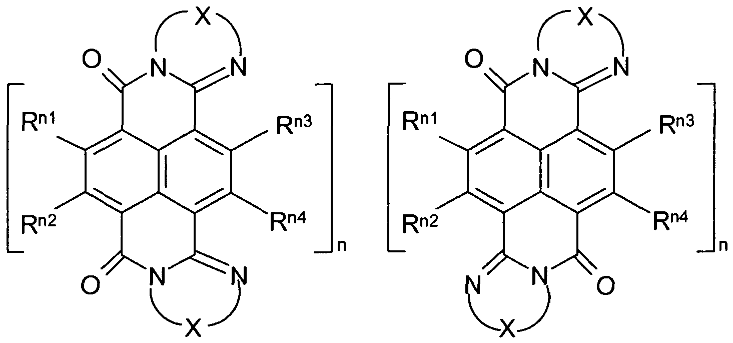 worin
n für 2, 3 oder 4 steht,
wenigstens einer der Reste Rⁿ¹, Rⁿ², Rⁿ³ und Rⁿ⁴ für Fluor steht,
die übrigen Reste Rⁿ¹, Rⁿ², Rⁿ³ und Rⁿ⁴ für Wasserstoff stehen, und X für eine zweiwertige verbrückende Gruppe mit 2 bis 5 Atomen zwischen
den flankierenden Bindungen steht,
bei dem man ein Dianhydrid der Formel (I.A) 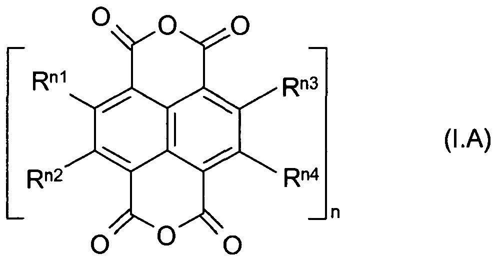 wobei
n für 3 oder 4 steht,
wenigstens einer der Reste Rⁿ¹, Rⁿ², Rⁿ³ und Rⁿ⁴ für Fluor steht, die übrigen Reste Rⁿ¹, Rⁿ², Rⁿ³ und Rⁿ⁴ für Wasserstoff stehen, einer Umsetzung mit einem Amin der Formel H₂N-X-NH₂ unterzieht.

15. Verfahren zur Herstellung von Verbindungen der Formel (I.BaH) 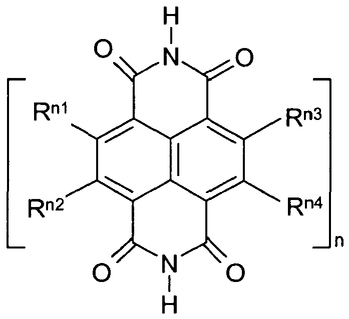 worin
n für 3 oder 4 steht,
wenigstens einer der Reste Rⁿ¹, Rⁿ², Rⁿ³ und Rⁿ⁴ für Fluor steht, und die übrigen Reste Rⁿ¹, Rⁿ², Rⁿ³ und Rⁿ⁴ für Wasserstoff stehen, bei dem man ein Diimid der Formel (I.Ba2), 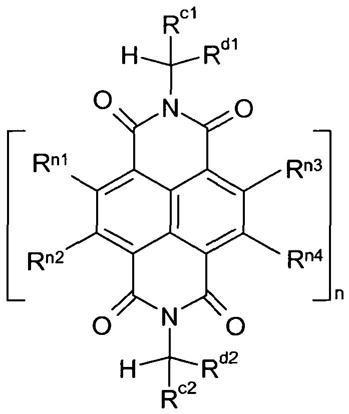 worin
n für 2, 3 oder 4 steht,
wenigstens einer der Reste Rⁿ¹, Rⁿ², Rⁿ³ und Rⁿ⁴ für Fluor steht,
die übrigen Reste Rⁿ¹, Rⁿ², Rⁿ³ und Rⁿ⁴ für Wasserstoff stehen,
R^{c1} und R^{c2} unabhängig voneinander für Aryl stehen, und
R^{d1} und R^{d2} unabhängig voneinander für Alkyl stehen,
einer Umsetzung mit einer starken Lewis-Säure und einem Protonendonor unterzieht.

16. Verbindungen der allgemeinen Formel I 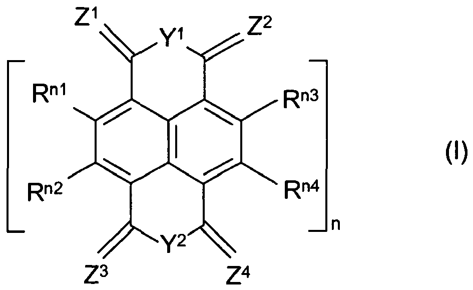 oder von Verbindungen der Formeln I.Bb 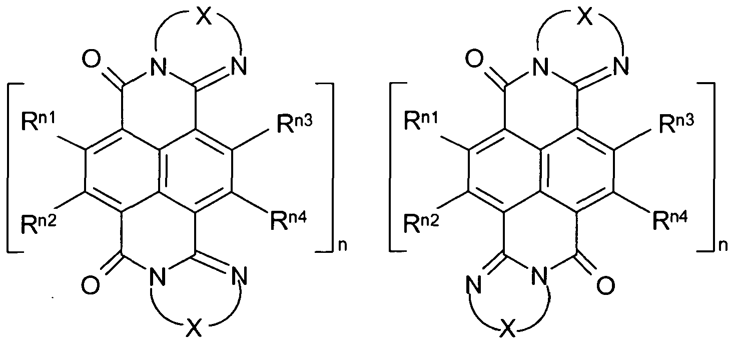 wobei
n in den Verbindungen der allgemeinen Formel I für 3, 4, 5 oder 6 steht und
in den Verbindungen der allgemeinen Formeln I.Bb für 2, 3 oder 4 steht, wenigstens einer der Reste Rⁿ¹, Rⁿ², Rⁿ³ und Rⁿ⁴ für Fluor steht, gegebenenfalls wenigstens ein weiterer Rest Rⁿ¹, Rⁿ², Rⁿ³ und Rⁿ⁴ für einen Substituenten steht, der ausgewählt ist unter Cl und Br, und die übrigen Reste für Wasserstoff stehen,
X für eine zweiwertige verbrückende Gruppe mit 2 bis 5 Atomen zwischen
den flankierenden Bindungen steht,
Y¹ für O oder NR^{a} steht, wobei R^{a} für Wasserstoff oder einen Organylrest
steht,
Y² für O oder NR^{b} steht, wobei R^{b} für Wasserstoff oder einen Organylrest
steht,
Z¹, Z², Z³ und Z⁴ für O stehen.

17. Organischer Feldeffekttransistor, umfassend ein Substrat mit wenigstens einer Gate-Struktur, einer Source-Elektrode und einer Drain-Elektrode und wenigstens einer Verbindung der Formel I, wie in Anspruch 1 definiert, als n-Halbleiter.

18. Substrat mit einer Vielzahl von organischen Feldeffekttransistoren, wobei zumindest ein Teil der Feldeffekttransistoren wenigstens einer Verbindung der Formel I, wie in Anspruch 1 definiert, als n-Halbleiter enthält.

19. Halbleiterbaustein, umfassend wenigstes ein Substrat, wie in Anspruch 18 definiert.

## Claims

1. The use of compounds of the general formula I 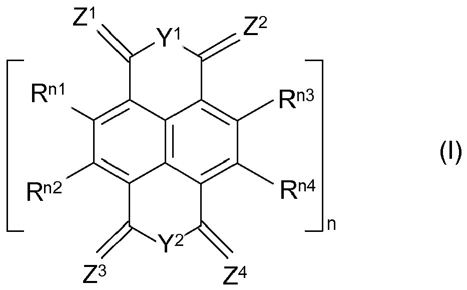 or of compounds of the formulae I.Bb 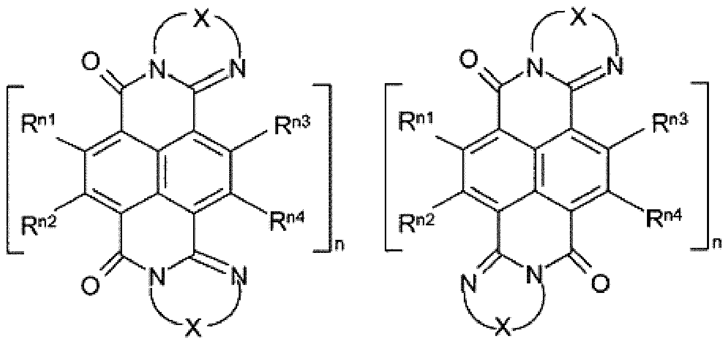 where n in the compounds of the general formula I is 3 or 4 and in the compounds
of the general formulae I.Bb is 2, 3 or 4,
at least one of the Rⁿ¹, Rⁿ², Rⁿ³ and Rⁿ⁴ radicals is fluorine,
optionally at least one further Rⁿ¹, Rⁿ², Rⁿ³ and Rⁿ⁴ radical is a substituent which is selected from Cl and Br, and the remaining radicals are each hydrogen,
X is a divalent bridging group having from 2 to 5 atoms between the flanking
bonds,
Y¹ is O or NR^{a} where R^{a} is hydrogen or an organyl radical,
Y² is O or NR^{b} where R^{b} is hydrogen or an organyl radical, Z¹, Z², Z³ and Z⁴ are each O,
as semiconductors, especially as n-semiconductors.

2. The use according to claim 1 in organic electronics, especially in organic field-effect transistors, organic light-emitting diodes or in solar cells.

3. The use according to claim 1 in organic photovoltaics, especially in excitonic solar cells.

4. The use of a compound of the general formula I as defined in claim 1 for optical labels, for invisible marking of products, as fluorescent dyes, as fluorescent labels for biomolecules and as pigments.

5. The use of a compound of the general formula I as defined in claim 1 as a fluorescent dye in a display based on fluorescence conversion; in a light-collecting plastics part optionally combined with a solar cell; as a pigment dye in electrophoretic displays; as a fluorescent dye in an application based on chemiluminescence.

6. A process for preparing compounds of the formula I 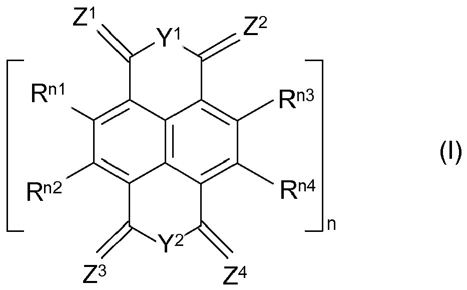 where n, Rⁿ¹, Rⁿ², Rⁿ³ and Rⁿ⁴, Y¹, Y², Z¹, Z², Z³ and Z⁴ are each as defined in claim 1, in which a compound of the formula I in which at least one of the Rⁿ¹, Rⁿ², Rⁿ³ and Rⁿ⁴, R¹, R², R³ and R⁴ radicals is Cl or Br is subjected to a reaction with an alkali metal fluoride under anhydrous conditions.

7. The process according to claim 6, wherein a complexing agent, preferably a crown ether, is additionally used for the reaction with the alkali metal fluoride.

8. The process according to either of claims 6 and 7, wherein reaction with the alkali metal fluoride is effected additionally using a phase transfer catalyst selected from 2-azaallenium compounds, carbophosphazenium compounds, aminophosphonium compounds and diphosphazenium compounds.

9. The process according to claim 8, wherein the phase transfer catalyst used is (N,N-dimethylimidazolidino)tetramethylguanidinium chloride.

10. A process for preparing compounds of the formula I.Ba 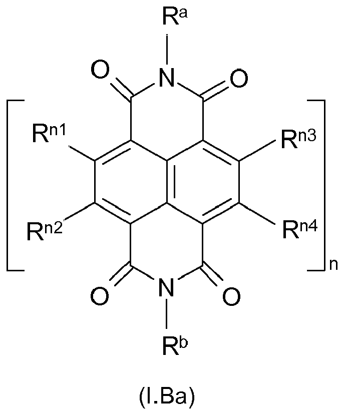 where n, Rⁿ¹, Rⁿ², Rⁿ³ and Rⁿ⁴ are each as defined in claim 1, and
R^{a} and R^{b} are each independently hydrogen or unsubstituted or substituted alkyl,
alkenyl, alkynyl, cycloalkyl, bicycloalkyl, cycloalkenyl, heterocycloalkyl, aryl or heteroaryl,
in which
the expression "alkyl" also comprises alkyl radicals whose carbon chains may be
interrupted by one or more nonadjacent groups selected from -O-, -S-, -NR^{e}-, -CO-, -SO- and/or -SO₂-, in which R^{e} is hydrogen, alkyl, cycloalkyl,
heterocycloalkyl, aryl or hetaryl,
substituted alkyl groups, depending on the length of the alkyl chain, have one or
more substituents independently selected from cycloalkyl, heterocycloalkyl, aryl, hetaryl, halogen, hydroxyl, thiol, COOH, carboxylate, SO₃H, sulfonate, NE¹E², nitro and cyano, where E¹ and E² are each independently hydrogen, alkyl, cycloalkyl, heterocycloalkyl, aryl or hetaryl,
the expression "alkenyl" comprises straight-chain and branched alkenyl groups
which, depending on the chain length, may bear one or more double bonds,
substituted alkenyl groups may bear one or more substituents selected
independently from cycloalkyl, heterocycloalkyl, aryl, hetaryl, halogen, hydroxyl, thiol, COOH, carboxylate, SO₃H, sulfonate, NE³E⁴, nitro and cyano, where E³ and E⁴ are each independently hydrogen, alkyl, cycloalkyl, heterocycloalkyl, aryl or hetaryl,
substituted alkynyls bear one or more of the substituents mentioned above for
alkyl,
substituted cycloalkyl groups have one or more substituents independently
selected from alkyl and the substituents mentioned above for the alkyl groups,
the expression "bicycloalkyl" comprises bicyclic hydrocarbyl radicals having 5 to 10 carbon atoms,
the expression "heterocycloalkyl" comprises nonaromatic, unsaturated or fully
saturated cycloaliphatic groups having 5 to 8 ring atoms, in which 1, 2 or 3 of the ring carbon atoms are replaced by heteroatoms selected from oxygen, nitrogen, sulfur and an -NRe- group, and which is unsubstituted or substituted by one or more C₁-C₆-alkyl groups,
substituted aryls, depending on the number and size of the ring systems thereof,
have one or more substituents independently selected from alkyl, alkoxy, cycloalkyl, heterocycloalkyl, aryl, hetaryl, halogen, hydroxyl, thiol, COOH, carboxylate, SO₃H, sulfonate, NE⁵E⁶, nitro and cyano, where E⁵ and E⁶ are each independently hydrogen, alkyl, cycloalkyl, heterocycloalkyl, aryl or hetaryl,
the substituents of heteroaryl are selected from C₁-C₆-alkyl, C₁-C₆-alkoxy,
hydroxyl, carboxyl, halogen and cyano,
in which
a1) a rylene dianhydride of the formula II, 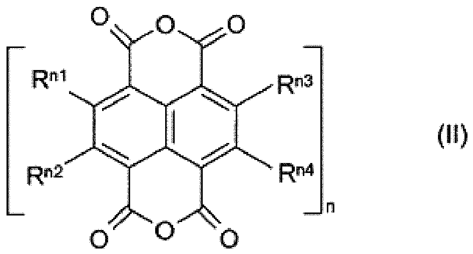 where at least one of the Rⁿ¹, Rⁿ², Rⁿ³ and Rⁿ⁴ radicals is Cl or Br and n is 3 or 4, is subjected to a reaction with an alkali metal fluoride under substantially anhydrous conditions,
b1) the compound obtained in step a1) is subjected to a reaction with an amine
of the formula R^{a}-NH₂ and optionally a different amine of the formula R^{b}-NH₂.

11. A process for preparing compounds of the formulae I.Bb 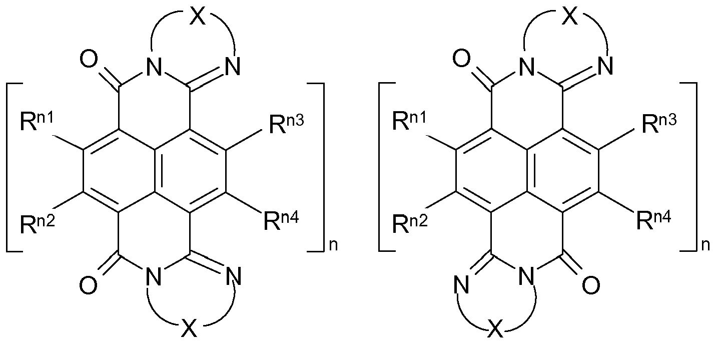 where n, X, Rⁿ¹, Rⁿ², Rⁿ³ and Rⁿ⁴ are each as defined in claim 1, in which
a2) a rylene dianhydride of the formula II, 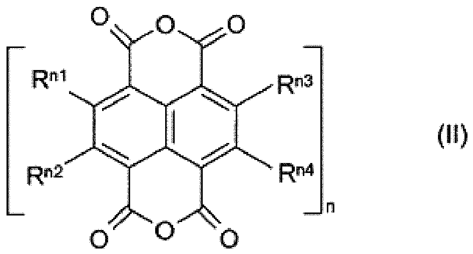 where at least one of the Rⁿ¹, Rⁿ², Rⁿ³ and Rⁿ⁴ radicals is Cl or Br and n is 2, 3 or 4, is subjected to a reaction with an alkali metal fluoride under substantially anhydrous conditions,
b2) the compound obtained in step a2) is subjected to a reaction with an amine
of the formula H₂N-X-NH₂.

12. A process for preparing dianhydrides of the formula (I.A) 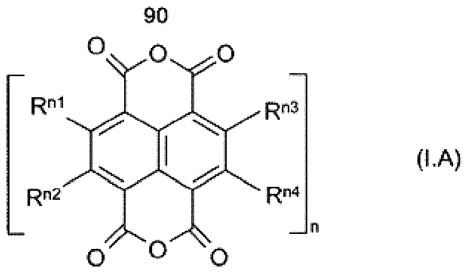 where
n is 3 or 4,
at least one of the Rⁿ¹, Rⁿ², Rⁿ³ and Rⁿ⁴ radicals is fluorine, the rest of the Rⁿ¹, Rⁿ², Rⁿ³ and Rⁿ⁴ radicals are hydrogen,
in which a diimide of the formula (I.Ba), 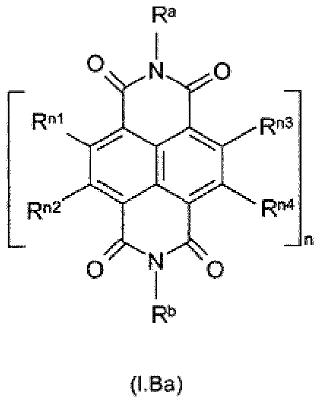 where R^{a} and R^{b} are each independently hydrogen or unsubstituted or
substituted alkyl, alkenyl, alkynyl, cycloalkyl, bicycloalkyl, cycloalkenyl heterocycloalkyl, aryl or heteroaryl,
in which
the expression "alkyl" also comprises alkyl radicals whose carbon chains may be
interrupted by one or more nonadjacent groups selected from -O-, -S-, -NR^{e}-, -CO-, -SO- and/or -SO₂-, in which R^{e} is hydrogen, alkyl, cycloalkyl,
heterocycloalkyl, aryl or hetaryl,
substituted alkyl groups, depending on the length of the alkyl chain, have one or
more substituents independently selected from cycloalkyl, heterocycloalkyl, aryl, hetaryl, halogen, hydroxyl, thiol, COOH, carboxylate, SO₃H, sulfonate, NE¹E², nitro and cyano, where E¹ and E² are each independently hydrogen, alkyl, cycloalkyl, heterocycloalkyl, aryl or hetaryl,
the expression "alkenyl" comprises straight-chain and branched alkenyl groups
which, depending on the chain length, may bear one or more double bonds,
substituted alkenyl groups may bear one or more substituents selected
independently from cycloalkyl, heterocycloalkyl, aryl, hetaryl, halogen, hydroxyl, thiol, COOH, carboxylate, SO₃H, sulfonate, NE³E⁴, nitro and cyano, where E³ and E⁴ are each independently hydrogen, alkyl, cycloalkyl, heterocycloalkyl, aryl or hetaryl,
substituted alkynyls bear one or more of the substituents mentioned above for
alkyl,
substituted cycloalkyl groups have one or more substituents independently
selected from alkyl and the substituents mentioned above for the alkyl groups,
the expression "bicycloalkyl" comprises bicyclic hydrocarbyl radicals having 5 to 10 carbon atoms,
the expression "heterocycloalkyl" comprises nonaromatic, unsaturated or fully
saturated cycloaliphatic groups having 5 to 8 ring atoms, in which 1, 2 or 3 of the ring carbon atoms are replaced by heteroatoms selected from oxygen, nitrogen, sulfur and an -NRe- group, and which is unsubstituted or substituted by one or more C₁-C₆-alkyl groups,
substituted aryls, depending on the number and size of the ring systems thereof,
have one or more substituents independently selected from alkyl, alkoxy, cycloalkyl, heterocycloalkyl, aryl, hetaryl, halogen, hydroxyl, thiol, COOH, carboxylate, SO₃H, sulfonate, NE⁵E⁶, nitro and cyano, where E⁵ and E⁶ are each independently hydrogen, alkyl, cycloalkyl, heterocycloalkyl, aryl or hetaryl,
the substituents of heteroaryl are selected from C₁-C₆-alkyl, C₁-C₆-alkoxy,
hydroxyl, carboxyl, halogen and cyano,
is subjected to a two-stage hydrolysis, the first stage being effected in the
presence of a base and the second in the presence of an acid.

13. A process for preparing compounds of the formula (I.Ba) 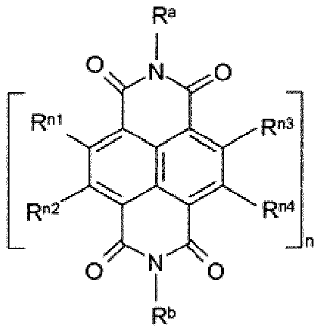 in which
n is 3 or 4,
at least one of the Rⁿ¹, Rⁿ², Rⁿ³ and Rⁿ⁴ radicals is fluorine,
the rest of the Rⁿ¹, Rⁿ², Rⁿ³ and Rⁿ⁴ radicals are hydrogen, and
R^{a} and R^{b} are each independently hydrogen or unsubstituted or substituted alkyl,
alkenyl, alkynyl, cycloalkyl, bicycloalkyl, cycloalkenyl, heterocycloalkyl, aryl or heteroaryl,
in which
the expression "alkyl" also comprises alkyl radicals whose carbon chains
may be interrupted by one or more nonadjacent groups selected from -O-, -S-, -NR^{e}-, -CO-, -SO- and/or -SO₂-, in which R^{e} is hydrogen,
alkyl, cycloalkyl, heterocycloalkyl, aryl or hetaryl,
substituted alkyl groups, depending on the length of the alkyl chain, have
one or more substituents independently selected from cycloalkyl, heterocycloalkyl, aryl, hetaryl, halogen, hydroxyl, thiol, COOH, carboxylate, SO₃H, sulfonate, NE¹E², nitro and cyano, where E¹ and E² are each independently hydrogen, alkyl, cycloalkyl, heterocycloalkyl, aryl or hetaryl,
the expression "alkenyl" comprises straight-chain and branched alkenyl
groups which, depending on the chain length, may bear one or more double bonds,
substituted alkenyl groups may bear one or more substituents selected
independently from cycloalkyl, heterocycloalkyl, aryl, hetaryl, halogen, hydroxyl, thiol, COOH, carboxylate, SO₃H, sulfonate, NE³E⁴, nitro and cyano, where E³ and E⁴ are each independently hydrogen, alkyl, cycloalkyl, heterocycloalkyl, aryl or hetaryl,
substituted alkynyls bear one or more of the substituents mentioned above
for alkyl,
substituted cycloalkyl groups have one or more substituents independently
selected from alkyl and the substituents mentioned above for the alkyl groups,
the expression "bicycloalkyl" comprises bicyclic hydrocarbyl radicals having 5 to 10 carbon atoms,
the expression "heterocycloalkyl" comprises nonaromatic, unsaturated or
fully saturated cycloaliphatic groups having 5 to 8 ring atoms, in which 1, 2 or 3 of the ring carbon atoms are replaced by heteroatoms selected from oxygen, nitrogen, sulfur and an -NRe- group, and which is unsubstituted or substituted by one or more C₁-C₆-alkyl groups,
substituted aryls, depending on the number and size of the ring systems
thereof, have one or more substituents independently selected from alkyl, alkoxy, cycloalkyl, heterocycloalkyl, aryl, hetaryl, halogen, hydroxyl, thiol, COOH, carboxylate, SO₃H, sulfonate, NE⁵E⁶, nitro and cyano, where E⁵ and E⁶ are each independently hydrogen, alkyl, cycloalkyl, heterocycloalkyl, aryl or hetaryl,
the substituents of heteroaryl are selected from C₁-C₆-alkyl, C₁-C₆-alkoxy,
hydroxyl, carboxyl, halogen and cyano,
in which a dianhydride of the formula (I.A) 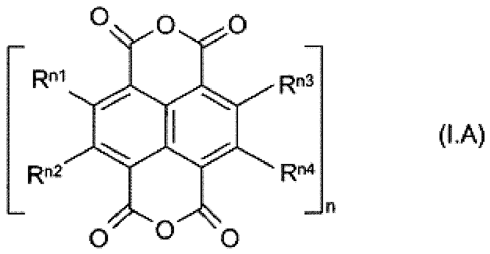 where
n is 3 or 4,
at least one of the Rⁿ¹, Rⁿ², Rⁿ³ and Rⁿ⁴ radicals is fluorine, the rest of the Rⁿ¹, Rⁿ², Rⁿ³ and Rⁿ⁴ radicals are hydrogen,
is subjected to a reaction with an amine of the formula R^{a}-NH₂ and optionally a
different amine of the formula R^{b}-NH₂.

14. A process for preparing at least one compound of the formulae I.Bb 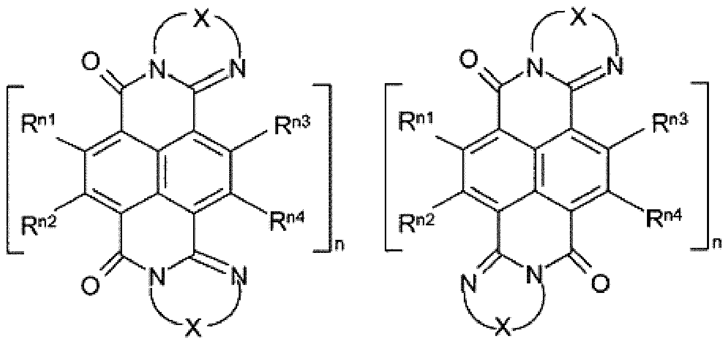 in which
n is 2, 3 or 4,
at least one of the Rⁿ¹, Rⁿ², Rⁿ³ and Rⁿ⁴ radicals is fluorine,
the rest of the Rⁿ¹, Rⁿ², Rⁿ³ and Rⁿ⁴ radicals are hydrogen, and
X is a divalent bridging group having 2 to 5 atoms between the flanking
bonds,
in which a dianhydride of the formula (I.A) 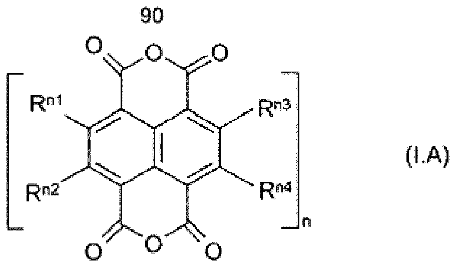 where
n is 3 or 4,
at least one of the Rⁿ¹, Rⁿ², Rⁿ³ and Rⁿ⁴ radicals is fluorine,
the rest of the Rⁿ¹, Rⁿ², Rⁿ³ and Rⁿ⁴ radicals are hydrogen, is subjected to a reaction with an amine of the formula H₂N-X-NH₂.

15. A process for preparing compounds of the formula (I.BaH) 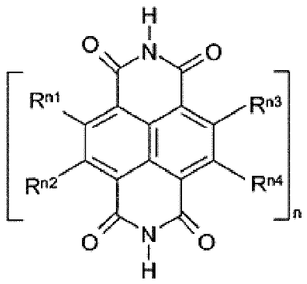 in which
n is 3 or 4,
at least one of the Rⁿ¹, Rⁿ², Rⁿ³ and Rⁿ⁴ radicals is fluorine, and the rest of the Rⁿ¹, Rⁿ², Rⁿ³ and Rⁿ⁴ radicals are hydrogen,
in which a diimide of the formula (I.Ba2), 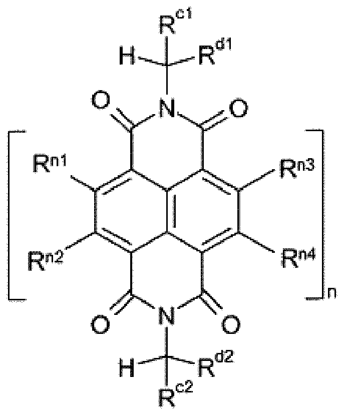 in which
n is 2, 3 or 4,
at least one of the Rⁿ¹, Rⁿ², Rⁿ³ and Rⁿ⁴ radicals is fluorine, the rest of the Rⁿ¹, Rⁿ², Rⁿ³ and Rⁿ⁴ radicals are hydrogen, R^{c1} and R^{c2} are each independently aryl, and
R^{d1} and R^{d2} are each independently alkyl,
is subjected to a reaction with a strong Lewis acid and a proton donor.

16. A compound of the general formula I 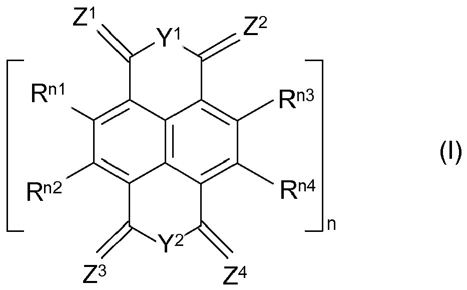 or compounds of the formulae I.Bb 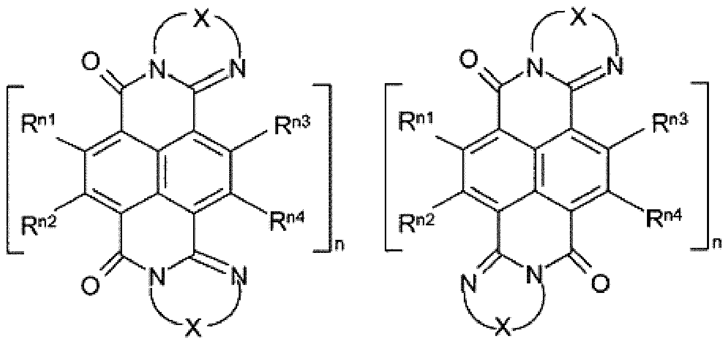 where
n in the compounds of the general formula I is 3, 4, 5 or 6 and in the
compounds of the general formulae I.Bb is 2, 3 or 4,
at least one of the Rⁿ¹, Rⁿ², Rⁿ³ and Rⁿ⁴ radicals is fluorine,
optionally at least one further Rⁿ¹, Rⁿ², Rⁿ³ and Rⁿ⁴ radical is a substituent which is selected from Cl and Br, and the remaining radicals are each hydrogen,
X is a divalent bridging group having from 2 to 5 atoms between the flanking
bonds,
Y¹ is O or NR^{a} where R^{a} is hydrogen or an organyl radical,
Y² is O or NR^{b} where R^{b} is hydrogen or an organyl radical, Z¹, Z², Z³ and Z⁴ are each O.

17. An organic field-effect transistor comprising a substrate having at least one gate structure, a source electrode and a drain electrode, and at least one compound of the formula I as defined in claim 1 as an n-semiconductor.

18. A substrate having a multitude of organic field-effect transistors, at least some of the field-effect transistors comprising at least one compound of the formula I as defined in claim 1 as an n-semiconductor.

19. A semiconductor unit comprising at least one substrate as defined in claim 18.

## Revendications

1. Utilisation de composés de formule générale I 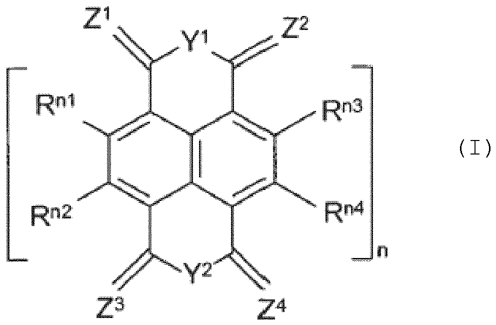 ou de composés de formules I.Bb 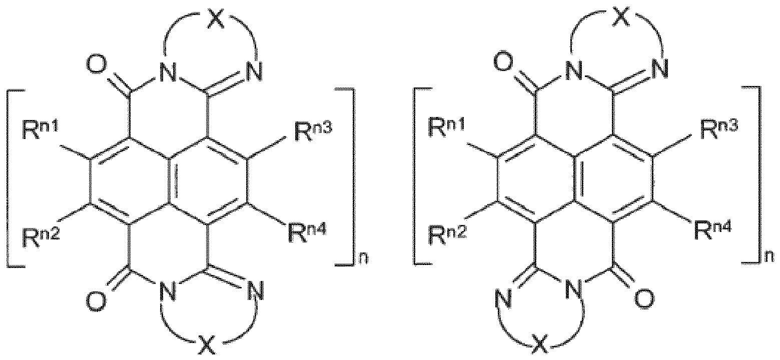 dans lesquelles n représente 3 ou 4 dans les composés de formule
générale I et 2, 3 ou 4 dans les composés de formules générales I.Bb,
au moins l'un des radicaux Rⁿ¹, Rⁿ², Rⁿ³ et Rⁿ⁴ représente un atome de fluor,
éventuellement au moins un autre radical Rⁿ¹, Rⁿ², Rⁿ³ et Rⁿ⁴ représente un substituant qui est choisi parmi Cl et Br, et les autres radicaux représentent un atome d'hydrogène,
X représente un groupe pontant divalent ayant de 2 à 5 atomes entre les liaisons qui l'encadrent,
Y¹ représente O ou NR^{a}, R^{a} représentant un atome
d'hydrogène ou un radical organyle,
Y² représente O ou NR^{b}, R^{b} représentant un atome
d'hydrogène ou un radical organyle,
z¹, z², z³ et z⁴ représentent 0,
en tant que semi-conducteurs, en particulier en tant que semi-conducteurs n.

2. Utilisation selon la revendication 1 en électronique organique, en particulier dans des transistors à effet de champ organiques, des diodes électroluminescentes organiques ou dans des cellules solaires.

3. Utilisation selon la revendication 1 en photovoltaïque organique, en particulier dans des cellules solaires excitoniques.

4. Utilisation d'un composé de formule générale I, tel que défini dans la revendication 1, pour des marqueurs optiques, pour le marquage invisible de produits, en tant que colorants fluorescents, en tant que marqueur fluorescent pour des biomolécules et en tant que pigments.

5. Utilisation d'un composé de formule générale I, tel que défini dans la revendication 1, en tant que colorant fluorescent dans un affichage reposant sur la conversion de fluorescence ; dans une pièce en matière plastique collectrice de lumière, qui est éventuellement associée à une cellule solaire ; en tant que colorant pigmentaire dans des affichages électrophorétiques ; en tant que colorant fluorescent dans une application basée sur la chimiluminescence.

6. Procédé pour la préparation de composés de formule I 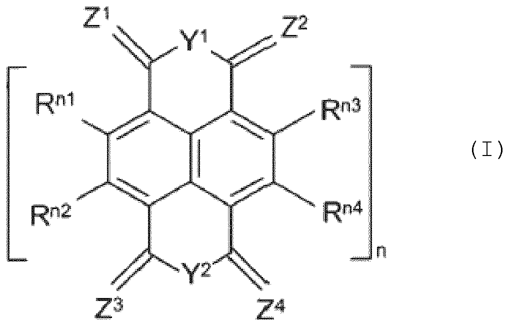 dans laquelle n, R , Rⁿ², Rⁿ³ et Rⁿ⁴, Y¹, Y², Z¹, Z², Z³ et Z⁴ sont tels que définis dans la revendication 1, dans lequel on soumet un composé de formule I, dans lequel au moins l'un des radicaux R , Rⁿ², Rⁿ³ et Rⁿ⁴, R¹, R², R³ et R⁴ représente Cl ou Br, à une réaction avec un fluorure de métal alcalin, dans des conditions anhydres.

7. Procédé selon la revendication 6, dans lequel on utilise dans la réaction avec le fluorure de métal alcalin en plus un complexant, de préférence un éther-couronne.

8. Procédé selon la revendication 6 ou 7, dans lequel on utilise dans la réaction avec le fluorure de métal alcalin en plus un catalyseur de transfert de phase, qui est choisi parmi des composés 2-azaallénium, des composés carbophosphazénium, des composés aminophosphonium et des composés diphosphazénium.

9. Procédé selon la revendication 8, dans lequel on utilise comme catalyseur de transfert de phase le chlorure de (N,N-diméthylimidazolidino)-tétraméthyl-guanidinium.

10. Procédé pour la préparation de composés de formule I.Ba 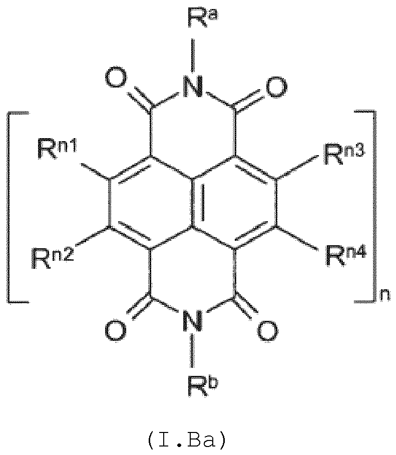 dans laquelle n, Rⁿ¹, Rⁿ², Rⁿ³ et Rⁿ⁴ sont tels que définis dans la revendication 1, et
R^{a} et R^{b} représentent indépendamment l'un de l'autre un
atome d'hydrogène ou un groupe alkyle, alcényle, alcynyle, cycloalkyle, bicycloalkyle, cycloalcényle, hétérocycloalkyle, aryle ou hétéroaryle, substitué ou non substitué,
où
le terme alkyle englobe également des radicaux alkyle
dont les chaînes carbonées peuvent être interrompues par un ou plusieurs groupes non contigus, qui sont choisis parmi -O-, -S-, -NR^{e}-, -CO-, -SO- et/ou -SO₂-, R^{e} représentant un atome d'hydrogène, un groupe alkyle, cycloalkyle, hétérocycloalkyle, aryle ou hétéroaryle,
les groupes alkyle substitués comportent, en fonction
de la longueur de la chaîne alkyle, un ou plusieurs substituants qui sont choisis, indépendamment les uns des autres, parmi cycloalkyle, hétérocycloalkyle, aryle, hétéroaryle, halogéno, hydroxy, thiol, COOH, carboxylate, SO₃H, sulfonate, NE¹E², nitro et cyano, E¹ et E² représentant, indépendamment l'un de l'autre, un atome d'hydrogène, un groupe alkyle, cycloalkyle, hétérocycloalkyle, aryle ou hétéroaryle,
le terme « alcényle » englobe des groupes alcényle à
chaîne droite ou ramifiée, qui peuvent comporter, en fonction de la longueur de chaîne, une ou plusieurs doubles liaisons,
les groupes alcényle substitués peuvent porter un ou
plusieurs substituants qui sont choisis, indépendamment les uns des autres, parmi cycloalkyle, hétérocycloalkyle, aryle, hétéroaryle, halogéno, hydroxy, thiol, COOH, carboxylate, SO₃H, sulfonate, NE³E⁴, nitro et cyano, E³ et E⁴ représentant, indépendamment l'un de l'autre, un atome d'hydrogène, un groupe alkyle, cycloalkyle, hétérocycloalkyle, aryle ou hétéroaryle,
les groupes alcynyle substitués portent un ou plusieurs
des substituants nommés précédemment pour alkyle, les groupes cycloalkyle substitués comportent un ou
plusieurs substituants qui sont choisis, indépendamment les uns des autres, parmi des groupes alkyle ainsi que les substituants nommés précédemment pour les groupes alkyle,
le terme bicycloalkyle englobe des radicaux
hydrocarbonés bicycliques ayant de 5 à 10 atomes de carbone,
le terme hétérocycloalkyle englobe des groupes
cycloaliphatiques non aromatiques, insaturés ou totalement saturés, ayant de 5 à 8 atomes formant le cycle, dans lesquels 1, 2 ou 3 des atomes de carbone formant le cycle sont remplacés par des hétéroatomes choisis parmi les atomes d'oxygène, d'azote, de soufre et un groupe -NR^{e}-, et qui est non substitué ou est substitué par un ou plusieurs groupes alkyle en C₁-C₆,
les groupes aryle substitués comportent, en fonction du
nombre et la taille de leurs systèmes cycliques, un ou plusieurs substituants qui sont choisis, indépendamment les uns des autres, parmi alkyle, alcoxy, cycloalkyle, hétérocycloalkyle, aryle, hétéroaryle, halogéno, hydroxy, thiol, COOH, carboxylate, SO₃H, sulfonate, NE⁵E⁶, nitro et cyano, E⁵ et E⁶ représentant, indépendamment l'un de l'autre, un atome d'hydrogène, un groupe alkyle, cycloalkyle, hétérocycloalkyle, aryle ou hétéroaryle,
les substituants du groupe hétéroaryle sont choisis
parmi des groupes alkyle en C₁-C₆, alcoxy en C₁-C₆, hydroxy, carboxy, halogéno et cyano,
dans lequel
a1) on soumet un Rylènedianhydride de formule II, 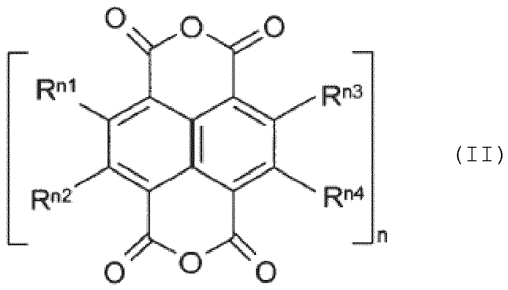 dans laquelle au moins l'un des radicaux Rⁿ¹, Rⁿ², Rⁿ³ et Rⁿ⁴ représente Cl ou Br et n représente 3 ou 4, à une réaction avec un fluorure de métal alcalin, dans des conditions essentiellement anhydres,
b1) on soumet le composé obtenu dans l'étape a1) à une
réaction avec une amine de formule R^{a}-NH₂ et éventuellement une amine différente de celle-ci, de formule R^{b}-NH₂.

11. Procédé pour la préparation de composés de formules I.Bb 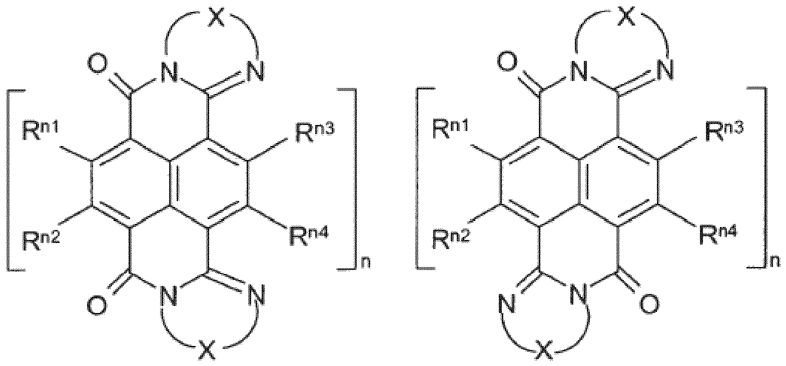 dans laquelle n, X, Rⁿ¹, Rⁿ², Rⁿ³ et Rⁿ⁴ sont tels que définis dans la revendication 1, dans lequel
a2) on soumet un Rylènedianhydride de formule II, 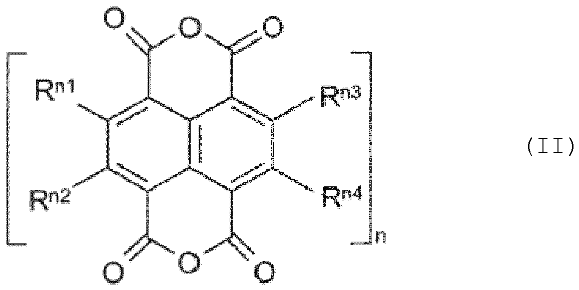 dans laquelle au moins l'un des radicaux Rⁿ¹, Rⁿ², Rⁿ³ et Rⁿ⁴ représente Cl ou Br et n représente 2, 3 ou 4, à une réaction avec un fluorure de métal alcalin, dans des conditions essentiellement anhydres,
b2) on soumet le composé obtenu dans l'étape a2) à une
réaction avec une amine de formule H₂N-X-NH₂.

12. Procédé pour la préparation de dianhydrides de formule (I.A) 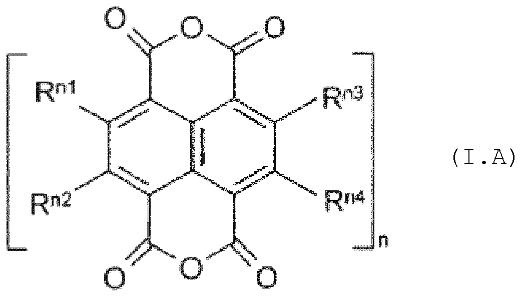 dans laquelle
n représente 3 ou 4,
au moins l'un des radicaux R , Rⁿ², Rⁿ³ et R
représente un atome de fluor,
les radicaux Rⁿ¹, Rⁿ², Rⁿ³ et Rⁿ⁴ restants
représentent un atome d'hydrogène,
dans lequel on soumet un diimide de formule (I.Ba), 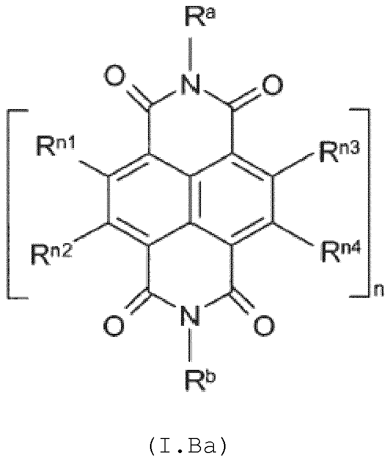 R^{a} et R^{b} représentant indépendamment l'un de l'autre un
atome d'hydrogène ou un groupe alkyle, alcényle, alcynyle, cycloalkyle, bicycloalkyle, cycloalcényle, hétérocycloalkyle, aryle ou hétéroaryle, substitué ou non substitué,
où
le terme alkyle englobe également des radicaux
alkyle dont les chaînes carbonées peuvent être interrompues par un ou plusieurs groupes non contigus, qui sont choisis parmi -O-, -S-, -NR^{e}-, -CO-, -SO- et/ou -SO₂-, R^{e} représentant un atome d'hydrogène, un groupe alkyle, cycloalkyle, hétérocycloalkyle, aryle ou hétéroaryle,
les groupes alkyle substitués comportent, en
fonction de la longueur de la chaîne alkyle, un ou plusieurs substituants, qui sont choisis, indépendamment les uns des autres, parmi cycloalkyle, hétérocycloalkyle, aryle, hétéroaryle, halogéno, hydroxy, thiol, COOH, carboxylate, SO₃H, sulfonate, NE¹E², nitro et cyano, E¹ et E² représentant, indépendamment l'un de l'autre, un atome d'hydrogène, un groupe alkyle, cycloalkyle, hétérocycloalkyle, aryle ou hétéroaryle,
le terme « alcényle » englobe des groupes alcényle
à chaîne droite ou ramifiée, qui peuvent comporter, en fonction de la longueur de chaîne, une ou plusieurs doubles liaisons,
les groupes alcényle substitués peuvent porter un
ou plusieurs substituants qui sont choisis, indépendamment les uns des autres, parmi cycloalkyle, hétérocycloalkyle, aryle, hétéroaryle, halogéno, hydroxy, thiol, COOH, carboxylate, SO₃H, sulfonate, NE³E⁴, nitro et cyano, E³ et E⁴ représentant, indépendamment l'un de l'autre, un atome d'hydrogène, un groupe alkyle, cycloalkyle, hétérocycloalkyle, aryle ou hétéroaryle,
les groupes alcynyle substitués portent un ou
plusieurs des substituants nommés précédemment pour alkyle,
les groupes cycloalkyle substitués comportent un
ou plusieurs substituants qui sont choisis, indépendamment les uns des autres, parmi des groupes alkyle ainsi que les substituants nommés précédemment pour les groupes alkyle,
le terme bicycloalkyle englobe des radicaux
hydrocarbonés bicycliques ayant de 5 à 10 atomes de carbone,
le terme hétérocycloalkyle englobe des groupes
cycloaliphatiques non aromatiques, insaturés ou totalement saturés, ayant de 5 à 8 atomes formant le cycle, dans lesquels 1, 2 ou 3 des atomes de carbone formant le cycle sont remplacés par des hétéroatomes choisis parmi les atomes d'oxygène, d'azote, de soufre et un groupe -NR^{e}-, et qui est non substitué ou est substitué par un ou plusieurs groupes alkyle en C₁-C₆,
les groupes aryle substitués comportent, en
fonction du nombre et la taille de leurs systèmes cycliques, un ou plusieurs substituants qui sont choisis, indépendamment les uns des autres, parmi alkyle, alcoxy, cycloalkyle, hétérocycloalkyle, aryle, hétéroaryle, halogéno, hydroxy, thiol, COOH, carboxylate, SO₃H, sulfonate, NE⁵E⁶, nitro et cyano, E⁵ et E⁶ représentant, indépendamment l'un de l'autre, un atome d'hydrogène, un groupe alkyle, cycloalkyle, hétérocycloalkyle, aryle ou hétéroaryle,
les substituants du groupe hétéroaryle sont
choisis parmi des groupes alkyle en C₁-C₆, alcoxy en C₁-C₆,hydroxy, carboxy, halogéno et cyano,
à une hydrolyse en deux stades, le premier stade s'effectuant en présence d'une base et le deuxième en présence d'un acide.

13. Procédé pour la préparation de composés de formule (I.Ba) 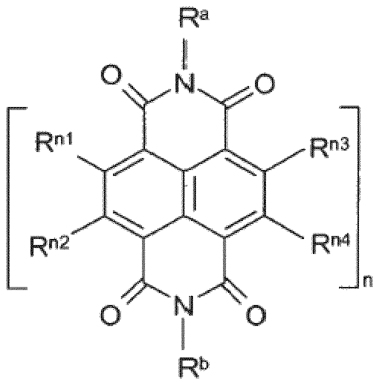 dans laquelle
n représente 3 ou 4,
au moins l'un des radicaux Rⁿ¹, Rⁿ², Rⁿ³ et Rⁿ⁴
représente un atome de fluor,
les radicaux Rⁿ¹, Rⁿ², Rⁿ³ et Rⁿ⁴ restants
représentent un atome d'hydrogène, et
R^{a} et R^{b} représentent indépendamment l'un de
l'autre un atome d'hydrogène ou un groupe alkyle, alcényle, alcynyle, cycloalkyle, bicycloalkyle, cycloalcényle, hétérocycloalkyle, aryle ou hétéroaryle, substitué ou non substitué,
où
le terme alkyle englobe également des radicaux
alkyle dont les chaînes carbonées peuvent être interrompues par un ou plusieurs groupes non contigus, qui sont choisis parmi -O-, -S-, -NR^{e}-, -CO-, -SO- et/ou -SO₂-, R^{e} représentant un atome d'hydrogène, un groupe alkyle, cycloalkyle, hétérocycloalkyle, aryle ou hétéroaryle,
les groupes alkyle substitués comportent, en
fonction de la longueur de la chaîne alkyle, un ou plusieurs substituants qui sont choisis, indépendamment les uns des autres, parmi cycloalkyle, hétérocycloalkyle, aryle, hétéroaryle, halogéno, hydroxy, thiol, COOH, carboxylate, SO₃H, sulfonate, NE¹E², nitro et cyano, E¹ et E² représentant, indépendamment l'un de l'autre, un atome d'hydrogène, un groupe alkyle, cycloalkyle, hétérocycloalkyle, aryle ou hétéroaryle,
le terme « alcényle » englobe des groupes alcényle
à chaîne droite ou ramifiée, qui peuvent comporter, en fonction de la longueur de chaîne, une ou plusieurs doubles liaisons,
les groupes alcényle substitués peuvent porter un
ou plusieurs substituants, qui sont choisis, indépendamment les uns des autres, parmi cycloalkyle, hétérocycloalkyle, aryle, hétéroaryle, halogéno, hydroxy, thiol, COOH, carboxylate, SO₃H, sulfonate, NE³E⁴, nitro et cyano, E³ et E⁴ représentant, indépendamment l'un de l'autre, un atome d'hydrogène, un groupe alkyle, cycloalkyle, hétérocycloalkyle, aryle ou hétéroaryle,
les groupes alcynyle substitués portent un ou
plusieurs des substituants nommés précédemment pour alkyle,
les groupes cycloalkyle substitués comportent un
ou plusieurs substituants, qui sont choisis, indépendamment les uns des autres, parmi des groupes alkyle ainsi que les substituants nommés précédemment pour les groupes alkyle, le terme bicycloalkyle englobe des radicaux
hydrocarbonés bicycliques ayant de 5 à 10 atomes de carbone,
le terme hétérocycloalkyle englobe des groupes
cycloaliphatiques non aromatiques, insaturés ou totalement saturés, ayant de 5 à 8 atomes formant le cycle, dans lesquels 1, 2 ou 3 des atomes de carbone formant le cycle sont remplacés par des hétéroatomes choisis parmi les atomes d'oxygène, d'azote, de soufre et un groupe -NR^{e}-, et qui est non substitué ou est substitué par un ou plusieurs groupes alkyle en C₁-C₆,
les groupes aryle substitués comportent, en
fonction du nombre et la taille de leurs systèmes cycliques, un ou plusieurs substituants qui sont choisis, indépendamment les uns des autres, parmi alkyle, alcoxy, cycloalkyle, hétérocycloalkyle, aryle, hétéroaryle, halogéno, hydroxy, thiol, COOH, carboxylate, SO₃H, sulfonate, NE⁵E⁶, nitro et cyano, E⁵ et E⁶ représentant, indépendamment l'un de l'autre, un atome d'hydrogène, un groupe alkyle, cycloalkyle, hétérocycloalkyle, aryle ou hétéroaryle,
les substituants du groupe hétéroaryle sont
choisis parmi des groupes alkyle en C₁-C₆, alcoxy en C₁-C₆,hydroxy, carboxy, halogéno et cyano,
dans lequel on soumet un dianhydride de formule (I.A) 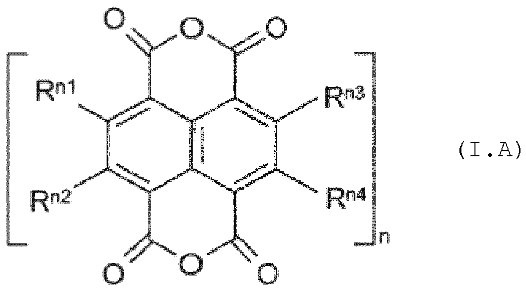 dans laquelle
n représente 3 ou 4,
au moins l'un des radicaux Rⁿ¹, Rⁿ², Rⁿ³ et Rⁿ⁴
représente un atome de fluor,
les radicaux Rⁿ¹, Rⁿ², Rⁿ³ et Rⁿ⁴ restants
représentent un atome d'hydrogène,
à une réaction avec une amine de formule R^{a}-NH₂ et éventuellement une amine différente de celle-ci, de formule R^{b}-NH₂.

14. Procédé pour la préparation d'au moins un composé de formules I.Bb 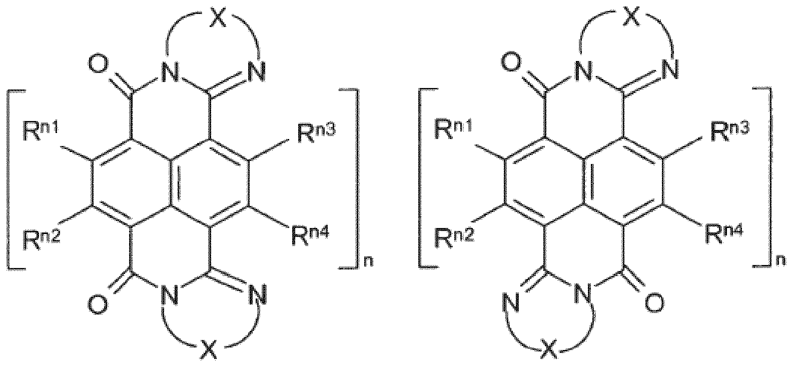 dans lesquelles
n représente 2, 3 ou 4,
au moins l'un des radicaux Rⁿ¹, Rⁿ², Rⁿ³ et Rⁿ⁴
représente un atome de fluor,
les radicaux Rⁿ¹, Rⁿ², Rⁿ³ et Rⁿ⁴ restants
représentent un atome d'hydrogène, et
X représente un groupe pontant divalent ayant de 2 à 5 atomes entre les liaisons qui l'encadrent,
dans lequel on soumet un dianhydride de formule (I.A) 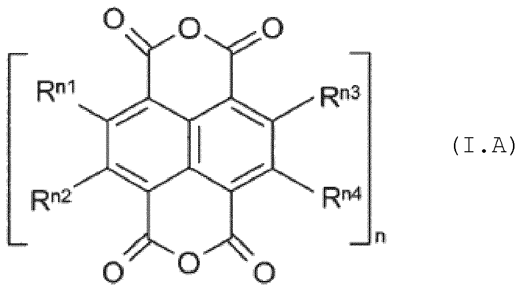 dans laquelle
n représente 3 ou 4,
au moins l'un des radicaux Rⁿ¹, Rⁿ², Rⁿ³ et Rⁿ⁴
représente un atome de fluor,
les radicaux Rⁿ¹, Rⁿ², Rⁿ³ et Rⁿ⁴ restants
représentent un atome d'hydrogène,
à une réaction avec une amine de formule H₂N-X-NH₂.

15. Procédé pour la préparation de composés de formules (I.BaH) 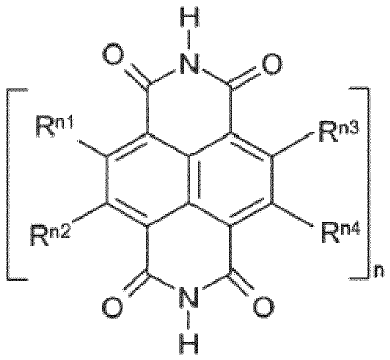 dans lesquelles
n représente 3 ou 4,
au moins l'un des radicaux R , Rⁿ², Rⁿ³ et R
représente un atome de fluor, et
les radicaux Rⁿ¹, Rⁿ², R et R restants
représentent un atome d'hydrogène,
dans lequel on soumet un diimide de formule (I.Ba2) 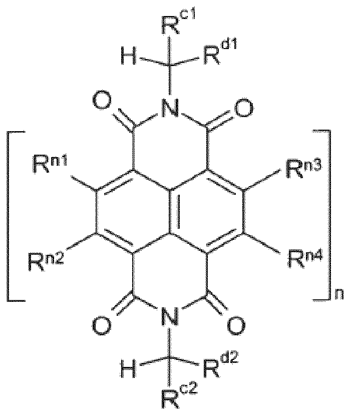 dans laquelle
n représente 2, 3 ou 4,
au moins l'un des radicaux Rⁿ¹, Rⁿ², Rⁿ³ et Rⁿ⁴
représente un atome de fluor,
les radicaux Rⁿ¹, Rⁿ², Rⁿ³ et Rⁿ⁴ restants
représentent un atome d'hydrogène,
R^{c1} et R^{c2} représentent indépendamment l'un de
l'autre un groupe aryle, et
R^{d1} et R^{d2} représentent indépendamment l'un de
l'autre un groupe alkyle,
à une réaction avec un acide de Lewis fort et un donneur de protons.

16. Composés de formule générale I 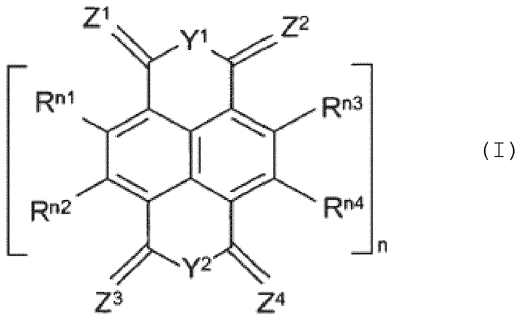 ou composés de formules I.Bb 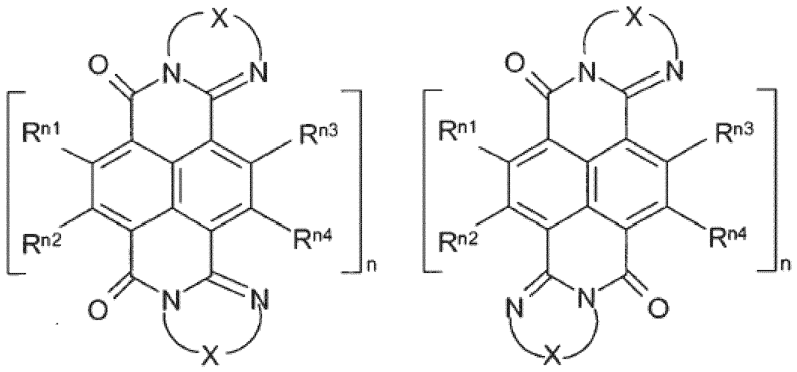 où
n représente 3, 4, 5 ou 6 dans les composés de
formule générale I et
représente 2, 3 ou 4 dans les composés de formules générales I.Bb,
au moins l'un des radicaux Rⁿ¹, Rⁿ², Rⁿ³ et Rⁿ⁴
représente un atome de fluor,
éventuellement au moins un autre radical Rⁿ¹, Rⁿ², Rⁿ³ et
Rⁿ⁴ représente un substituant qui est choisi parmi Cl et Br, et les autres radicaux représentent un atome d'hydrogène,
X représente un groupe pontant divalent ayant de 2 à 5 atomes entre les liaisons qui l'encadrent,
Y¹ représente O ou NR^{a} R^{a} représentant un atome
d'hydrogène ou un radical organyle,
Y² représente O ou NR^{b}, R^{b} représentant un atome
d'hydrogène ou un radical organyle,
Z¹, Z², Z³ et Z⁴ représentent O.

17. Transistor à effet de champ organique, comprenant un substrat ayant au moins une structure de grille, une électrode source et une électrode drain, et au moins un composé de formule I, tel que défini dans la revendication 1, en tant que semi-conducteur n.

18. Substrat comportant une multiplicité de transistors à effet de champ organiques, au moins une partie des transistors à effet de champ contenant au moins un composé de formule I, tel que défini dans la revendication 1, en tant que semi-conducteur n.

19. Composant semi-conducteur, comprenant au moins un substrat tel que défini dans la revendication 18.
